(19)

Europäisches
Patentamt
European
Patent Office
Office européen
des brevets

(11)     **EP 2 225 268 B1**

(12)     **EUROPEAN PATENT SPECIFICATION**

(45) Date of publication and mention
of the grant of the patent:
**29.08.2012  Bulletin 2012/35**

(21) Application number: **08840586.5**

(22) Date of filing: **17.10.2008**

(51) Int Cl.:
*C07K 14/47* (2006.01)     *C07K 16/18* (2006.01)

(86) International application number:
**PCT/US2008/080331**

(87) International publication number:
**WO 2009/052392 (23.04.2009 Gazette 2009/17)**

(54) **IMMUNOASSAYS AND KITS FOR THE DETECTION OF NGAL**

IMMUNASSAYS UND KITS ZUM NACHWEIS VON NGAL

DOSAGES IMMUNOLOGIQUES ET KITS DE DÉTECTION DE LA NGAL

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **19.10.2007  US 981470 P
19.10.2007  US 981471 P
19.10.2007  US 981473 P
16.04.2008  US 104408
16.04.2008  US 104410
16.04.2008  US 104413**

(43) Date of publication of application:
**08.09.2010  Bulletin 2010/36**

(73) Proprietor: **Abbott Laboratories
Abbott Park, Illinois 60064-6008 (US)**

(72) Inventors:
• **BIRKENMEYER, Larry G.
Glenview
Illinois 60025 (US)**
• **DESAI, Suresh M.
Libertyville
Illinois 60048 (US)**
• **GRENIER, Frank C.
Libertyville
Illinois 60048 (US)**
• **HAWKSWORTH, David J.
Lake Villa
Illinois 60046 (US)**
• **OLEJNICZAK, Edward T.
Grayslake
Illinois 60030 (US)**

• **RUAN, Qiaoqiao
Round Lake
Illinois 60073 (US)**
• **SIEGEL, Robert W.
Fountaintown
Indiana 46130 (US)**
• **TETIN, Sergey Y.
Lindenhurst
Illinois 60046 (US)**
• **TIEMAN, Bryan C.
Elmhurst
Illinois 60126 (US)**
• **TU, Bailin
Libertyville
Illinois 60048 (US)**
• **TYNER, Joan D.
Beach Park
Illinois 60087 (US)**
• **WORKMAN, Ryan F.
Waukegan
Illinois 60085 (US)**
• **ZIEMANN, Robert N.
Lindenhurst
Illinois 60046 (US)**

(74) Representative: **Modiano, Micaela Nadia et al
Modiano & Partners
Thierschstrasse 11
80538 München (DE)**

(56) References cited:
**WO-A-2006/086638      WO-A-2007/044994
WO-A-2007/098102**

EP 2 225 268 B1

**(Cont. next page)**

- KJELDSEN L ET AL: "Characterization of two ELISAs for NGAL, a newly described lipocalin in human neutrophils" JOURNAL OF IMMUNOLOGICAL METHODS, ELSEVIER SCIENCE PUBLISHERS B.V.,AMSTERDAM, NL, vol. 198, no. 2, 13 November 1996 (1996-11-13), pages 155-164, XP004071802 ISSN: 0022-1759
- "QUANTIKINE. HUMAN LIPOCALIN-2/NGAL IMMUNOASSAY" INTERNET CITATION, [Online] XP002440684 Retrieved from the Internet: URL: http://www.rndsystems.com/pdf/DLCN20.p df> [retrieved on 2007-07-04]
- GRENIER F ET AL: "Multi-site evaluation of an assay in development for NGAL (Neutrophil Gelatinase-Associated Lipocalin) on the Abbott ARCHITECT (R) analyzer" CLINICAL CHEMISTRY, vol. 54, no. 6, June 2008 (2008-06), pages Suppl-A170, XP009110203 ISSN: 0009-9147 [retrieved on 2008-06-01]

**Description**

RELATED APPLICATION INFORMATION

This application claims the priority of U.S. Provisional Application Serial Numbers 60/981,470, 60/981,471 and 60/981,473, all filed on October 19, 2007, and of U.S. Nonprovisional Application Serial Numbers 12/104,408, 12/104,410, and 12/104,413, all filed on April 16, 2008.

TECHNICAL FIELD

The present invention relates to NGAL immunoassays and kits, and to antibodies that bind to mammalian NGAL in immunoassays and kits. Such immunoassays and kits among other things optionally provide for improved detection of monomer. The methods and kits can be employed to determine the amount of human NGAL monomer in a test sample, as well as to determine the proportion of human NGAL monomer to human NGAL dimer contained in a test sample.

BACKGROUND

Lipocalins are a family of extracellular ligand-binding proteins that are found in a variety of organisms from bacteria to humans. Lipocalins possess many different functions, such as the binding and transport of small hydrophobic molecules, nutrient transport, cell growth regulation, and modulation of the immune response, inflammation and prostaglandin synthesis. Moreover, some lipocalins are also involved in cell regulatory processes and serve as diagnostic and prognostic markers in a variety of disease states. For example, the plasma level of alpha glycoprotein is monitored during pregnancy and in diagnosis and prognosis of conditions including cancer chemotherapy, renal dysfunction, myocardial infarction, arthritis, and multiple sclerosis.

The novel lipocalin neutrophil gelatinase-associated lipocalin (or NGAL, also known as Lipocalin-2 or LCN2) from human neutrophils was identified in 1993. NGAL is a 25-kDa lipocalin that exists in monomeric and homo- and heterodimeric forms, the latter as a 46-kDa dimer with human neutrophil gelatinase. A trimer form of NGAL has also been identified. NGAL is secreted from specific granules of activated human neutrophils. Homologous proteins have been identified in mouse (24p3/uterocalin) and rat (alpha (2)-microglobulin-related protein/neu-related lipocalin). Structural data have confirmed a typical lipocalin fold of NGAL with an eight-stranded beta-barrel, but with an unusually large cavity lined with more polar and positively charged amino acid residues than normally seen in lipocalins. The 25-kDa NGAL protein is believed to bind small lipophilic substances such as bacteria-derived lipopolysaccharides and formylpeptides, and may function as a modulator of inflammation.

Renal injuries or disease, such as acute kidney failure or chronic kidney failure, can result from a variety of different causes (such as illness, injury, and the like). The early identification and treatment of renal injuries and disease would be useful in preventing disease progression. Currently, serum creatinine is frequently used as a biomarker of kidney function. However, serum creatinine measurements are influenced by muscle mass, gender, race and medications. Unfortunately, these limitations often result in the diagnosis of kidney disease only after significant damage has already occurred.

NGAL is an early marker for acute renal injury or disease. In addition to being produced by specific granules of activated human neutrophils, NGAL is also produced by nephrons in response to tubular epithelial damage and is a marker of tubulointerstitial (TI) injury. NGAL levels rise in acute tubular necrosis (ATN) from ischemia or nephrotoxicity, even after mild "subclinical" renal ischemia, as compared to normal serum creatinine levels. Moreover, NGAL is known to be expressed by the kidney in cases of chronic kidney disease (CKD). Elevated urinary NGAL levels have been suggested as predictive of progressive kidney failure. It has been previously demonstrated that NGAL is markedly expressed by kidney tubules very early after ischemic or nephrotoxic injury in both animal and human models. NGAL is rapidly secreted into the urine, where it can be easily detected and measured, and precedes the appearance of any other known urinary or serum markers of ischemic injury. The protein is resistant to proteases, suggesting that it can be recovered in the urine as a faithful marker of tubule expression of NGAL. Further, NGAL derived from outside of the kidney, for example, filtered from the blood, does not appear in the urine, but rather is quantitatively taken up by the proximal tubule.

A variety of immunoassays are known in the art for detecting NGAL. As mentioned previously herein, NGAL is found as a monomer, as a dimer (a homodimer or heterodimer) and even as a trimer. Thus, there is a need in the art for new antibodies and immunoassays which are able to specifically detect and distinguish between NGAL monomer, dimer or trimer in a test sample. Additionally, there is also a need in the art for immunoassays that are able to quantify the relative proportion of monomer to dimer contained in a test sample. Such new antibodies and immunoassays can be used to assess among other things the extent of any renal injury or disease in a patient, monitor the kidney status of a patient suffering from renal injury or disease, or assess the extent of any renal injury in a patient and thereafter monitor the patient's kidney status. Additional objects and advantages of the invention will be apparent from the description provided herein.

## SUMMARY

In one embodiment, the instant invention pertains to a method for determining the amount of human NGAL monomer in a test sample, the method comprising the steps of:

(a) contacting a test sample suspected of containing human NGAL monomer and human NGAL dimer with at least one first antibody so as to form a first antibody/human NGAL complex, wherein said at least one capture first antibody binds to human NGAL and is an antibody selected from the group consisting of an antibody produced by murine hybridoma cell line 1-2322-455 having ATCC Accession No. PTA-8024 and an antibody produced by murine hybridoma cell line 1-903-430 having ATCC Accession No. PTA-8026;
(b) contacting said antibody/human NGAL complex with a second antibody that binds to human NGAL and that has been conjugated to a detectable label to form a second antibody/human NGAL/first antibody complex, wherein said second antibody differs from said first antibody and is an antibody selected from the group consisting of: an antibody produced by murine hybridoma cell line 1-2322-455 having ATCC Accession No. PTA-8024 and an antibody produced by murine hybridoma cell line 1-903-430 having ATCC Accession No. PTA-8026; and
(c) determining with at least about 75% specificity the amount of human NGAL monomer contained in the test sample based on the amount of the second antibody/human NGAL/first antibody complex formed in step (b).

In a further embodiment, the instant invention pertains to a method for determining the proportion of human NGAL monomer to human NGAL dimer contained in a test sample, said method comprising the steps of:

(a) contacting a test sample suspected of containing human NGAL monomer and human NGAL dimer with at least one first antibody so as to form a first antibody/human NGAL complex, wherein said at least one first antibody binds to human NGAL and is an antibody selected from the group consisting of an antibody produced by murine hybridoma cell line 1-2322-455 having ATCC Accession No. PTA-8024 and an antibody produced by murine hybridoma cell line 1-903-430 having ATCC Accession No. PTA-8026;
(b) contacting said first antibody/human NGAL complex with a second antibody that binds to human NGAL and that has been conjugated to a detectable label so as to form a second antibody/human NGAL/first antibody complex, wherein said second antibody differs from said first antibody and is an antibody selected from the group consisting of an antibody produced by murine hybridoma cell line 1-2322-455 having ATCC Accession No. PTA-8024 and an antibody produced by murine hybridoma cell line 1-903-430 having ATCC Accession No. PTA-8026;
(c) determining the amount of the second antibody/human NGAL/first antibody complex formed in step (b), wherein steps (a) and (b) are performed in the absence of any reducing agents;
(d) determining the amount of said second antibody/human NGAL complex formed in step (b), wherein steps (a) and (b) are performed in the presence of or following treatment of said test sample with at least one reducing agent; and
(e) determining the proportion of human NGAL monomer to human NGAL dimer in said test sample based on the amount of the second antibody/human NGAL/first antibody complex determined in step (c) and the amount of the second antibody/human NGAL/first antibody complex determined in step (d).

In a still further embodiment, the instant invention pertains to a method for evaluating the renal tubular cell injury status of a subject, in which method the level of human NGAL monomer and/or the proportion of human NGAL monomer to human NGAL dimer in a test sample is determined according to one or more of the above-described embodiments.
In a further embodiment, the instant invention pertains to a method for detecting the presence of human NGAL antigen in a test sample, said method comprising:

(1) contacting a test sample suspected of containing human NGAL with the immunodiagnostic reagent as set forth herein for a time and under conditions that allow formation of a human NGAL/antibody complex; and
(2) detecting any human NGAL/antibody complex formed as indicating the presence of said human NGAL antigen,

wherein said immunodiagnostic reagent comprises one or more antibodies selected from the group consisting of:

(a) an antibody that specifically binds to human NGAL protein at a conformational epitope comprising amino acid residues 112, 118 and 147 of human NGAL protein as set forth in SEQ ID NOS:1, 2, 10 or 13 and at least one additional amino acid that is residue 117 or 119 of wild-type human NGAL of SEQ ID NOS: 1 or 13;
(b) an isolated antibody that specifically binds to human NGAL, wherein said antibody has a variable heavy domain region comprising the amino acid sequence of SEQ ID NO:5 and a variable light domain region comprising the amino acid sequence of SEQ ID NO:6; and
(c) an antibody produced by murine hybridoma cell line 1-2322-455 having ATCC Accession No. PTA-8024;

and one or more antibodies selected from the group consisting of:

(d) an isolated antibody that specifically binds to human NGAL, wherein said antibody has a variable heavy domain region comprising the amino acid sequence of SEQ ID NO:7 and a variable light domain region comprising the amino acid sequence of SEQ ID NO:8; and
(e) an antibody produced by murine hybridoma cell line 1-903-430 having ATCC Accession No. PTA-8026.

In a further embodiment, the instant invention pertains to a diagnostic kit for the detection of human NGAL comprising instructions and an immunodiagnostic reagent that comprises one or more antibodies selected from the group consisting of:

(a) an antibody that specifically binds to human NGAL protein at a conformational epitope comprising amino acid residues 112, 118 and 147 of human NGAL protein as set forth in SEQ ID NOS:1, 2, 10 or 13 and at least one additional amino acid that is residue 117 or 119 of wild-type human NGAL of SEQ ID NOS: 1 or 13;
(b) an isolated antibody that specifically binds to human NGAL, wherein said antibody has a variable heavy domain region comprising the amino acid sequence of SEQ ID NO:5 and a variable light domain region comprising the amino acid sequence of SEQ ID NO:6; and
(c) an antibody produced by murine hybridoma cell line 1-2322-455 having ATCC Accession No. PTA-8024;
and one or more antibodies selected from the group consisting of:
(d) an isolated antibody that specifically binds to human NGAL, wherein said antibody has a variable heavy domain region comprising the amino acid sequence of SEQ ID NO:7 and a variable light domain region comprising the amino acid sequence of SEQ. ID. NO:8; and
(e) an antibody produced by murine hybridoma cell line 1-903-430 having ATCC Accession No. PTA-8026.

## BRIEF DESCRIPTION OF THE FIGURE

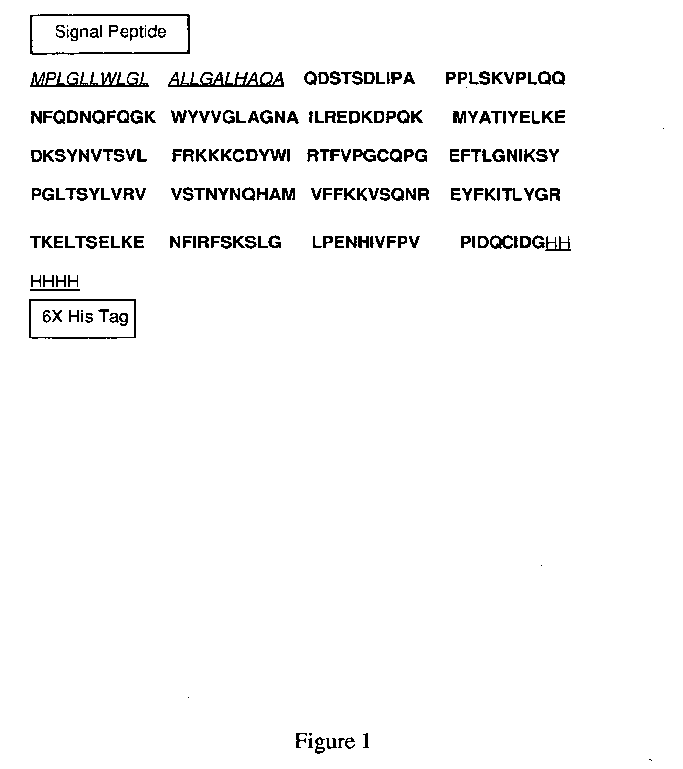

Figure 1 shows the human NGAL wild-type antigen sequence (SEQ ID NO: 1). Native human NGAL signal peptide residues are in italics and underlined. Wild-type human NGAL sequences in pJV-NGAL-A3 plasmid are in bold. The 6xHis tag in the C-terminal is underlined.

## DETAILED DESCRIPTION

Glycosylated mammalian NGAL proteins, and antibodies that bind to mammalian NGAL proteins have been discovered. These NGAL proteins and antibodies alone or in or in combination have a variety of uses, for example, as a component of a diagnostic assay, or present in an immunoassay kit.

All NGAL polynucleotide and polypeptide sequences, and wild-type NGAL recombinant antigen (rAg) and mutant C87S NGAL NGAL rAg clones, subclones. hybrids, and hybridomas (including names and numbering) are as described in U.S. Provisional Application Serial Number 60/981,470 filed October 19, 2007 (incorporated by reference for its teachings regarding same).

Antibodies that bind to certain mammalian NGAL proteins also have been discovered. These anti-NGAL antibodies (also loosely referred to herein as "NGAL antibodies), are as described in U.S. Provisional Application Serial Number 60/981,471 filed October 19, 2007 (incorporated by reference for its teachings regarding same).

## A. Definitions

As used herein, the singular forms "a," "an" and "the" include plural referents unless the context clearly dictates otherwise. For the recitation of numeric ranges herein, each intervening number there between with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

### a) Antibody

As used herein, the terms "antibody" and "antibodies" refer to monoclonal antibodies, multispecific antibodies, human antibodies, humanized antibodies (fully or partially humanized), animal antibodies (in one aspect, a bird (for example, a duck or goose), in another aspect, a shark or whale, in yet another aspect, a mammal, including a non-primate (for example, a cow, pig, camel, llama, horse, goat, rabbit, sheep, hamsters, guinea pig, feline, canine, rat, murine, etc) and a non-human primate (for example, a monkey, such as a cynomologous monkey, a chimpanzee, etc), recombinant antibodies, chimeric antibodies, single-chain Fvs (scFv), single chain antibodies, single domain antibodies, Fab fragments, $F(ab')_2$ fragments, disulfide-linked Fv (sdFv), and anti-idiotypic (anti-Id) antibodies (including, for example, anti-Id antibodies to antibodies of the present invention), and functionally active epitope-binding fragments of any of the above. In particular, antibodies include immunoglobulin molecules and immunologically active fragments of immunoglobulin molecules, namely, molecules that contain an antigen binding site. Immunoglobulin molecules can be of any type (for example, IgG, IgE, IgM, IgD, IgA and IgY), class (for example, $IgG_1$, $IgG_2$, $IgG_3$, $IgG_4$, $IgA_1$ and $IgA_2$) or subclass. For simplicity sake, an antibody against an analyte is frequently referred to as being either an "anti-analyte antibody", or merely an "analyte antibody" (e.g., an NGAL antibody).

### b) Renal Tubular Cell Injury

As used herein the expression "renal tubular cell injury" means a renal or kidney failure or dysfunction, either sudden (acute) or slowly declining over time (chronic), that can be triggered by a number of disease or disorder processes. Both acute and chronic forms of renal tubular cell injury can result in a life-threatening metabolic derangement.

### c) Acute Kidney Disease

An "acute renal tubular cell injury" means acute ischemic renal injury (IRI) or acute nephrotoxic renal injury (NRI). IRI includes but is not limited to ischemic injury and chronic ischemic injury, acute renal failure, acute glomerulonephritis, and acute tubulo-interstitial nephropathy. NRI toxicity includes but is not limited to, sepsis (infection), shock, trauma, kidney stones, kidney infection, drug toxicity, poisons or toxins, or after injection with a radiocontrast dye.

### d) Chronic Kidney Disease

The phrases "chronic renal tubular cell injury", "progressive renal disease", "chronic renal disease (CRD)", "chronic kidney disease (CKD)" as used interchangeably herein, include any kidney condition or dysfunction that occurs over a period of time, as opposed to a sudden event, to cause a gradual decrease of renal tubular cell function or worsening of renal tubular cell injury. One endpoint on the continuum of chronic renal disease is "chronic renal failure (CRF)". For example, chronic kidney disease or chronic renal injury as used interchangeably herein, includes, but is not limited to, conditions or dysfunctions caused by chronic infections, chronic inflammation, glomerulonephritides, vascular diseases, interstitial nephritis, drugs, toxins, trauma, renal stones, long standing hypertension, diabetes, congestive heart failure, nephropathy from sickle cell anemia and other blood dyscrasias, nephropathy related to hepatitis, HIV, parvovirus and BK virus (a human polyomavirus), cystic kidney diseases, congenital malformations, obstruction, malignancy, kidney disease of indeterminate causes, lupus nephritis, membranous glomerulonephritis, membranoproliferative glomerulonephritis, focal glomerular sclerosis, minimal change disease, cryoglobulinemia, Anti-Neutrophil Cytoplasmic Antibody (ANCA)-positive vasculitis, ANCA-negative vasculitis, amyloidosis, multiple myeloma, light chain deposition disease, complications of kidney transplant, chronic rejection of a kidney transplant, chronic allograft nephropathy, and the chronic effects of immunosuppressives. Preferably, chronic renal disease or chronic renal injury refers to chronic renal failure or chronic glomerulonephritis.

### e) Immunodiagnostic reagent

An "immunodiagnostic reagent" according to the present invention comprises one or more antibodies that specifically bind to a region of an NGAL protein. Immunodiagnostic reagents are as set forth in U.S. Provisional Application Serial Number 60/981,471 filed October 19, 2007.

### f) NGAL Polynucleotide and Polypeptide Sequences

NGAL polynucleotide and polypeptide sequences are as described in U.S. Provisional Application Serial Number 60/981,470 filed October 19, 2007. Generally, the NGAL can be any NGAL sequence, e.g., including that set forth as Genbank accession numbers Genpept CAA58127 (SEQ ID NO:1), AAB26529, XP_862322, XP_548441, P80108, P11672, X83006.1, X99133.1, CAA67574.1, BC033089.1, AAH33089.1, S75256.1, AD14168.1, JC2339, 1DFVA, 1DFVB, 1L6MA, 1L6MB, 1L6MC, 1NGLA, 1QQSA, 1X71A, 1X71B, 1X71C, 1X89A, 1X89B, 1X89C, 1X8UA, 1X8UB, and 1X8UC. NGAL polynucleotide and polypeptide (e.g., polyamino acid) sequences are as found in nature, based on sequences found in nature, isolated, synthetic, semi-synthetic, recombinant, or other. In one embodiment, the NGAL is human NGAL (also known as "hNGAL"). Unless specified otherwise, NGAL polypeptide sequences are numbered according to the mature human NGAL sequence minus the 20 residue amino acid signal peptide typically found in nature (and minus any other signal peptide sequence). When a signal peptide is present, it is numbered with negative numbers, e.g., as residues -1 to -20, with comparable numbering applied for the encoding polynucleotide sequence.

Likewise, an initial Met residue at the N-terminus of NGAL is present only in NGAL produced in prokaryotes (e.g., *E. coli*), or in synthetic (including semi-synthetic) or derived sequences, and not in NGAL produced in eukaryotes (e.g., mammalian cells, including human and yeast cells). Consequently, when present, an initial Met residue is counted herein as a negative number, e.g., as residue -1, with no similar numbering adjustment being made for the polynucleotide sequence in a prokaryotic versus eukaryotic background or expression system inasmuch as the polynucleotide sequence is replicated and transcribed the same in both backgrounds and the difference lies at the level of translation.

Accordingly, the disclosure herein encompasses a multitude of different NGAL polynucleotide and polypeptide sequences as present and/or produced in a prokaryotic and/or eukaryotic background (e.g., with consequent optimization for codon recognition). In sum, the sequences may or may not possess or encode: (a) a signal peptide; (b) an initiator Met residue present in the mature NGAL sequence at the N-terminus; (c) an initiator Met residue present at the start of a signal peptide that precedes the mature NGAL protein; and (d) other variations such as would be apparent to one skilled in the art.

Exemplary sequences include, but are not limited to, those as set forth herein: SEQ ID NO:1 (NGAL wild-type polypeptide including signal peptide); SEQ ID NO:2 (NGAL mutant polypeptide including signal peptide); SEQ ID NO: 10 (NGAL mutant polypeptide not including any signal peptide, and which can be preceded by a Met initiator residue when produced in prokaryotes and a Met initiator codon is present; however, there is no Met initiator residue when produced in eukaryotes, regardless of whether a Met initiator codon is present); SEQ ID NO:13 (NGAL wild-type polypeptide not including any signal peptide, and which can be preceded by a Met initiator residue when produced in prokaryotes and a Met initiator codon is present; however, there is no Met initiator residue when produced in eukaryotes, regardless of whether a Met initiator codon is present); SEQ ID NO:3 (NGAL wild-type polynucleotide sequence including that encoding a signal peptide); SEQ ID NO:4 (NGAL mutant polynucleotide including that encoding a signal peptide); SEQ ID NO:12 (NGAL mutant polynucleotide, synthetic or for eukaryotic expression, not including that encoding any signal peptide, but which optionally further can be preceded at the N-terminus either with or without a Met initiator codon, e.g., ATG); SEQ ID NO:9 (NGAL mutant polynucleotide, synthetic or for prokaryotic expression, not including that encoding any signal peptide, but which optionally further can be preceded at the N-terminus either with or without a Met initiator codon, e.g., ATG).

### g) Glycosylated Mammalian NGAL

Glycosylated mammalian NGAL (e.g., employed as an immunogen and/or assessing the binding of various antibodies) is as described in U.S. Provisional Application Serial Number 60/981,470 filed October 19, 2007.

Generally, as used herein, the phrases "oligosaccharide moiety" or "oligosaccharide molecule" as used interchangeably herein refers to a carbohydrate-containing molecule comprising one or more monosaccharide residues, capable of being attached to a polypeptide (to produce a glycosylated polypeptide, such as, for example; mammalian NGAL) by way of *in vivo* or *in vitro* glycosylation. Except where the number of oligosaccharide moieties attached to the polypeptide is expressly indicated, every reference to "oligosaccharide moiety" referred to herein is intended as a reference to one or more such moieties attached to a polypeptide. Preferably, the polypeptide to which said carbohydrate-containing molecule is capable of being attached is wild-type or mutant mammalian NGAL, i.e., to provide "glycosylated mammalian NGAL" as described further herein.

The term "*in vivo* glycosylation" is intended to mean any attachment of an oligosaccharide moiety occurring *in vivo*, for example, during posttranslational processing in a glycosylating cell used for expression of the polypeptide, for example, by way of N-linked and O-linked glycosylation. Usually, the N-glycosylated oligosaccharide-moiety has a common basic core structure composed of five monosaccharide residues, namely two N-acetylglucosamine residues and three mannose residues. The exact oligosaccharide structure depends, to a large extent, on the glycosylating organism in question and on the specific polypeptide.

The phrase "*in vitro* glycosylation" refers to a synthetic glycosylation performed *in vitro*, normally involving covalently linking an oligosaccharide moiety to an attachment group of a polypeptide, optionally using a cross-linking agent. *In vitro* glycosylation can be achieved by attaching chemically synthesized oligosaccharide structures to a polypeptide (such as, for example, mammalian NGAL) using a variety of different chemistries. For example, the chemistries that can be employed are those used for the attachment of polyethylene glycol (PEG) to proteins, wherein the oligosaccharide is linked to a functional group, optionally, via a short spacer. *In vitro* glycosylation can be carried out in a suitable buffer at a pH of about 4.0 to about 7.0 in protein concentrations of about 0.5 to about 2.0 mg/mL in a volume of about 0.02 to about 2.0 ml. Other *in vitro* glycosylation methods are described, for example in WO 87/05330, by Aplin et al., CRC Crit. Rev. Biochem. 259-306 (1981), by Lundblad et al. in Chemical Reagents for Protein Modification, CRC Press Inc., Boca Raton, Fla, Yan et al., Biochemistry, 23:3759-3765 (1982) and Doebber et al., J. Biol. Chem., 257:2193-2199(1982).

### h) Human NGAL fragment

A human NGAL fragment (e.g., employed as an immunogen, calibrator, control and/or for assessing the binding of various antibodies) is as described in U.S. Provisional Application Serial Number 60/981,470 filed October 19, 2007

Generally, as used herein, the term "human NGAL fragment" herein refers to a polypeptide that comprises a part that is less than the entirety of a mature human NGAL or NGAL including a signal peptide. In particular, a human NGAL fragment comprises from about 5 to about 178 or about 179 contiguous amino acids of SEQ ID NOS:1, 2, 10 or 13. In particular, a human NGAL fragment comprises from about 5 to about 170 contiguous amino acids of SEQ ID NOS: 1, 2, 10 or 13. In particular, a human NGAL fragment comprises at least about 5 contiguous amino acids of SEQ ID NO: 1, 2, 10 or 13, at least about 10 contiguous amino acids residues of SEQ ID NOS:1, 2, 10 or 13; at least about 15 contiguous amino acids residues of amino acids of SEQ ID NOS:1, 2, 10 or 13; at least about 20 contiguous amino acids residues of SEQ ID NOS:1, 2, 10 or 13; at least about 25 contiguous amino acids residues of SEQ ID NOS:1, 2, 10 or 13, at least about 30 contiguous amino acid residues of amino acids of SEQ ID NOS:1, 2, 10 or 13, at least about 35 contiguous amino acid residues of SEQ ID NOS:1, 2, 10 or 13, at least about 40 contiguous amino acid residues of SEQ ID NOS:1, 2, 10 or 13, at least about 45 contiguous amino acid residues of SEQ ID NOS:1, 2, 10 or 13, at least about 50 contiguous amino acid residues of SEQ ID NOS: 1, 2, 10 or 13, at least about 55 contiguous amino acid residues of SEQ ID NOS:1, 2, 10 or 13, at least about 60 contiguous amino acid residues of SEQ ID NOS:1, 2, 10 or 13, at least about 65 contiguous amino acid residues of SEQ ID NOS:1, 2, 10 or 13, at least about 70 contiguous amino acid residues of SEQ ID NOS:1, 2, 10 or 13, at least about 75 contiguous amino acid residues of SEQ ID NOS:1, 2, 10 or 13, at least about 80 contiguous amino acid residues of SEQ ID NOS:1, 2, 10 or 13, at least about 85 contiguous amino acid residues of SEQ ID NOS:1, 2, 10 or 13, at least about 90 contiguous amino acid residues of SEQ ID NOS:1, 2, 10 or 13, at least about 95 contiguous amino acid residues of SEQ ID NOS:1, 2, 10 or 13, at least about 100 contiguous amino acid residues of SEQ ID NOS:1, 2, 10 or 13, at least about 105 contiguous amino acid residues of SEQ ID NOS:1, 2, 10 or 13, at least about 110 contiguous amino acid residues of SEQ ID NOS:1, 2, 10 or 13, at least about 115 contiguous amino acid residues of SEQ ID NOS:1, 2, 10 or 13, at least about 120 contiguous amino acid residues of SEQ ID NOS: 1, 2, 10 or 13, at least about 125 contiguous amino acid residues of SEQ ID NOS:1, 2, 10 or 13, at least about 130 contiguous amino acid residues of SEQ ID NOS: 1, 2, 10 or 13, at least about 135 contiguous amino acid residues of SEQ ID NOS: 1, 2, 10 or 13, at least about 140 contiguous amino acid residues of SEQ ID NOS:1, 2, 10 or 13, at least about 145 contiguous amino acid residues of SEQ ID NOS:1, 2, 10 or 13, at least about 150 contiguous amino acid residues of SEQ ID NOS:1, 2, 10 or 13, at least about 160 contiguous amino acid residues of SEQ ID NOS:1, 2, 10 or 13, at least about 165 contiguous amino acid residues of SEQ ID NOS:1, 2, 10 or 13, at least about 170 contiguous amino acid residues of SEQ ID NOS:1, 2, 10 or 13 or at least about 175 contiguous amino acid residues of SEQ ID NOS:1, 2, 10 or 13.

Examples of human NGAL fragments contemplated by the present invention include, but are not limited to:

(a) a human NGAL fragment of at least about 7 contiguous amino acids which includes amino acid residues 112, 113, 114, 115, 116, 117 and 118 of SEQ ID NOS:1, 2, 10 or 13 (with the numbering of SEQ ID NO:1 and 2 beginning at the Gln residue of the mature sequence immediately following the signal peptide and any Met initiator residue, and the signal peptide and any Met initiator residue(s) being numbered in the negative, as previously described herein);

(b) a human NGAL fragment of at least about 8 contiguous amino acids which includes amino acid residues 112, 113, 114, 115, 116, 117, 118 and 119 of SEQ ID NOS:1, 2, 10 or 13 (with the numbering of SEQ ID NO:1 and 2 beginning at the Gln residue of the mature sequence immediately following the signal peptide and any Met initiator residue);

(c) a human NGAL fragment of at least about 36 contiguous amino acid which includes amino acid residues 112, 118 and 147 of SEQ ID NOS:1, 2, 10 or 13 (with the numbering of SEQ ID NO: 1 and 2 beginning at the Gln residue of the mature sequence immediately following the signal peptide and any Met initiator residue);

(d) a human NGAL fragment of at least about 95 contiguous amino acids which includes amino acid residues 15 and 109 of SEQ ID NOS:1, 2, 10 or 13 (with the numbering of SEQ ID NO:1 and 2 beginning at the Gln residue of the mature sequence immediately following the signal peptide and any Met initiator residue);

(e) a human NGAL fragment of at least about 144 contiguous amino acids which includes amino acid residues 15, 109 and 158 of SEQ ID NOS:1, 2, 10 or 13 (with the numbering of SEQ ID NO:1 and 2 beginning at the Gln residue of the mature sequence immediately following the signal peptide and any Met initiator residue);

(f) a human NGAL fragment of at least about 145 contiguous amino acids which includes amino acid residues 15, 109,158 and 159 of SEQ ID NOS:1, 2, 10 or 13 (with the numbering of SEQ ID NO:1 and 2 beginning at the Gln residue of the mature sequence immediately following the signal peptide and any Met initiator residue); or

(g) a human NGAL fragment of at least about 146 contiguous amino acids which includes amino acid residues 15, 109, 158, 159 and 160 of SEQ ID NOS:1, 2, 10 or 13 (with the numbering of SEQ ID NO:1 and 2 beginning at the Gln residue of the mature sequence immediately following the signal peptide and any Met initiator residue).

Optionally, such human NGAL fragments as described herein are encoded either in part or in the entirety by the corresponding sequences of SEQ ID NOS:3, 4 or 12. Along these lines, in one embodiment, the present invention provides an isolated, purified, or isolated and purified human NGAL polynucleotide comprising or consisting of the sequence of SEQ ID NOS:4 or 12.

### i) NGAL Hybrid

As used herein, the term "NGAL hybrid" or 'NGAL hybridoma" refers to a particular hybridoma clone or subclone (as specified) that produces an anti-NGAL antibody of interest. Generally, there may be some small variation in the affinity of antibodies produced by a hybridoma clone as compared to those from a subclone of the same type, e.g., reflecting purity of the clone. By comparison, it is well established that all hybridoma subclones originating from the same clone and further, that produce the anti-NGAL antibody of interest produce antibodies of identical sequence and/or identical structure. NGAL hybrids are set forth in U.S. Provisional Application Serial Number 60/981,471 filed October 19, 2007.

### j) Specificity

"Specificity" for the amount of human NGAL as used herein is defined as the converse of cross-reactivity for dimer (i.e., specificity is equal to 1/cross-reactivity), such that a specificity of about 75% is equivalent to a cross-reactivity of about 25%, a specificity of about 90% is equivalent to a cross-reactivity of about 10%, etc. For purposes herein, an assay that is about 75% specific for NGAL monomer demonstrates about 25% cross-reactivity with NGAL dimer. An alternative way of stating this is that an assay with about 75% specificity for monomer is about 4 times as specific for NGAL monomer as compared to NGAL dimer.

### k) Specific Binding

The term "specific binding" is defined herein as the preferential binding of one binding partner to another (e.g., two polypeptides, a polypeptide and nucleic acid molecule, or two nucleic acid molecules) at specific sites. The term "specifically binds" indicates that the binding preference (e.g., affinity) for the target molecule/sequence is at least 2-fold, more preferably at least 5-fold, and most preferably at least 10- or 20-fold over a non-specific target molecule (e.g. a random molecule lacking the specifically recognized site(s)).

### l) Binding Partner

A "binding partner," as used herein, is a member of a binding pair, i.e., a pair of molecules wherein one of the molecules binds to the second molecule. Binding partners that bind specifically are termed "specific binding partners." In addition to the antigen and antibody binding partners commonly used in immunoassays, other specific binding partners can include biotin and avidin, carbohydrates and lectins, complementary nucleotide sequences, effector and receptor molecules, cofactors and enzymes, enzyme inhibitors and enzymes, and the like. Furthermore, specific binding partners can include partner(s) that is/are analog(s) of the original specific binding partner, for example, an analyte-analog. Immunoreactive specific binding partners include antigens, antigen fragments, antibodies and antibody fragments, both monoclonal and polyclonal, and complexes thereof, including those formed by recombinant DNA methods.

### m) Epitope

As used herein, the term "epitope", "epitopes" or "epitopes of interest" refer to a site(s) on any molecule that is recognized and is capable of binding to a complementary site(s) on its specific binding partner. The molecule and specific binding partner are part of a specific binding pair. For example, an epitope can be a polypeptide, protein, hapten, carbohydrate antigen (such as, but not limited to, glycolipids, glycoproteins or lipopolysaccharides) or polysaccharide and its specific binding partner, can be, but is not limited to, an antibody.

In particular, an epitope refers to a particular region (composed of one or more amino acids) of an antigen, namely a protein to which an antibody binds. More specifically, an antigenic epitope is the area on protein surface that interacts with the complementary area (paratope) on the surface of the antibody binding domains. The epitope thus participates in electrostatic interactions, hydrophobic interactions and hydrogen bonding with the antibody and also contains residues responsible for the correct geometry of the surface, its malleability and structural dynamics. There are also buried "second sphere" residues that carry a strong supporting role for the antigenic epitope.

A particular type of epitope known as a "conformational epitope" or a "discontinuous epitope" is a type of epitope formed by residues that are sequentially discontinuous but close together in three-dimensional space.

**n) Binding Constants (e.g., $K_D$, $k_a$ and $k_d$)**

The terms "equilibrium dissociation constant" or "$K_D$", as used interchangeably herein, refer to the value obtained in a titration measurement at equilibrium, or by dividing the dissociation rate constant ($k_{off}$) by the association rate constant ($k_{on}$) The association rate constant, the dissociation rate constant and the equilibrium dissociation constant are used to represent the binding affinity of an antibody to an antigen.

The terms "relative affinity" or "relative $K_R$", can be defined as the binding avidity of antibody to antigen revealed using the same test method to measure antibody/antigen $K_D$ within a test population that includes antiserum test samples or uncloned hybrid test samples, thus providing relative affinity values rather than 'absolute' specificity data. (See, e.g., Immunology, 32:49 (1977) and Essential Immunology, Blackwell Scientific Publications, 7th edition, page 74 (1991)).

The term "association rate constant", "$k_{on}$" or "$k_a$" as used interchangeably herein, refers to the value indicating the binding rate of an antibody to its target antigen or the rate of complex formation between an antibody and antigen as shown by the equation below:

$$\text{Antibody (``Ab'') + Antigen (``Ag'')} \rightarrow \text{Ab-Ag.}$$

The term "dissociation rate constant", "$k_{off}$" or "$k_d$" as used interchangeably herein, refers to the value indicating the dissociation rate of an antibody from its target antigen or separation of Ab-Ag complex over time into free antibody and antigen as shown by the equation below:

$$\text{Ab + Ag} \leftarrow \text{Ab-Ag.}$$

Methods for determining association and dissociation rate constants are well known in the art. Using fluorescence-based techniques offers high sensitivity and the ability to examine samples in physiological buffers at equilibrium. Other experimental approaches and instruments such as a BIAcore® (biomolecular interaction analysis) assay can be used (e.g., instrument available from BIAcore International AB, a GE Healthcare company, Uppsala, Sweden). Additionally, a KinExA® (Kinetic Exclusion Assay) assay, available from Sapidyne Instruments (Boise, Idaho) can also be used.

**o) Subject**

As used herein, the terms "subject" and "patient" are used interchangeably irrespective of whether the subject has or is currently undergoing any form of treatment. As used herein, the terms "subject" and "subjects" refer to a mammal including, a non-primate (for example, a cow, pig, camel, llama, horse, goat, rabbit, sheep, hamsters, guinea pig, feline, canine, rat, and murine), a non-human primate (for example, a monkey, such as a cynomolgous monkey, chimpanzee, etc) and a human. Preferably, the subject is a human.

**p) Test Sample**

As used herein, the term "test sample" refers to a biological sample derived from serum, plasma, blood (including, but not limited to, whole blood), lymph, urine or other bodily fluids of a subject. The test sample can be prepared using routine techniques known to those skilled in the art. Preferably, the test sample is urine or blood.

**q) Pretreatment Reagent (e.g., lysis, precipitation and/or solubilization reagent)**

A pretreatment reagent used in a diagnostic assay as described herein is one that lyses any cells and/or solubilizes any analyte that are present in a test sample. Pretreatment is not necessary for all samples, as described further herein. Among other things, solubilizing the analyte (i.e., NGAL) entails release of the analyte from any endogenous binding proteins present the sample. A pretreatment reagent may be homogenous (not requiring a separation step) or heterogeneous (requiring a separation step). With use of a heterogenous pretreatment reagent there is removal of any precipitated analyte binding proteins from the test sample prior to proceeding to the next step of the assay. The pretreatment reagent optionally can comprise: (a) one or more solvents and salt, (b) one or more solvents, salt and detergent, (c) detergent, (d) detergent and salt, or (e) any reagent or combination of reagents appropriate for cell lysis and/or solubilization of analyte. Also, proteases, either alone or in combination with any other pretreament agents (e.g., solvents, detergents, salts, and the like) can be employed.

**r) Solid Phase**

A "solid phase," as used herein, refers to any material that is insoluble, or can be made insoluble by a subsequent reaction. The solid phase can be chosen for its intrinsic ability to attract and immobilize a capture agent. Alternatively, the solid phase can have affixed thereto a linking agent that has the ability to attract and immobilize the capture agent. The linking agent can, for example, include a charged substance that is oppositely charged with respect to the capture agent itself or to a charged substance conjugated to the capture agent. In general, the linking agent can be any binding partner (preferably specific) that is immobilized on (attached to) the solid phase and that has the ability to immobilize the capture agent through a binding reaction. The linking agent enables the indirect binding of the capture agent to a solid phase material before the perfomance of the assay or during the performance of the assay. The solid phase can, for example, be plastic, derivatized plastic, magnetic or non-magnetic metal, glass or silicon, including, for example, a test tube, microtiter well, sheet, bead, microparticle, chip, and other configurations known to those of ordinary skill in the art.

The terminology used herein is for the purpose of describing particular embodiments only and is not otherwise intended to be limiting.

**B. Glycosylated Mammalian NGAL**

Glycosylated mammalian NGAL employed in the context of the present disclosure (e.g., as a calibrator or control) is as described in U.S. Provisional Application Serial Number 60/981,470 filed October 19, 2007. Generally, the present invention contemplates use of mammalian NGAL of any type (e.g., isolated, recombinant, mutant, wild-type, synthetic, semi-synthetic, and the like), especially mammalian NGAL that optionally is glycosylated, and particularly human NGAL as set forth herein. Such mammalian NGAL is employed, e.g., as immunogen for making antibodies, and/or in assessing binding of such antibodies.

In one embodiment, the present disclosure relates to isolated glycosylated mammalian NGAL. More specifically, the present disclosure relates to glycosylated mammalian NGAL that contains at least one oligosaccharide molecule or moiety and up to ten (10) oligosaccharide molecules or moieties. The glycosylated mammalian NGAL of the present disclosure includes, but is not limited to, glycosylated canine NGAL, glycosylated feline NGAL, glycosylated rat NGAL, glycosylated murine NGAL, glycosylated horse NGAL, glycosylated non-human primate NGAL and glycosylated human NGAL. Preferably, the glycosylated mammalian NGAL is human NGAL Moreover, the glycosylated mammalian NGAL can be wild-type NGAL (namely, any wild-type mammalian NGAL, such as, but not limited to, wild-type canine NGAL, wild-type feline NGAL, wild-type rat NGAL, wild-type murine NGAL, wild-type horse NGAL, wild-type non-human primate NGAL or wild-type human NGAL). Preferably, the wild-type mammalian NGAL, is wild-type human NGAL having the amino acid sequence shown in SEQ ID NO:1 (including a signal peptide, and with the numbering of SEQ ID NO:1 beginning at the Gln residue of the mature sequence immediately following the signal peptide and any Met initiator residue) or SEQ ID NO:13 (not including a signal peptide). Alternatively, the glycosylated mammalian NGAL can be a glycosylated mutant mammalian NGAL that comprises an amino acid sequence comprising one or more amino acid substitutions, deletions or additions when compared to the corresponding amino acid sequence of the wild-type mammalian NGAL. For example, the glycosylated mammalian NGAL can be human NGAL wherein the amino acid sequence of the wild-type human NGAL (See, e.g., SEQ ID NOS: 1 or 13) contains at least one amino acid substitution. Specifically, at least one amino acid substitution can be made at amino acid residue 87 of SEQ ID NOS:1 or 13. Specifically, the cysteine at amino acid 87 shown in SEQ ID NOS:1 or 13 can be replaced with a serine (See, e.g., SEQ ID NOS:2 and 10). Other substitutions for amino acids other than serine or cysteine can be made, e.g., glycine or alanine. Moreover, other amino acid substitutions, deletions or additions other than the single amino acid substitution at amino acid 87 of SEQ ID NOS:1 or 13 can be made by those skilled in the art using routine experimentation.

The mammalian NGAL employed herein (e.g., optionally glycosylated) can be made using recombinant DNA technology, by chemical synthesis or by a combination of chemical synthesis and recombinant DNA technology. Specifically, a polynucleotide sequence encoding mammalian NGAL may be constructed by isolating or synthesizing a polynucleotide sequence encoding the mammalian NGAL of interest. As mentioned above, the mammalian NGAL (e.g.,

optionally glycosylated) can be a wild-type mammalian NGAL or can be a mutant mammalian NGAL containing one more amino acid substitutions, deletions or additions. Such amino acid substitutions, deletions or additions can be made using routine techniques known in the art, such as by mutagenesis (for example, using site-directed mutagenesis in accordance with well known methods, e.g., as described in Nelson and Long, Analytical Biochemistry 180:147-151 (1989), random mutagenesis, or shuffling).

The polynucleotide sequence encoding the mammalian NGAL of interest may be prepared by chemical synthesis, such as by using an oligonucleotide synthesizer, wherein oligonucleotides are designed based on the amino acid sequence of the desired mammalian NGAL (wild-type or mutant), and by preferably selecting those codons that are favored in the host cell in which the recombinant mammalian NGAL will be produced. For example, several small oligonucleotides coding for portions of the desired mammalian NGAL may be synthesized and assembled by polymerase chain reaction (PCR), ligation or ligation chain reaction (LCR). The individual oligonucleotides typically contain 5' or 3' overhangs for complementary assembly.

Once assembled (such as by synthesis, site-directed mutagenesis or another method), the polynucleotide sequence encoding the mammalian NGAL of interest may be inserted into a recombinant vector and operably linked to any control sequences necessary for expression of thereof in the desired transformed host cell.

Although not all vectors and expression control sequences may function equally well to express a polynucleotide sequence of interest and not all hosts function equally well with the same expression system, it is believed that those skilled in the art will be able to easily make a selection among these vectors, expression control sequences, optimized codons, and hosts for use in the present invention without any undue experimentation. For example, in selecting a vector, the host must be considered because the vector must be able to replicate in it or be able to integrate into the chromosome. The vector's copy number, the ability to control that copy number, and the expression of any other proteins encoded by the vector, such as antibiotic markers, should also be considered. In selecting an expression control sequence, a variety of factors can also be considered. These include, but are not limited to, the relative strength of the sequence, its controllability, and its compatibility with the polynucleotide sequence encoding the mammalian NGAL, particularly as regards potential secondary structures. Hosts should be selected by consideration of their compatibility with the chosen vector, their codon usage, their secretion characteristics, their ability to fold the polypeptide correctly, their fermentation or culture requirements, their ability (or lack thereof) to glycosylate the protein, and the ease of purification of the products coded for by the nucleotide sequence, etc.

The recombinant vector may be an autonomously replicating vector, namely, a vector existing as an extrachromosomal entity, the replication of which is independent of chromosomal replication (such as a plasmid). Alternatively, the vector can be one which, when introduced into a host cell, is integrated into the host cell genome and replicated together with the chromosome(s) into which it has been integrated.

The vector is preferably an expression vector, in which the polynucleotide sequence encoding the mammalian NGAL is operably linked to additional segments required for transcription of the polynucleotide sequence. The vector is typically derived from plasmid or viral DNA. A number of suitable expression vectors for expression in the host cells mentioned herein are commercially available or described in the literature. Useful expression vectors for eukaryotic hosts, include, but are not limited to, vectors comprising expression control sequences from SV40, bovine papilloma virus, adenovirus and cytomegalovirus. Specific vectors include, pCDNA3.1 (+)\Hyg (Invitrogen Corp., Carlsbad, CA) and pCI-neo (Stratagene, La Jolla, CA, USA). Examples of expression vectors for use in yeast cells include, but are not limited to, the 2μ plasmid and derivatives thereof, the POT1 vector (See, U.S. Patent No. 4,931,373), the pJSO37 vector (described in Okkels, Ann. New YorkAcad. Sci., 782:202-207, (1996)) and pPICZ A, B or C (Invitrogen Corp., Carlsbad, CA). Examples of expression vectors for use in insect cells include, but are not limited to, pVL941, pBG311 (Cate et al., "Isolation of the Bovine and Human Genes for Mullerian Inhibiting Substance And Expression of the Human Gene In Animal Cells" Cell, 45:685-698 (1986), pBluebac 4.5 and pMelbac (both of which are available from Invitrogen Corp., Carlsbad, CA.). A preferred vector for use in the invention is pJV (available from Abbott Laboratories, Abbott Bioresearch Center, Worcester, MA).

Other vectors that can be used allow the polynucleotide sequence encoding the mammalian NGAL to be amplified in copy number. Such amplifiable vectors are well known in the art. These vectors include, but are not limited to, those vector that can be amplified by DHFR amplification (See, for example, Kaufinan, U.S. Patent No. 4,470,461, Kaufinan et al., "Construction Of A Modular Dihydrofolate Reductase cDNA Gene: Analysis Of Signals Utilized For Efficient Expression" Mol. Cell. Biol., 2:1304-1319 (1982)) and glutamine synthetase (GS) amplification (See, for example, U.S. Patent No. 5,122,464 and EP Patent Application 0 338,841).

The recombinant vector may further comprise a DNA sequence enabling the vector to replicate in the host cell in question. An example of such a sequence (when the host cell is a mammalian cell) is the SV40 origin of replication. When the host cell is a yeast cell, suitable sequences enabling the vector to replicate are the yeast plasmid 2μ replication genes REP 1-3 and origin of replication.

The vector may also comprise a selectable marker, namely, a gene or polynucleotide, the product of which complements a defect in the host cell, such as the gene coding for dihydrofolate reductase (DHFR) or the *Schizosac-*

*charomyces pombe* TPI gene (See, P. R. Russell, Gene, 40: 125-130 (1985)), or one which confers resistance to a drug, such as, ampicillin, kanamycin, tetracycline, chloramphenicol, neomycin, hygromycin or methotrexate. For filamentous fungi, selectable markers include, but are not limited to, amdS, pyrG, arcB, niaD and sC.

As used herein, the phrase "control sequences" refers to any components, which are necessary or advantageous for the expression of mammalian NGAL. Each control sequence may be native or foreign to the nucleic acid sequence encoding the mammalian NGAL. Such control sequences include, but are not limited to, a leader, polyadenylation sequence, propeptide sequence, promoter, enhancer or upstream activating sequence, signal peptide sequence and transcription terminator. At a minimum, the control sequences include at least one promoter operably linked to the polynucleotide sequence encoding the mammalian NGAL.

As used herein, the phrase "operably linked" refers to the covalent joining of two or more polynucleotide sequences, by means of enzymatic ligation or otherwise, in a configuration relative to one another such that the normal function of the sequences can be performed. For example, a polynucleotide sequence encoding a presequence or secretory leader is operably linked to a polynucleotide sequence for a polypeptide if it is expressed as a preprotein that participates in the secretion of the polypeptide: a promoter or enhancer is operably linked to a coding sequence if it affects the transcription of the sequence; a ribosome binding site is operably linked to a coding sequence if it is positioned so as to facilitate translation. Generally, "operably linked" means that the polynucleotide sequences being linked are contiguous and, in the case of a secretory leader, contiguous and in reading phase. Linking is accomplished by ligation at convenient restriction sites. If such sites do not exist, then synthetic oligonucleotides adaptors or linkers are used, in conjunction with standard recombinant DNA methods.

A wide variety of expression control sequences may be used in the present disclosure. Such useful expression control sequences include the expression control sequences associated with structural genes of the foregoing expression vectors as well as any sequence known to control the expression of genes of prokaryotic or eukaryotic cells or their viruses, and various combinations thereof. Examples of suitable control sequences for directing transcription in mammalian cells include the early and late promoters of SV40 and adenovirus, for example, the adenovirus 2 major late promoter, the MT-I (metallothionein gene) promoter, the human cytomegalovirus immediate-early gene promoter (CMV), the human elongation factor $1\alpha$ (EF-$1\alpha$) promoter, the *Drosophila* minimal heat shock protein 70 promoter, the Rous Sarcoma Virus (RSV) promoter, the human ubiquitin C (UbC) promoter, the human growth hormone terminator, SV40 or adenovirus Elb region polyadenylation signals and the Kozak consensus sequence (Kozak, J Mol Biol., 196:947-50 (1987)).

In order to improve expression in mammalian cells a synthetic intron may be inserted in the 5' untranslated region of the polynucleotide sequence encoding the mammalian NGAL. An example of a synthetic intron is the synthetic intron from the plasmid pCI-Neo (available from Promega Corporation, WI, USA).

Examples of suitable control sequences for directing transcription in insect cells include, but are not limited. to, the polyhedrin promoter, the P10 promoter, the baculovirus immediate early gene 1 promoter and the baculovirus 39K delayed-early gene promoter and the SV40 polyadenylation sequence.

Examples of suitable control sequences for use in yeast host cells include the promoters of the yeast $\alpha$-mating system, the yeast triose phosphate isomerase (TPI) promoter, promoters from yeast glycolytic genes or alcohol dehydrogenase genes, the ADH2-4c promoter and the inducible GAL promoter.

Examples of suitable control sequences for use in filamentous fungal host cells include the ADH3 promoter and terminator, a promoter derived from the genes encoding *Aspergillus oryzae* TAKA amylase triose phosphate isomerase or alkaline protease, an *A. niger* $\alpha$-amylase, *A. niger* or *A. nidulas* glucoamylase, *A. nidulans* acetamidase, *Rhizomucor miehei* aspartic proteinase or lipase, the TPII terminator and the ADH3 terminator.

The polynucleotide sequence encoding the mammalian NGAL may or may not also include a polynucleotide sequence that encodes a signal peptide. The signal peptide is present when the mammalian NGAL is to be secreted from the cells in which it is expressed. Such signal peptide, if present, should be one recognized by the cell chosen for expression of the polypeptide. The signal peptide may be homologous (for example, it may be that normally associated with the mammalian NGAL of interest) or heterologous (namely, originating from another source than the mammalian NGAL of interest) to the mammalian NGAL of interest or may be homologous or heterologous to the host cell, namely, be a signal peptide normally expressed from the host cell or one which is not normally expressed from the host cell. Accordingly, the signal peptide may be prokaryotic, for example, derived from a bacterium, or eukaryotic, for example, derived from a mammalian, or insect, filamentous fungal or yeast cell.

The presence or absence of a signal peptide will, for example, depend on the expression host cell used for the production of the mammalian NGAL. For use in filamentous fungi, the signal peptide may conveniently be derived from a gene encoding an *Aspergillus sp.* amylase or glucoamylase, a gene encoding a *Rhizomucor miehei* lipase or protease or a *Humicola lanuginosa* lipase. For use in insect cells, the signal peptide may be derived from an insect gene (See, WO 90/05783), such as the lepidopteran *Manduca sexta* adipokinetic hormone precursor, (See, U.S. Patent No. 5,023,328), the honeybee melittin (Invitrogen Corp., Carlsbad, CA), ecdysteroid UDP glucosyltransferase (egt) (Murphy et al., Protein Expression and Purification 4: 349-357 (1993), or human pancreatic lipase (hpl) (Methods in Enzymology, 284:262-272 (1997)).

Specific examples of signal peptides for use in mammalian cells include murine Ig kappa light chain signal peptide (Coloma, M, J. Imm. Methods, 152:89-104 (1992)). For use in yeast cells suitable signal peptides include the α-factor signal peptide from S. *cerevisiae* (See, U.S. Patent No. 4,870,008), the signal peptide of mouse salivary amylase (See, O. Hagenbuchle et al., Nature, 289:643-646 (1981)), a modified carboxypeptidase signal peptide (See, L. A. Valls et al., Cell, 48:887-897 (1987)), the yeast BAR1 signal peptide (See, WO 87/02670), and the yeast aspartic protease 3 (YAP3) signal peptide (See, M. Egel-Mitani et al., Yeast, 6:127-137 (1990)).

Any suitable host may be used to produce the glycosylated mammalian NGAL of the present disclosure, including bacteria, fungi (including yeasts), plant, insect mammal or other appropriate animal cells or cell lines, as well as non-human transgenic animals or plants. When a non-glycosylating organism such as *E. coli* is used, the expression in *E. coli* is preferably followed by suitable *in vitro* glycosylation in order to produce the glycosylated mammalian NGAL of the present disclosure.

Examples of bacterial host cells include, but are not limited to, gram positive bacteria such as strains of *Bacillus*, for example, *B. brevis* or *B. subtilis, Pseudomonas* or *Streptomyces,* or gram negative bacteria, such as strains of *E. coli.* The introduction of a vector into a bacterial host cell may, for instance, be effected by protoplast transformation (See, for example, Chang et al.. Molecular General Genetics, 168:111-115 (1979)), using competent cells (See, for example, Young et al., Journal of Bacteriology, 81:823-829 (1961)), or Dubnau et al., Journal of molecular Biology, 56: 209-221 (1971)), electroporation (See, for example, Shigekawa et al., Biotechniques, 6:742-751 (1988)), or conjugation (See, for example, Koehler et al., Journal of Bacteriology, 169:5771-5278 (1987)).

Examples of suitable filamentous fungal host cells include, but are not limited to, strains of *Aspergillus*, for example, *A. oryzae, A. niger*, or *A. nidulans, Fusarium* or *Trichoderma.* Fungal cells may be transformed by a process involving protoplast formation, transformation of the protoplasts, and regeneration of the cell wall using techniques known to those skilled in the art. Suitable procedures for transformation of *Aspergillus* host cells are described in EP Patent Application 238 023 and U.S. Patent No. 5,679,543. Suitable methods for transforming *Fusarium* species are described by Malardier et al., Gene, 78:147-156 (1989) and WO 96/00787. Yeast may be transformed using the procedures described by Becker and Guarente, In Abelson, J. N. and Simon, M. I., editors, Guide to Yeast Genetics and Molecular Biology, Methods in Enzymology, Volume 194, pp 182-187, Academic Press, Inc., New York; Ito et al, Journal of Bacteriology, 153:163 (1983); and Hinnen et al., Proceedings of the National Academy of Sciences USA, 75:1920 (1978).

Preferably, the mammalian NGAL employed by the present invention is glycosylated *in vivo*. When the mammalian NGAL is to be glycosylated *in vivo*, the host cell is selected from a group of host cells capable of generating the desired glycosylation of the mammalian NGAL. Thugs, the host cell may be selected from a yeast cell, insect cell, or mammalian cell.

Examples of suitable yeast host cells include strains of *Saccharomyces*, for example, *S. cerevisiae, Schizosaccharomyces, Klyveromyces, Pichia*, such as *P. pastoris* or *P. methanolica, Hansenula*, such as *H. polymorpha* or *yarrowia*. Methods for transforming yeast cells with heterologous polynucleotides and producing heterologous polypeptides therefrom are disclosed by Clontech Laboratories, Inc, Palo Alto, CA, USA (in the product protocol for the Yeastmaker™ Yeast Tranformation System Kit), and by Reeves et al., FEMS Microbiology Letters, 99:193-198 (1992), Manivasakam et al., Nucleic Acids Research, 21:4414-4415 (1993) and Ganeva et al., FEMS Microbiology Letters, 121:159-164 (1994).

Examples of suitable insect host cells include, but are not limited to, a *Lepidoptora* cell line, such as *Spodoptera frugiperda* (Sf9 or Sf21) or *Trichoplusia ni* cells (High Five) (See, U.S. Patent No. 5,077,214). Transformation of insect cells and production of heterologous polypeptides are well known to those skilled in the art.

Examples of suitable mammalian host cells include Chinese hamster ovary (CHO) cell lines, Green Monkey cell lines (COS), mouse cells (for example, NS/O), Baby Hamster Kidney (BHK) cell lines, human cells (such as, human embryonic kidney cells (for example, HEK293 (ATCC Accession No. CRL-1573))) and plant cells in tissue culture. Preferably, the mammalian host cells are CHO cell lines and HEK293 cell lines. Another preferred host cell is the B3 cell line (e.g., Abbott Laboratories, Abbott Bioresearch Center, Worcester, MA), or another dihydrofolate reductase deficient (DHFR⁻) CHO cell line (e.g., available from Invitrogen Corp., Carlsbad, CA). In one aspect, the present invention employs a CHO cell line which produces glycosylated human wild-type NGAL (namely, that which has the amino acid sequence of SEQ ID NOS:1 or 13), wherein the CHO cell line has been deposited with American Type Culture Collection (ATCC) on November 21, 2006 and received ATCC Accession No. PTA-8020. Preferably, the wild-type human NGAL produced by the CHO cell line having ATCC Accession No. PTA-8020 has a molecular weight of about 25 kilodaltons (kDa). In another aspect, the present invention employs a CHO cell line which produces glycosylated mutant human NGAL. Preferably, the Glycosylated mutant human NGAL comprises an amino acid substitution at the amino acid corresponding to amino acid 87 of the amino acid sequence of wild-type human NGAL (namely, SEQ ID NOS: 1 or 13). More preferably, the amino acid substitution is the replacement of a cysteine with a serine (See, SEQ ID NOS:2 or 10). Most preferably, the CHO cell line is a CHO cell line that has been deposited with the ATCC on January 23, 2007 and received ATCC Accession No. PTA-8168. The CHO cell line having ATCC Accession No. PTA-8168 produces a glycosylated mutant human NGAL comprising an amino acid sequence of SEQ ID NOS:2 or 10. In yet another aspect, the present invention employs an isolated mutant glycosylated human NGAL comprising the amino acid sequence of SEQ ID NOS:2 or 10.

Methods for introducing exogenous polynucleotides into mammalian host cells include calcium phosphate-mediated transfection, electroporation, DEAE-dextran mediated transfection, liposome-mediated transfection, viral vectors and the transfection method described by Life Technologies Ltd, Paisley, UK using Lipofectamine™ 2000. These methods are well known in the art and are described, for example by Ausbel et al. (eds.) Current Protocols in Molecular Biology John Wiley & Sons, New York, USA (1996). The cultivation of mammalian cells are conducted according to established methods, e.g. as disclosed in Jenkins, Ed., Animal Cell Biotechnology, Methods and Protocols, Human Press Inc. Totowa, N.J., USA (1999) and Harrison and Rae General Techniques of Cell Culture, Cambridge University Press (1997).

In the production methods, cells are cultivated in a nutrient medium suitable for production of the mammalian NGAL using methods known in the art. For example, cells are cultivated by shake flask cultivation, small-scale or large-scale fermentation (including continuous, batch, fed-batch, or solid state fermentations) in laboratory or industrial fermenters performed in a suitable medium and under conditions allowing the glycosylated mammalian NGAL to be expressed and/or isolated. The cultivation takes place in a suitable nutrient medium comprising carbon and nitrogen sources and inorganic salts, using procedures known in the art. Suitable media are available from Commercial suppliers or may be prepared according to published compositions (e.g., in catalogues of the American Type Culture Collection). If the glycosylated mammalian NGAL is secreted into the nutrient medium, the mammalian NGAL can be recovered directly from the medium. If the mammalian NGAL is not secreted, it can be recovered from cell lysates.

The resulting mammalian NGAL may be recovered by methods known in the art. For example, the mammalian NGAL may be recovered from the nutrient medium by conventional procedures including, but not limited to, centrifugation, filtration, extraction, spray drying, evaporation, or precipitation.

The mammalian NGAL may be purified by a variety of procedures known in the art including, but not limited to, chromatography (such as, but not limited to, ion exchange, affinity, hydrophobic, chromatofocusing, and size exclusion), electrophoretic procedures (such as, but not limited to, preparative isoelectric focusing), differential solubility (such as, but not limited to, ammonium sulfate precipitation), SDS-PAGE, or extraction (See, for example, J-C Janson and Lars Ryden, editors, Protein Purification. VCH Publishers, New York (1989)).

The glycosylated mammalian NGAL (wild-type and mutant) described herein can be used for a variety of different purposes and in a variety of different ways. Specifically, the glycosylated mammalian NGAL described herein can be used as one or more calibrators, one or more controls or as a combination of one or more calibrators or controls in an assay, preferably, an immunoassay, for detecting mammalian NGAL in a test sample. Glycosylated mammalian NGAL (wild-type and mutant) is part of the subject matter of U.S. Provisional Application Serial Number 60/981,470 filed October 19, 2007. Preferably, the glycosylated mammalian NGAL comprises the amino acid sequence of SEQ ID NOS: 1 or 13. Alternatively, the glycosylated mammalian NGAL comprises the amino acid sequence of SEQ ID NOS:2 or 10.

For example, the glycosylated mammalian NGAL described herein can be used to improve the methods for detecting the presence of mammalian NGAL in a test sample. The exact steps of the method and the order of these steps for detecting mammalian NGAL in a test sample are not critical. Rather, the improvement in the method involves the use of the glycosylated mammalian NGAL described herein as one or more calibrators, one or more controls or as a combination of one or more calibrators and controls. For example, such a method might comprise the steps of:

(a) contacting a test sample suspected of containing mammalian NGAL with at least one antibody specific for the mammalian NGAL (such as, but not limited to, a capture antibody) for a time and under conditions that allow for the formation of a mammalian NGAL/antibody complex (such as a mammalian antibody/capture antibody complex); and
(b) detecting any mammalian NGAUantibody complex formed (such as by adding a conjugate) as indicating the presence of the mammalian NGAL antigen,

wherein the method employs as at least one calibrator, at least one control, or as a combination of at least one calibrator or at least one control, at least one glycosylated mammalian NGAL described herein (such as a glycosylated mammalian NGAL having the amino acid sequence of SEQ ID NO:1, SEQ ID NO:2, SEQ ID NO:10, or SEQ ID NO:13 or a combination of SEQ ID NOS:1, 2, 10 or 13 (namely, one used as a calibrator and one as a control, etc)).

One primary advantage for using mutant NGAL (e.g., as set forth in SEQ ID NOS:2 or 10), optionally glycosylated, is that calibrators made with wild-type NGAL could slowly form dimers over time. Because, as described in the examples herein traditional NGAL assays detect monomers better than dimers, this will be perceived as a loss of monomers (i.e., as an instability). The calibration curve will shift due to this, leading to decreased accuracy.

The mammalian NGAL (e.g., glycosylated mammalian NGAL) also optionally can be employed in kits for detecting the presence of NGAL in a sample as described herein.

Furthermore, the mammalian NGAL can be employed as immunogen to immunize animals for antibody production, e.g., where the animal can be a murine, rabbit, chicken, rat, sheep, goat, shark, camel, horse, feline, canine, non-human primate, human or other animal. In one embodiment, the immunogen comprises glycosylated mammalian NGAL, especially glycosylated human NGAL comprising the sequence of SEQ ID NO: 1, 2, 10 or 13. In another embodiment, the mammalian NGAL is that of a canine, feline, rat, murine, horse, non-human primate, human, or other mammal.

### C. Human NGAL Antibodies

Antibodies directed against NGAL polypeptides, and methods of making such antibodies using NGAL polypeptides are described in U.S. Provisional Application Serial Number 60/981,471 filed October 19, 2007. Such antibodies are further described herein in the context of their employ in the assays according to the invention. The present invention employs antibodies that specifically bind to wild-type human NGAL (namely, SEQ ID NOS:1 or 13) or human NGAL fragment. The antibodies also optionally bind to human NGAL wherein the amino acid sequence contains at least one amino acid substitution of the wild-type sequence (SEQ ID NOS:1 or 13) so as to comprise a mutant or non-native sequence (e.g., SEQ ID NOS:2 or 10),

In particular, in one aspect, the present invention employs isolated antibodies that bind to an epitope, e. g., a conformational epitope comprising the noncontiguous amino acid residues 112, 118 and 147 of wild-type human NGAL (namely, SEQ ID NOS:1 or 13; with the numbering of SEQ ID NO:1 beginning at the Gln residue of the mature sequence immediately following the signal peptide and any Met initiator residue). In another aspect, the present invention employs isolated antibodies that bind to a conformational epitope comprising amino acid residues 112, 118 and 147 of human NGAL (namely, SEQ ID NOS:1, 2, 10 or 13) and at least one (1) additional amino acid of human NGAL protein, wherein the additional amino acid is amino acid residue 117 or 119 of wild-type human NGAL (namely, SEQ ID NOS:1 or 13). In yet another aspect, the present invention employs isolated antibodies that bind to a conformational epitope comprising amino acid residues 112, 117, 118,119 and 147 of wild-type human NGAL (namely, SEQ ID NOS:1 or 13).

In another aspect, the present invention relates to the use of an isolated antibody that specifically binds to wild-type human NGAL, wherein the antibody has a variable heavy domain region comprising an amino acid sequence of SEQ ID NO:5.

In another aspect, the present invention relates to the use of an isolated antibody that specifically binds to wild-type human NGAL, wherein the antibody has a variable heavy domain region comprising an amino acid sequence of SEQ ID NO:5 and further wherein the antibody binds to: (1) amino acid residues 112, 118 and 147 of will-type human NGAL protein (namely, SEQ ID NOS:1 or 13); (2) amino acid residues 112, 118 and 147 of human NGAL protein (namely, SEQ ID NOS:1, 2, 10 or 13) and at least one additional amino acid of wild-type human NGAL protein, wherein the additional amino acid is amino acid residue 117 of 119 of wild-type human NGAL (namely, SEQ ID NOS:1 or 13); or (3) to a conformational epitope comprising amino acid residues 112, 117, 118, 119 and 147 of wild-type human NGAL (namely, SEQ ID NOS:1 or 13).

In another aspect, the present invention relates to the use of an isolated antibody that specifically binds to wild-type human NGAL, wherein the antibody has a variable light domain region comprising an amino acid sequence of SEQ ID NO:6.

In another aspect, the present invention relates to the use of an isolated antibody that specifically binds to wild-type human NGAL, wherein the antibody has a variable light domain region comprising an amino acid sequence of SEQ ID NO:6 and further wherein the antibody binds to: (1) amino acid residues 112, 118 and 147 of wild-type human NGAL protein (namely, SEQ ID NOS:1 or 13); (2) amino acid residues 112, 118 and 147 of human NGAL protein (namely, SEQ ID NOS:1, 2, 10 or 13) and at least one additional amino acid of wild-type human NGAL protein, wherein the additional amino acid is amino acid residue 117 of 119 of wild-type human NGAL (namely, SEQ ID NOS:1 or 13); or (3) to a conformational epitope comprising amino acid residues 112, 117,118, 119 and 147 of wild-type human NGAL (namely, SEQ ID NOS:1 or 13).

In another aspect, the present invention relates to the use of an isolated antibody that specifically binds to wild-type human NGAL, wherein the antibody has a variable heavy domain region comprising an amino acid sequence of SEQ ID NO:5 and a variable light domain region comprising an amino acid sequence of SEQ ID NO:6,

In another aspect, the present invention relates to the use of an isolated antibody that specifically binds to wild-type human NGAL, wherein the antibody has a variable heavy domain region comprising an amino acid sequence of SEQ ID NO:5 and a variable light domain region comprising an amino acid sequence of SEQ ID NO:6 and further wherein the antibody binds to: (1) amino acid residues 112, 118 and 147 of wild-type human NGAL protein (namely, SEQ ID NOS:1 or 13); (2) amino acid residues 112, 118 and 147 of human NGAL protein (namely, SEQ ID NOS:1, 2, 10 or 13) and at least one additional amino acid of wild-type human NGAL protein, wherein the additional amino acid is amino acid residue 117 of 119 of wild-type human NGAL (namely, SEQ ID NOS:1 or 13): or (3) to a conformational epitope comprising amino acid residues 112, 117, 118, 119 and 147 of wild-type human NGAL (namely, SEQ ID NOS:1 or 13).

In yet another aspect, the present invention relates to the use of antibodies as produced by a murine hybridoma cell line 1-2322-455 having ATCC Accession No. PTA-8024, deposited on November 21, 2006. The antibody produced by murine hybridoma cell line 1-2322-455 can bind to: (1) amino acid residues 112, 118 and 147 of wild-type human NGAL protein (namely, SEQ ID NOS:1 or 13); (2) amino acid residues 112, 118 and 147 of human NGAL protein (namely, SEQ ID NOS:1, 2, 10 or 13) and at least one additional amino acid of human NGAL protein, wherein the additional amino acid is amino acid residue 117 of 119 of wild-type human NGAL (namely, SEQ ID NOS:1 or 13); or (3) to a conformational epitope comprising amino acid residues 112, 117, 118, 119 and 147 of wild-type human NGAL (namely, SEQ ID NOS: 1 or 13). Murine hybridoma cell line 1-2322-455 has a variable heavy domain comprising the amino acid sequence of SEQ ID NO:5 and a variable light domain comprising the amino acid sequence of SEQ ID NO:6.

In yet another aspect, the present invention relates to the use of an isolated antibody that specifically binds to wild-type human NGAL, wherein the antibody has a variable heavy domain region comprising an amino acid sequence of SEQ ID NO:7.

In yet another aspect, the present invention relates to the use of an isolated antibody that specifically binds to wild-type human NGAL, wherein the antibody has a variable heavy domain region comprising an amino acid sequence of SEQ ID NO:7 and further wherein the antibody binds to (1) amino acid residues 15 and 109 of wild-type human NGAL protein (namely, SEQ ID NOS:1 or 13); (2) amino acid residues 15 and 109 of wild-type human NGAL protein (namely, SEQ ID NOS:1 or 13) and at least one additional amino acid of wild-type human NGAL protein, wherein the additional amino acid is amino acid residue 158, 159 or 160 of wild-type human NGAL (namely, SEQ ID NOS:1 or 13); or (3) a conformational epitope comprising amino acid residues 15, 109, 158, 159 or 160 of wild-type human NGAL (namely, SEQ ID NOS:1 or 13).

In yet another aspect, the present invention relates to the use of an isolated antibody that specifically binds to wild-type human NGAL, wherein the antibody has a variable light domain region comprising an amino acid sequence of SEQ ID NO:8.

In yet another aspect, the present invention relates to the use of an isolated antibody that specifically binds to wild-type human NGAL, wherein the antibody has a variable light domain region comprising an amino acid sequence of SEQ ID NO:8 and further wherein the antibody binds to (1) amino acid residues 15 and 109 of wild-type human NGAL protein (namely, SEQ ID NOS:1 or 13); (2) amino acid residues 15 and 109 of wild-type human NGAL protein (namely, SEQ ID NOS:1 or 13) and at least one additional amino acid of wild-type human NGAL protein, wherein the additional amino acid is amino acid residue 158, 159 or 160 of wild-type human NGAL (namely, SEQ ID NOS:1 or 13); or (3) to a conformational epitope comprising amino acid residues 15, 109, 158, 159 or 160 of wild-type human NGAL (namely, SEQ ID NOS:1 or 13).

In another aspect, the present invention relates to the use of an isolated antibody that specifically binds to wild-type human NGAL, wherein the antibody has a variable heavy domain region comprising an amino acid sequence of SEQ ID NO:7 and a variable light domain region comprising an amino acid sequence of SEQ ID NO:8.

In another aspect, the present invention relates to the use of an isolated antibody that specifically binds to wild-type human NGAL, wherein the antibody has a variable heavy domain region comprising an amino acid sequence of SEQ ID NO:7 and a variable light domain region comprising an amino acid sequence of SEQ ID NO:8 and further wherein the antibody binds to: (1) amino acid residues 15 and 109 of wild-type human NGAL protein (namely, SEQ ID NOS:1 or 13); (2) amino acid residues 15 and 109 of wild-type human NGAL protein (namely, SEQ ID NOS:1 or 13) and at least one additional amino acid of wild-type human NGAL protein, wherein the additional amino acid is amino acid residue 158, 159 or 160 of wild-type human NGAL (namely, SEQ ID NOS:1 or 13); or (3) to a conformational epitope comprising amino acid residues 15, 109, 158, 159 or 160 of wild-type human NGAL (namely, SEQ ID NOS:1 or 13).

In yet another aspect, the present invention relates to the use of an antibody produced by murine hybridoma cell line 1-903-430 having ATCC Accession No. PTA-8026, deposited on November 21, 2006. The antibody produced by murine hybridoma cell line 1-903-430 can bind to: (1) amino acid residues 15 and 109 of wild-type human NGAL protein (namely, SEQ ID NOS:1 or 13); (2) amino acid residues 15 and 109 of wild-type human NGAL protein (namely, SEQ ID NOS:1 or 13) and at least one additional amino acid of wild-type human NGAL protein, wherein the additional amino acid is amino acid residue 158, 159 or 160 of wild-type human NGAL (namely, SEQ ID NOS:1 or 13); or (3) to a conformational epitope comprising amino acid residues 15, 109, 158, 159 or 160 of wild-type human NGAL (namely, SEQ ID NOS:1 or 13). Murine hybridoma cell line 1-903-430 has a variable heavy domain comprising the amino acid sequence of SEQ ID NO:7 and a variable light domain comprising the amino acid sequence of SEQ ID NO:8.

In still yet another embodiment, the present invention relates to the use of an isolated antibody that specifically binds to a human NGAL protein as set forth in SEQ ID NOS:1, 2, 10 or 13 (especially as set forth in SEQ ID NOS: 10 or 13), wherein as a result of adding the antibody to the human NGAL protein (generally done in excess, particularly stoichiometric excess), the antibody causes as compared to when the antibody is not added,

(1) a perturbation of from about 0.05 ppm to about 1.0 ppm in a $^1$H resonance position, particularly from about 0.04 ppm to about 0.06 ppm, especially of about 0.05 ppm in a $^1$H resonance position,
(2) a perturbation of from about 0.3 ppm to about 3.0 ppm in a $^{15}$N resonance position, particularly of from about 0.1 ppm to about 2.0 ppm, especially of about 0.1 ppm, about 0.3 ppm, or about 0.6 ppm in a $^{15}$N resonance position, or (3) from about a 2.5-fold to about a 20-fold decrease in resonance intensity, especially from about a 3-fold to about a 15-fold decrease, and particularly about a 4-fold to about a 10-fold decrease in resonance intensity,

in a TROSY proton-nitrogen correlation NMR spectra of at least three, four or five of the amide resonance positions for amino acids corresponding to residues of SEQ ID NOS:1 or 13, particularly from about two to six of the amide resonance positions for amino acids corresponding to residues of SEQ ID NOS:1, 2, 10 or 13 (especially of SEQ ID NOS: 10 or 13), selected from the group consisting of:

(a) for residue N116, a resonance position located at about H=9.47 or about $^{15}$N=118.30;
(b) for residue Q117, a resonance position located at about H=7.79 or about $^{15}$N=117.67;
(c) for residue H118, a resonance position located at about $^{1}$H=8.75 or about $^{15}$N=116.43;
(d) for residue T141, a resonance position located at about $^{1}$H=7.99 or about $^{15}$N=109.06;
(e) for residue K142, a resonance position located at about $^{1}$H=7.82 or about $^{15}$N=114.25;
(f) for residue E143, a resonance position located at about $^{1}$H=7.40 or about $^{15}$N=114.00; and
(g) for residue E150, a resonance position located at about $^{1}$H=8.70 or about $^{15}$N=118.80. In other words, the shifts are in resonance positions that correspond to residues in the wild-type NGAL protein.

In still yet another embodiment, the present invention relates to the use of an isolated antibody that specifically binds to a human NGAL protein as set forth in SEQ ID NOS:1, 2, 10 or 13 (especially as set forth in SEQ ID NOS: 10 or 13), wherein as a result of adding the antibody to the human NGAL protein (generally done in excess, particularly stoichiometric excess), the antibody causes as compared to when the antibody is not added,

(1) a perturbation of from about 0.05 ppm to about 1.0 ppm in a $^{1}$H resonance position, particularly from about 0.04 ppm to about 0.06 ppm, especially of about 0.05 ppm in a $^{1}$H resonance position,
(2) a perturbation of from about 0.3 ppm to about 3.0 ppm in a $^{15}$N resonance position, particularly of from about 0.1 ppm to about 2.0 ppm, especially of about 0.1 ppm, about 0.3 ppm, or about 0.6 ppm, in a $^{15}$N resonance position, or
(3) from about a 2.5-fold to about a 20-fold decrease in resonance intensity, especially from about a 3-fold to about a 15-fold decrease, and particularly about a 4-fold to about a 10-fold decrease in resonance intensity,

in a TROSY proton-nitrogen correlation NMR spectra of at least three, four or five of the amide resonance positions for amino acids corresponding to residues of SEQ ID NOS:1 or 13, particularly from about two to six of the amide resonance positions for amino acids corresponding to residues of SEQ ID NOS:1, 2, 10 or 13 (especially of SEQ ID NOS: 10 or 13), selected from the group consisting of:

(a) for residue Y64, a resonance position located at about $^{1}$H=9.15 or about $^{15}$N=113.30;
(b) for residue V84, a resonance position located at about $^{1}$H=9.34 or about $^{15}$N=121.50;
(c) for residue G86, a resonance position located at about $^{1}$H=8.32 or about $^{15}$N=111.60;
(d) for residue T93, a resonance position located at about $^{1}$H=9.32 or about $^{15}$N=112.80;
(e) for residue L94, a resonance position located at about $^{1}$H=7.71 or about $^{15}$N=122.72;
(f) for residue G95, a resonance position located at about $^{1}$H=9.30 or about $^{15}$N=113.70; and
(g) for residue S99, a resonance position located at about $^{1}$H=8.18 or about $^{15}$N=114.50.

## D. Methods of Making and Using NGAL Antibodies

The antibodies employed in the immunoassays of the present invention can be made using a variety of different techniques known in the art. For example, polyclonal and monoclonal antibodies against wild-type human NGAL can be raised by immunizing a suitable subject (such as, but not limited to, a rabbit, goat, murine or other mammal) with an immunogenic preparation which contains a suitable immunogen. The immunogen that can be used for the immunization can include cells such as cells from immortalized cell lines NSO which is known to express human NGAL. In particular, antibodies as employed herein can be made as described in U.S. Provisional Application Serial Number 60/981,471 filed October 19, 2007.

Alternatively, the immunogen can be the purified or isolated human wild-type NGAL protein itself (namely, SEQ ID NOS:1 or 13) or a human NGAL fragment thereof. For example, wild-type human NGAL (See, SEQ ID NOS:1 or 13) that has been isolated from a cell which produces the protein (such as NSO) using affinity chromatography, immunoprecipitation or other techniques which are well known in the art, can be used as an immunogen. Alternatively, immunogen can be prepared using chemical synthesis using routine techniques known in the art (such as, but not limited to, a synthesizer).

The antibodies raised in the subject can then be screened to determine if the antibodies bind to wild-type human NGAL or human NGAL fragment. Such antibodies can be further screened using the methods described herein (See, e.g., Example 5). For example, these antibodies can be assayed to determine if they bind to amino acid residues 112, 118 and 147 of wild-type human NGAL or amino acid residues 15 and 109 of wild-type human NGAL (See, SEQ ID NOS:1 or 13). Suitable methods to identify an antibody with the desired characteristics are described herein (See, Example, 5). Moreover, it is fully anticipated that results obtained with antibodies that bind to mutant NGAL (See, SEQ ID NOS:2 or 10). are fully translatable to binding of wild-type NGAL, and that antibodies will bind to comparable residues of wild-type human NGAL (See, SEQ ID NOS:1 or 13). Accordingly, for convenience, and unless there lacks a rational basis in a particular instance for not doing so, mutant NGAL can be employed to assess binding properties of antibodies.

The unit dose of immunogen (namely, the purified protein, tumor cell expressing the protein, or recombinantly expressed human NGAL protein) and the immunization regimen will depend upon the subject to be immunized, its immune status, and the body weight of the subject. To enhance an immune response in the subject, an immunogen can be administered with an adjuvant, such as Freund's complete or incomplete adjuvant.

Immunization of a subject with an immunogen as described above induces a polyclonal antibody response. The antibody titer in the immunized subject can be monitored over time by standard techniques such as an ELISA using an immobilized antigen, namely, human NGAL (SEQ ID NOS:1 or 13, or human NGAL fragment thereof) as described herein.

Other methods of raising antibodies against human NGAL (SEQ ID NOS:1 or 13, or a human NGAL fragment thereof) include using transgenic mice which express human immunoglobin genes (See, for example, WO 91/00906, WO 91/10741 or WO 92/03918). Alternatively, human monoclonal antibodies can be produced by introducing an antigen into immune deficient mice that have been engrafted with human antibody-producing cells or tissues (for example, human bone marrow cells, peripheral blood lymphocytes (PBL), human fetal lymph node tissue, or hematopoietic stem cells). Such methods include raising antibodies in SCID-hu mice (See, for example, WO 93/05796, U.S. Patent No. 5,411,749; or McCune et al., Science, 241:1632-1639 (1988)) or Rag-1/Rag-2 deficient mice. Human antibody-immune deficient mice are also commercially available. For example, Rag-2 deficient mice are available from Taconic Farms (Germantown, NY).

Monoclonal antibodies can be generated by immunizing a subject with an immunogen. At the appropriate time after immunization, for example, when the antibody titers are at a sufficiently high level, antibody producing cells can be harvested from an immunized animal and used to prepare monoclonal antibodies using standard techniques. For example, the antibody producing cells can be fused by standard somatic cell fusion procedures with immortalizing cells such as myeloma cells to yield hybridoma cells. Such techniques are well known in the art, and include, for example, the hybridoma technique as originally developed by Kohler and Milstein, Nature, 256:495497 (1975)), the human B cell hybridoma technique (Kozbar et al., Immunology Today, 4:72 (1983)), and the EBV-hybridoma technique to produce human monoclonal antibodies (Cole et al., Monoclonal Antibodies and Cancer Therapy, Alan R. Liss, Inc. pp. 77-96 (1985)). The technology for producing monoclonal antibody hybridomas is well known to those skilled in the art.

Monoclonal antibodies can also be made by harvesting antibody producing cells, for example, splenocytes, from transgenic mice expressing human immunoglobulin genes and which have been immunized with the human NGAL protein. The splenocytes can be immortalized through fusion with human myelomas or through transformation with Epstein-Barr virus (EBV). These hybridomas can be made using human B cell-or EBV-hybridoma techniques described in the art (See, for example, Boyle et al., European Patent Publication No. 0 614 984).

Hybridoma cells producing a monoclonal antibody which specifically binds to the wild-type human NGAL protein (SEQ ID NOS:1 or 13) or a human NGAL fragment thereof are detected by screening the hybridoma culture supernatants by, for example, screening to select antibodies that specifically bind to the immobilized human NGAL protein, or by testing the antibodies as described herein to determine if the antibodies have the desired characteristics, namely, the ability to bind to human NGAL at the amino acid residues described herein. After hybridoma cells are identified that produce antibodies of the desired specificity, the clones may be subcloned, e.g., by limiting dilution procedures, for example the procedure described by Wands et al. (Gastroenterology 80:225-232 (1981)), and grown by standard methods.

Hybridoma cells that produce monoclonal antibodies that test positive in the screening assays described herein can be cultured in a nutrient medium under conditions and for a time sufficient to allow the hybridoma cells to secrete the monoclonal antibodies into the culture medium, to thereby produce whole antibodies. Tissue culture techniques and culture media suitable for hybridoma cells are generally described in the art (See, for example, R. H. Kenneth, in Monoclonal Antibodies: A New Dimension In Biological Analyses, Plenum Publishing Corp., New York, N.Y. (1980)). Conditioned hybridoma culture supernatant containing the antibody can then be collected. The monoclonal antibodies secreted by the subclones optionally can be isolated from the culture medium by conventional immunoglobulin purification procedures such as, for example, protein A chromatography, hydroxylapatite chromatography, gel electrophoresis, dialysis, or affinity chromatography.

Monoclonal antibodies can be engineered by constructing a recombinant combinatorial immunoglobulin library and screening the library with the human NGAL protein. Kits for generating and screening phage display libraries are commercially available (See, for example, the Pharmacia Recombinant Phage Antibody System, Catalog No. 27-9400-01; and the Stratagene SurfZAP Phage Display Kit, Catalog No. 240612). Likewise, yeast display vectors are known in the art and are commercially available (for example, pYD1 available from Invitrogen Corp., Carlsbad, CA). Briefly, the antibody library is screened to identify and isolate phages or yeast cells that express an antibody that specifically binds to the wild-type human NGAL protein (SEQ ID NOS:1 or 13). Preferably, the primary screening of the library involves screening with an immobilized wild-type human NGAL protein or a fragment thereof.

Following screening, the display phage or yeast is isolated and the polynucleotide encoding the selected antibody can be recovered from the display phage or yeast (for example, from the phage or yeast genome) and subcloned into other expression vectors (e.g., into *Saccharomyces cerevesiae* cells, for example EBY100 cells (Invitrogen Corporation, Carlsbad, CA)) by well known recombinant DNA techniques. The polynucleotide can be further manipulated (for example, linked to nucleic acid encoding additional immunoglobulin domains, such as additional constant regions) and/or expressed in a host cell.

Alternatively, recombinant forms of antibodies, such as chimeric and humanized antibodies, can also be prepared to minimize the response by a human patient to the antibody. When antibodies produced in non-human subjects or derived from expression of non-human antibody genes are used therapeutically in humans, they are recognized to varying degrees as foreign, and an immune response may be generated in the patient. One approach to minimize or eliminate this immune reaction is to produce chimeric antibody derivatives, namely, antibody molecules that combine a non-human animal variable region and a human constant region. Such antibodies retain the epitope binding specificity of the original monoclonal antibody, but may be less immunogenic when administered to humans, and therefore more likely to be tolerated by the patient.

Chimeric monoclonal antibodies can be produced by recombinant DNA techniques known in the art. For example, a gene encoding the constant region of a non-human antibody molecule is substituted with a gene encoding a human constant region (See, for example, PCT Patent Publication PCT/US86/02269, European Patent Application 184,187 or European Patent Application 171,496).

A chimeric antibody can be further "humanized" by replacing portions of the variable region not involved in antigen binding with equivalent portions from human variable regions. General reviews of "humanized" chimeric antibodies can be found in Morrison, S. L., Science, 229:1202-1207 (1985) and in Oi et al., BioTechniques, 4-214 (1986). Such methods include isolating, manipulating, and expressing the nucleic acid sequences that encode all or part of an immunoglobulin variable region from at least one of a heavy or light chain. The cDNA encoding the humanized chimeric antibody, or fragment thereof, can then be cloned into an appropriate expression vector. Suitable "humanized" antibodies can be alternatively produced by complementarity determining region (CDR) substitution (See, for example, U.S. Patent No. 5,225,539; Jones et al., Nature, 321:552-525 (1986); Verhoeyan et al., Science 239:1.534 (1988); and Beidler et al., J. Immunol,. 141:4053-4060 (1988)).

Epitope imprinting can also be used to produce a "human" antibody polypeptide dimer that retains the binding specificity of the antibodies (e.g., hamster antibodies) specific for the wild-type human NGAL protein (SEQ ID NOS:1 or 13) or human NGAL fragment thereof. Briefly, a gene encoding a non-human variable region (VH) with specific binding to an antigen and a human constant region (CH1), is expressed in *E. coli* and infected with a phage library of human Vλ.Cλ genes. Phage displaying antibody fragments are then screened for binding to the human NGAL protein. Selected human Vλ genes are recloned for expression of Vλ.Cλ. chains and *E. coli* harboring these chains are infected with a phage library of human VHCH1 genes and the library is subject to rounds of screening with antigen coated tubes (See, WO 93/06213).

In another aspect, the present invention contemplates that the antibody is an antibody fragment. For example, the antibody fragment can include, but is not limited to, a Fab, a Fab', a Fab'-SH fragment, a di-sulfide linked Fv, a single chain Fv (scFv) and a F(ab')$_2$ fragment. Various techniques are known to those skilled in the art for the production of antibody fragments. For example, such fragments can be derived via proteolytic digestion of intact antibodies (See, for example, Morimoto et al., J. Biochem. Biophys. Methods, 24:107-117 (1992) and Brennan et al., Science, 229:81 (1985)) or produced directly by recombinant host cells. For example, Fab'-SH fragments can be directly recovered from *E. coli* and chemically coupled to form F(ab')$_2$ fragments (See, Carter et al., Bio/Technology, 10:163-167 (1992)). In another embodiment, the F(ab')$_2$ is formed using the leucine zipper GCN4 to promote assembly of the F(ab')$_2$ molecule. Alternatively, Fv, Fab or F(ab')$_2$ fragments can be isolated directly from recombinant host cell culture. Single chain variable region fragments (scFv) are made by linking light and/or heavy chain variable regions by using a short linking peptide (See, Bird et al. Science, 242:423-426 (1998)). An example of a linking peptide is GPAKELTPLKEAKVS (SEQ ID NO: 11). Linkers can in turn be modified for additional functions, such as attachment of drugs or attachment to solid supports. Examples of other linker sequences that can be used in the present invention can be found in Bird et al., Science, 242: 423-426 (1988), Huston et al., Proc. Natl. Acad. Sci. USA, 85:5879-5883 (1988) and McCafferty et al., Nature, 348: 552-554 (1990).

The single chain variants can be produced either recombinantly or synthetically. For synthetic production of scFv, an automated synthesizer can be used. For recombinant production of scFv, a suitable plasmid containing polynucleotide that encodes the scFv can be introduced into a suitable host cell, either eukaryotic, such as yeast, plant, insect or mammalian cells, or prokaryotic, such as E. *coli.* Polynucleotides encoding the scFv of interest can be made by routine manipulations such as ligation of polynucleotides. The resultant scFv can be isolated using standard protein purification techniques known in the art. Moreover, other forms of single chain antibodies, such as diabodies are also contemplated by the present invention. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (See, for example, Holliger, P., et al., Proc. Natl. Acad. Sci. USA, 90:6444-6448 (1993); Poljak, R. J., et al., Structure, 2:1121-1123 (1994)).

Furthermore, in some aspects of the invention(s) as described herein (e.g., use as controls), it may be possible to employ commercially available anti-NGAL antibodies, or anti-NGAL antibodies or their methods for production described in the literature. These include but are not limited to: (1) anti-NGAL monoclonal antibodies (either HYB 211-01, HYB 211-02, or HYB 211-05, commercially available from AntibodyShop A/S, Gentofte, Denmark); (2) mouse anti-NGAL monoclonal antibody (e.g., Clone No. 697, Catalog No. HM2193B, HyCult Biotechnology, Uden, Netherlands); (3) rat anti-NGAL monoclonal antibody (e.g., Clone No. 220310, Catalog No. MAB 1757, R&D Systems, Minneapolis, MN); (4) anti-NGAL antibodies contained in Quantikine® NGAL ELISA kit DLCN20 (R&D Systems, Minneapolis, MN), which purportedly detect the free NGAL form (i.e., form not complexed in a heterodimer) (U.S. Patent Application Publication No. 2007/0196876); (5) rabbit antihuman NGAL monoclonal antibodies produced in mouse hybridoma cells (EP 0 756 708 and U.S. Patent No. 6, 136,526); (6) purified monoclonal or polyclonal antibody against human NGAL (Kjeldsen et al., J. Biolog. Chem., 268:10425-32 (1993); Kjeldsen et al., J. Immunolog. Methods, 198(2):155-64 (1996)); (7) polyclonal antibody against human NGAL (PCT International Application WO 2002/031507); and/or (8) discussing the use of solvent-exposed peptide loop areas of NGAL for making monoclonal antibody against human NGAL (U.S. Patent No. 7,056,702 and U.S. Patent Application Publication US2004/115728).

The antibodies of the present invention have a variety of uses. In one aspect, the antibodies of the present invention can be used as one or more immunodiagnostic reagents. For example, the antibodies of the present invention can be used as one or more immunodiagnostic reagents in one or more methods for detecting the presence of human NGAL antigen in a test sample. More specifically, the antibodies of the present invention can be used as one or more capture antibodies, one or more conjugate antibodies or as both one or more capture antibodies and one or more conjugate antibodies in immunoassays to detect the presence of human NGAL in a test sample.

### E. Sample Collection and Pretreatment

Methods well known in the art for collecting, handling and processing urine, blood, serum and plasma, and other body fluids, are used in the practice of the present invention.

The test sample may comprise further moieties in addition to the NGAL analyte of interest such as antibodies, antigens, haptens, hormones, drugs, enzymes, receptors, proteins, peptides, polypeptides, oligonucleotides or polynucleotides. For example, the sample may be a whole blood sample obtained from a subject. It may be necessary or desired that a test sample, particularly whole blood, be treated prior to immunoassay as described herein, e.g., with a pretreatment reagent. Even in cases where pretreatment is not necessary (e.g., most urine samples), pretreatment optionally may be done for mere convenience (e.g., as part of a regimen on a commercial platform). The pretreatment reagent can be a heterogeneous agent or a homogeneous agent.

With use of a heterogenous pretreatment reagent according to the invention, the pretreatment reagent precipitates analyte binding protein (e.g., protein capable of binding NGAL) present in the sample. Such a pretreatment step comprises removing any analyte binding protein by separating from the precipitated analyte binding protein the supernatant of the mixture formed by addition of the pretreatment agent to sample. In such an assay, the supernatant of the mixture absent any binding protein is used in the assay, proceeding directly to the antibody capture step.

With use of a homogeneous pretreatment reagent there is no such separation step. The entire mixture of test sample and pretreatment reagent are contacted with the capture antibody in the antibody capture step. The pretreatment reagent employed for such an assay typically is diluted in the pretreated test sample mixture, either before the antibody capture step or during encounter with the antibody in the antibody capture step. Despite such dilution, a certain amount of the pretreatment reagent (for example, 5 M methanol and/or 0.6 M ethylene glycol) is still present (or remains) in the test sample mixture during antibody capture.

The pretreatment reagent can be any reagent appropriate for use with the immunoassay and kits of the invention. The pretreatment optionally comprises: (a) one or more solvents (e.g., methanol and ethylene glycol) and salt, (b) one or more solvents, salt and detergent, (c) detergent, or (d) detergent and salt. Pretreatment reagents are known in the art, and such pretreatment can be employed, e.g., as used for assays on Abbott TDx, AxSYM®, and ARCHITECT® analyzers (Abbott Laboratories, Abbott Park, IL), as described in the literature (see, e.g., Yatscoff et al., Abbott TDx Monoclonal Antibody Assay Evaluated for Measuring Cyclosporine in Whole Blood, Clin. Chem., 36:1969-1973 (1990) and Wallemacq et al., Evaluation of the New AxSYM Cyclosporine Assay:Comparison with TDx Monoclonal Whole Blood and EMIT Cyclosporine Assays, Clin. Chem. 45: 432-435 (1999)), and/or as commercially available. Additionally, pretreatment can be done as described in Abbott's U.S. Patent No. 5,135,875, EP 0 471 293, U.S Patent Application 60/878,017 filed December 29, 2006; and U.S Patent Application 11/490624 filed June 21, 2006. Also, proteases, either alone or in combination with any other pretreatment agents (e.g., solvents, detergents, salts, and the like) can be employed.

## F. NGAL Immunoassays

Immunoassays can be conducted using any format known in the art, such as, but not limited to, a sandwich format. Specifically, in one aspect of the present invention, at least two antibodies are employed to separate and quantify human NGAL or human NGAL fragment in a test sample. More specifically, the at least two antibodies bind to certain epitopes of human NGAL or human NGAL fragment forming an immune complex which is referred to as a "sandwich". Generally, in the immunoassays one or more antibodies can be used to capture the human NGAL or human NGAL fragment in the test sample (these antibodies are frequently referred to as a "capture" antibody or "capture" antibodies) and one or more antibodies can be used to bind a detectable (namely, quantifiable) label to the sandwich (these antibodies are frequently referred to as the "detection antibody", "detection antibodies", a "conjugate" or "conjugates").

Excellent immunoassays, particularly, sandwich assays, can be performed using the antibodies of the present invention as the capture antibodies, detection antibodies or as capture and detection antibodies. For example, at least one of the antibodies of the present invention (such as antibody produced by murine hybridoma cell line 1-2322-455 or an antibody produced by murine hybridoma cell line 1-903-430 or a combination of an antibody produced by murine hybridoma cell line 1-2322-455 and an antibody produced by murine hybridoma cell line 1-903-430) can be used as a first capture antibody. Alternatively, in more than one capture antibody is being used, then the antibodies of the present invention can be used as a second or subsequent capture antibody. Alternatively, if one of the antibodies of the present invention is being used as a capture antibody, a different antibody (other than an antibody of the present invention) can be used as a second capture antibody. Alternatively, the antibodies of the present invention can be used only as detection antibodies and not as capture antibodies. Still in another alternative, the antibodies of the present invention can be used as both capture and detection antibodies. For example, an antibody produced by murine hybridoma cell line 1-2322-455 can be used as a capture antibody and an antibody produced by murine hybridoma cell line 1-903-422 can be used as a detection antibody. Alternatively, an antibody produced by murine hybridoma cell line 1-903-422 can be used as a capture antibody and an antibody produced by hybridoma cell line 1-2322-455 can be used as a detection antibody.

The test sample being tested for (for example, suspected of containing) human NGAL or human NGAL fragment can be contacted with at least one capture antibody (or antibodies) and at least one detection antibody (which is either a second detection antibody or a third detection antibody) either simultaneously or sequentially and in any order. For example, the test sample can be first contacted with at least one capture antibody and then (sequentially) with at least one detection antibody. Alternatively, the test sample can be first contacted with at least one detection antibody and then (sequentially) with at least one capture antibody. In yet another alternative, the test sample can be contacted simultaneously with a capture antibody and a detection antibody.

In the sandwich assay format, a test sample suspected of containing human NGAL or human NGAL fragment is first brought into contact with an at least one first capture antibody under conditions which allow the formation of a first antibody/human NGAL complex. If more than one capture antibody is used, a first multiple capture antibody/human NGAL complex is formed. In a sandwich assay, the antibodies, preferably, the at least one capture antibody, are used in molar excess amounts of the maximum amount of human NGAL or human NGAL fragment expected in the test sample. For example, from about 5 μg/mL to about 1 mg/mL of antibody per mL of buffer (e.g., microparticle coating buffer) can be used.

Optionally, prior to contacting the test sample with the at least one capture antibody (for example, the first capture antibody), the at least one capture antibody can be bound to a solid support which facilitates the separation the first antibody/human NGAL complex from the test sample. Any solid support known in the art can be used, including, but not limited to, solid supports made out of polymeric materials in the forms of wells, tubes or beads. The antibody (or antibodies) can be bound to the solid support by adsorption, by covalent bonding using a chemical coupling agent or by other means known in the art, provided that such binding does not interfere with the ability of the antibody to bind human NGAL or human NGAL fragment. Alternatively, the antibody (or antibodies) can be bound with microparticles that have previously coated with streptavidin or biotin (for example, using Power-Bind™-SA-MP streptavidin coated microparticles, available from Seradyn, Indianapolis, Indiana). Alternatively, the antibody (or antibodies) can be bound using microparticles that have been previously coated with anti-species specific monoclonal antibodies. Moreover, if necessary, the solid support can be derivatized to allow reactivity with various functional groups on the antibody. Such derivatization requires the use of certain coupling agents such as, but not limited to, maleic anhydride, N-hydroxysuccinimide and 1-ethyl-3-(3-dimethylaminopropyl)carbodiimide.

After the test sample being tested for and/or suspected of containing human NGAL or a human NGAL fragment is brought into contact with the at least one capture antibody (for example, the first capture antibody), the mixture is incubated in order to allow for the formation of a first antibody (or multiple antibody)-human NGAL complex. The incubation can be carried out at a pH of from about 4.5 to about 10.0, at a temperature of from about 2°C to about 45°C, and for a period from at least about one (1) minute to about eighteen (18) hours, preferably from about 1 to about 20 minutes, most preferably for about 18 minutes. The immunoassay described herein can be conducted in one step (meaning the test sample, at least one capture antibody and at least one detection antibody are all added sequentially or simultaneously to a reaction vessel) or in more than one step, such as two steps, three steps, etc.

After formation of the (first or multiple) capture antibody/human NGAL complex, the complex is then contacted with at least one detection antibody (under conditions which allow for the formation of a (first or multiple) capture antibody/human NGAL/second antibody detection complex). The at least one detection antibody can be the second, third, fourth, etc. antibodies used in the immunoassay. If the capture antibody/human NGAL complex is contacted with more than one detection antibody, then a (first or multiple) capture antibody/human NGAL/(multiple) detection antibody complex is formed. As with the capture antibody (e.g., the first capture antibody), when the at least second (and subsequent) detection antibody is brought into contact with the capture antibody/human NGAL complex, a period of incubation under conditions similar to those described above is required for the formation of the (first or multiple) capture antibody/human NGAL/(second or multiple) detection antibody complex. Preferably, at least one detection antibody contains a detectable label. The detectable label can be bound to the at least one detection antibody (e.g., the second detection antibody) prior to, simultaneously with or after the formation of the (first or multiple) capture antibody/human NGAL/(second or multiple) detection antibody complex. Any detectable label known in the art can be used. For example, the detectable label can be a radioactive label, such as, $^3$H, $^{125}$I, $^{35}$S, $^{14}$C, $^{32}$P, $^{33}$P, an enzymatic label, such as horseradish peroxidase, alkaline phosphatase, glucose 6-phosphate dehydrogenase, etc., a chemiluminescent label, such as, acridinium esters, luminal, isoluminol, thioesters, sulfonamides, phenanthridinium esters, etc. a fluorescence label, such as, fluorescein (5-fluorescein, 6-carboxyfluorescein, 3'6-carboxyfluorescein, 5(6)-carboxyfluorescein, 6-hexachlorofluorescein, 6-tetrachlorofluorescein, fluorescein isothiocyanate, etc.), rhodamine, phycobiliproteins, R-phycoerythrin, quantum dots (zinc sulfide-capped cadmium selenide), a thermometric label or an immuno-polymerase chain reaction label. An introduction to labels, labeling procedures and detection of labels is found in Polak and Van Noorden, Introduction to Immunocytochemistry, 2nd ed., Springer Verlag, N.Y. (1997) and in Haugland, Handbook of Fluorescent Probes and Research Chemicals (1996), which is a combined handbook and catalogue published by Molecular Probes, Inc., Eugene, Oregon.

The detectable label can be bound to the antibodies either directly or through a coupling agent. An example of a coupling agent that can be used is EDAC (1-methyl-3-(3-dimethylaminopropyl) carbodiimide, hydrochloride) that is commercially available from Sigma-Aldrich, St. Louis, MO. Other coupling agents that can be used are known in the art. Methods for binding a detectable label to an antibody are known in the art. Additionally, many detectable labels can be purchased or synthesized that already contain end groups that facilitate the coupling of the detectable label to the antibody, such as, N10-(3-sulfopropyl)-N-(3-carboxypropyl)-acridinium-9-carboxamide, otherwise known as CPSP-Acridinium Ester or N10-(3-sulfopropyl)-N-(3-sulfopropyl)-acridinium-9-carboxamide, otherwise known as SPSP-Acridinium Ester.

The (first or multiple) capture antibody/human NGAL/(second or multiple) detection antibody complex can be, but does not have to be, separated from the reminder of the test sample prior to quantification of the label. For example, if the at least one capture antibody (e.g., the first capture antibody) is bound to a solid support, such as a well or a bead, separation can be accomplished by removing the fluid (of the test sample) from contact with the solid support. Alternatively, if the at least first capture antibody is bound to a solid support it can be simultaneously contacted with the human NGAL-containing sample and the at least one second detection antibody to form a first (multiple) antibody/human NGAL/second (multiple) antibody complex, followed by removal of the fluid (test sample) from contact with the solid support. If the at least one first capture antibody is not bound to a solid support, then the (first or multiple) capture antibody/human NGAL/(second or multiple) detection antibody complex does not have to be removed from the test sample for quantification of the amount of the label.

After formation of the labeled capture antibody/human NGAL/detection antibody complex (e.g., the first capture antibody/human NGAL/second detection antibody complex), the amount of label in the complex is quantified using techniques known in the art. For example, if an enzymatic label is used, the labeled complex is reacted with a substrate for the label that gives a quantifiable reaction such as the development of color. If the label is a radioactive label, the label is quantified using a scintillation counter. If the label is a fluorescent label, the label is quantified by stimulating the label with a light of one color (which is known as the "excitation wavelength") and detecting another color (which is known as the "emission wavelength") that is emitted by the label in response to the stimulation. If the label is a chemiluminescent label, the label is quantified detecting the light emitted either visually or by using luminometers, x-ray film, high speed photographic film, a CCD camera, etc. Once the amount of the label in the complex has been quantified, the concentration of human NGAL or human NGAL fragment in the test sample is determined by use of a standard curve that has been generated using serial dilutions of human NGAL or human NGAL fragment of known concentration. Other than using serial dilutions of human NGAL or human NGAL fragment, the standard curve can be generated gravimetrically, by mass spectroscopy and by other techniques known in the art.

In another aspect, the antibodies of the present invention can be used to determine the amount of human NGAL monomer in a test sample that is suspected of containing both human NGAL monomer and human NGAL dimer (such as human NGAL homodimer or human NGAL heterodimer). More specifically, the antibodies of the present invention can be used in one or more immunoassays to determine the amount of human NGAL monomer contained in a test sample with at least about seventy-five percent (75%) specificity.

More specifically, the antibodies of the present invention can be used as capture and detection antibodies to identify the amount of human NGAL monomer contained in a test sample. For example, if a first capture antibody comprising one or more antibodies such as an antibody produced by murine hybridoma cell line 1-2322-455 or an antibody produced by murine hybridoma cell line 1-903-422 is selected for use in an immunoassay, then the same or a different antibody can be selected for use as a second capture antibody in addition to the first capture antibody. However, it is preferred that the second capture antibody bind to a different epitope than the epitope bound by the first capture antibody. Alternatively, the second capture antibody can be an antibody other than an antibody of the present invention. Alternatively, if a second capture antibody is not used, then the second antibody, namely, the detection antibody, can be one or more of antibodies of the present invention, such as, for example, antibody produced by murine hybridoma cell line 1-2322-455 or an antibody produced by murine hybridoma cell line 1-903-422. As with the second capture antibody above, it is preferred that the detection antibody bind to a different epitope than the epitope bound by the first capture antibody, and if present, the second capture antibody.

Immunoassays performed as described herein that employ the antibodies of the present invention as at least one capture antibody or as at least one detection antibody are capable of determining the amount of human NGAL monomer contained in a test sample with at least about 75% specificity. Preferably, the immunoassay is capable of determining the amount of human NGAL monomer in a test sample with at least about 85% specificity. Most preferably, immunoassay is capable of determining the amount of human NGAL monomer in a test sample with at least about 90% specificity. Even more preferably, immunoassay is capable of determining the amount of human NGAL monomer in a test sample with at least about 95% specificity. Even most preferably, immunoassay is capable of determining the amount of human NGAL monomer in a test sample with at least about 98% specificity.

In particular, the methods and immunoassays as described herein optimally have from about 75% specificity up to about 98% specificity for monomer (e.g., are at least about four times, e.g., from about 3 to about 5 times, or at least about 3, 4, 4.5, or 5 times, as specific for NGAL monomer as compared to NGAL dimer), especially throughout the calibration range of the assay (e.g., from about 1 ng/mL up to about 2000 ng/ml of NGAL, especially urinary NGAL). Thus, the methods and immunoassays as described herein exhibit at least about 75% specificity from about 5 ng/mL up to about 2000 ng/ml of NGAL (e.g., including urinary NGAL), especially from about 100 ng/mL up to about 2000 ng/mL of NGAL (e.g., including urinary NGAL), particularly from about 150 ng/mL up to about 2000 ng/ml of NGAL (e.g., including urinary NGAL), including (a) from about 200 ng/mL up to about 2000 ng/ml of NGAL, (b) from about 250 ng/mL up to about 2000 ng/ml of NGAL, (c) from about 300 ng/mL up to about 2000 ng/ml of NGAL, (d) from about 350 ng/mL up to about 2000 ng/ml of NGAL, (e.g., including urinary NGAL), (e) from about 400 ng/mL up to about 2000 ng/ml of NGAL, and (a) from about 500 ng/mL up to about 2000 ng/ml of NGAL (e.g., including urinary NGAL).

The test sample being tested to determine the amount of human NGAL monomer can be contacted with at least one capture antibody (or antibodies) and at least one detection antibody (which is either a second detection antibody or a third detection antibody) either simultaneously or sequentially and in any order. For example, the test sample can be first contacted with at least one capture antibody and then (sequentially) with at least one detection antibody. Alternatively, the test sample can be first contacted with at least one detection antibody and then (sequentially) with at least one capture antibody. In yet another alternative, the test sample can be contacted simultaneously with a capture antibody and a detection antibody.

In the sandwich assay format, a test sample is first brought into contact with the at least one first capture antibody which is an antibody of the present invention (such as, for example, an antibody produced by murine hybridoma cell line 1-2322-455 or an antibody produced by murine hybridoma cell line 1-903-422) under conditions which allow the formation of a first antibody/human NGAL complex. If more than one capture antibody is used, a first multiple capture antibody/human NGAL complex is formed. In a sandwich assay, the antibodies, preferably, the at least one capture antibody, are used in molar excess amounts of the maximum amount of human NGAL or human NGAL fragment expected in the test sample. For example, from about 5 μg/mL to about 1 mg/mL of antibody per mL of buffer (e.g., microparticle coating buffer) can be used.

Optionally, prior to contacting the test sample with the at least one capture antibody (e.g., the first capture antibody), the at least one capture antibody can be bound to a solid support using the techniques as discussed previously herein. After the test sample being suspected of containing human NGAL monomer and human NGAL dimer is brought into contact with the at least one capture antibody (e.g., the first capture antibody), the mixture is incubated in order to allow for the formation of a first antibody (or multiple antibody)/human NGAL complex. The incubation can be carried out at a pH of from about 4.5 to about 10.0, at a temperature of from about 2°C to about 45°C, and for a period from at least about one (1) minute to about eighteen (18) hours, preferably from about 1 to about 20 minutes, most preferably for about 18 minutes. The immunoassay described herein can be conducted in one step (meaning the test sample, at least one capture antibody and at least one detection antibody are all added sequentially or simultaneously to a reaction vessel) or in more than one step, such as two steps, three steps, etc.

After formation of the (first or multiple) capture antibody/human NGAL complex, the complex is then contacted with at least one detection antibody (under conditions which allow for the formation of a (first or multiple) capture antibody/human NGAL/second antibody detection complex). The at least one detection antibody can be the second, third, fourth, etc. antibodies used in the immunoassay. If the capture antibody/human NGAL complex is contacted with more than one detection antibody, then a (first or multiple) capture antibody/human NGAL/(multiple) detection antibody complex is formed. As with the capture antibody (e.g., the first capture antibody), when the at least second (and subsequent) detection antibody is brought into contact with the capture antibody/human NGAL complex, a period of incubation under conditions similar to those described above is required for the formation of the (first or multiple) capture antibody/human NGAL/(second or multiple) detection antibody complex. Preferably, at least one detection antibody contains a detectable label. The detectable label can be any detectable label as discussed previously herein.

The immunoassay described herein for determining the amount of human NGAL monomer in a test sample can be performed in the absence of any reducing agents. Alternatively, one or more of any of the steps of the immunoassay can be performed in the presence of one or more reducing agents. In yet another alternative, the test sample can be treated (namely, pre-treated) with one or more reducing agents prior to using the test sample in the immunoassay. Any reducing agent can be used in the immunoassay or to pre-treat the test sample. Examples of reducing agents that can be used include, but are not limited to, dithiothreitol, 2-mercaptoethanol, 2-mercaptoethylamine and Tris (2-carboxyethyl) phosphine. The amount of reducing agent that can be used in the immunoassay or can be used to pre-treat the test sample is from about 0.1 mM to about 500 mM, especially an amount of from about 0.1 mM to about 100 mM.

The (first or multiple) capture antibody/human NGAL/(second or multiple) detection antibody complex can be, but does not have to be, separated from the remainder of the test sample prior to quantification of the label. For example, if the at least one capture antibody (e.g., the first capture antibody) is bound to a solid support, such as a well or a bead, separation can be accomplished by removing the fluid (of the test sample) from contact with the solid support. Alternatively, if the at least first capture antibody is bound to a solid support it can be simultaneously contacted with the human NGAL-containing sample and the at least one second detection antibody to form a first (multiple) antibody/human NGAL/second (multiple) antibody complex, followed by removal of the fluid (test sample) from contact with the solid support. If the at least one first capture antibody is not bound to a solid support, then the (first or multiple) capture antibody/human NGAL/(second or multiple) detection antibody complex does not have to be removed from the test sample for quantification of the amount of the label.

After formation of the labeled capture antibody/human NGAL/detection antibody complex (e.g., the first capture antibody/human NGAL/second detection antibody complex), the amount of label in the complex is quantified using techniques known in the art. For example, if an enzymatic label is used, the labeled complex is reacted with a substrate for the label that gives a quantifiable reaction such as the development of color. If the label is a radioactive label, the label is quantified using a scintillation counter. If the label is a fluorescent label, the label is quantified by stimulating the label with a light of one color (which is known as the "excitation wavelength") and detecting another color (which is known as the "emission wavelength") that is emitted by the label in response to the stimulation. If the label is a chemiluminescent label, the label is quantified detecting the light emitted either visually or by using luminometers, x-ray film, high speed photographic film, a CCD camera, etc. Once the amount of the label in the complex has been quantified, the concentration of human NGAL monomer in the test sample is determined by use of a standard curve that has been generated using serial dilutions of human NGAL monomer of known concentration. Other than using serial dilutions of human NGAL monomer, the standard curve can be generated gravimetrically, by mass spectroscopy and by other techniques known in the art.

In yet another aspect, the antibodies of the present invention can be used in a method to determine the proportion of human NGAL monomer to human NGAL dimer contained in a test sample. The method for determining the proportion of human NGAL monomer to human NGAL dimer involves performing the steps of an immunoassay at least twice (or repeating certain steps of the immunoassay). More specifically, the antibodies of the present invention can be used as capture and detection antibodies to determine the proportion of human NGAL monomer to human NGAL dimer contained in a test sample. For example, if a first capture antibody comprising one or more antibodies such as an antibody produced by murine hybridoma cell line 1-2322-455 or an antibody produced by murine hybridoma cell line 1-903-422 is selected for use in an immunoassay, then the same or a different antibody can be selected for use as a second capture antibody in addition to the first capture antibody. However, it is preferred that the second capture antibody bind to a different epitope than the epitope bound by the first capture antibody. Alternatively, the second capture antibody can be an antibody other than an antibody of the present invention. Alternatively, if a second capture antibody is not used, then the second antibody, namely, the detection antibody, can be one or more of antibodies of the present invention, such as, for example, antibody produced by murine hybridoma cell line 1-2322-455 or an antibody produced by murine hybridoma cell line 1-903-422. As with the second capture antibody above, it is preferred that the detection antibody bind to a different epitope than the epitope bound by the first capture antibody, and if present, the second capture antibody.

Immunoassays performed as described herein not only employ the antibodies of the present invention as at least one capture antibody or as at least one detection antibody but also employ the use of one or more reducing agents at a specific point in the immunoassay. Any reducing agent can be used in the immunoassay. Examples of reducing agents that can be used include, but are not limited to, dithiothreitol, 2-mercaptoethanol, 2-mercaptoethylamine and Tris (2-carboxyethyl)phosphine. The amount of reducing agent that can be used in the immunoassay or can be used to pre-treat the test sample is from about 0.1 mM to about 500 mM, especially from about 0.1 mM to about 100 mM.

Specifically, the test sample being tested to determine proportion of human NGAL monomer to human NGAL dimer can be contacted with at least one capture antibody (or antibodies) and at least one detection antibody (which is either a second detection antibody or a third detection antibody) either simultaneously or sequentially and in any order. For example, the test sample can be first contacted with at least one capture antibody and then (sequentially) with at least one detection antibody. Alternatively, the test sample can be first contacted with at least one detection antibody and then (sequentially) with at least one capture antibody. In yet another alternative, the test sample can be contacted simultaneously with a capture antibody and a detection antibody.

In the sandwich assay format, a test sample is first brought into contact with the at least one first capture antibody which is an antibody of the present invention (such as, for example, an antibody produced by murine hybridoma cell line 1-2322-455 or an antibody produced by murine hybridoma cell line 1-903-422) under conditions which allow the formation of a first antibody/human NGAL complex. If more than one capture antibody is used, a first multiple capture antibody/human NGAL complex is formed. In a sandwich assay, the antibodies, preferably, the at least one capture antibody, are used in molar excess amounts of the maximum amount of human NGAL or human NGAL fragment expected in the test sample. For example, from about 5 µg/mL to about 1 mg/mL of antibody per mL of buffer (e.g., microparticle coating buffer) can be used.

Optionally, prior to contacting the test sample with the at least one capture antibody (e.g., the first capture antibody), the at least one capture antibody can be bound to a solid support using the techniques as discussed previously herein. After the test sample being suspected of containing human NGAL monomer and human NGAL dimer is brought into contact with the at least one capture antibody (e.g., the first capture antibody), the mixture is incubated in order to allow for the formation of a first (or multiple) antibody/human NGAL complex. The incubation can be carried out at a pH of from about 4.5 to about 10.0, at a temperature of from about 2°C to about 45°C, and for a period from at least about one (1) minute to about eighteen (18) hours, preferably from about 1 to about 20 minutes, most preferably for about 18 minutes. The immunoassay described herein can be conducted in one step (meaning the test sample, at least one capture antibody and at least one detection antibody are all added sequentially or simultaneously to a reaction vessel) or in more than one step, such as two steps, three steps, etc.

After formation of the (first or multiple) capture antibody/human NGAL complex, the complex is then contacted with at least one detection antibody (under conditions which allow for the formation of a (first or multiple) capture antibody/ human NGAL/second antibody detection complex). The at least one detection antibody can be the second, third, fourth, etc. antibodies used in the immunoassay. If the capture antibody/human NGAL complex is contacted with more than one detection antibody, then a (first or multiple) capture antibody/human NGAL/(multiple) detection antibody complex is formed. As with the capture antibody (e.g., the first capture antibody), when the at least second (and subsequent) detection antibody is brought into contact with the capture antibody/human NGAL complex, a period of incubation under conditions similar to those described above is required for the formation of the (first or multiple) capture antibody/human NGAL/(second or multiple) detection antibody complex. Preferably, at least one detection antibody contains a detectable label. The detectable label can be any detectable label as discussed previously herein.

The (first or multiple) capture antibody/human NGAL/(second or multiple) detection antibody complex can be, but does not have to be, separated from the remainder of the test sample prior to quantification of the label. For example, if the at least one capture antibody (e.g., the first capture antibody) is bound to a solid support, such as a well or a bead, separation can be accomplished by removing the fluid (of the test sample) from contact with the solid support. Alternatively, if the at least first capture antibody is bound to a solid support it can be simultaneously contacted with the human NGAL-containing sample and the at least one second detection antibody to form a first (multiple) antibody/human NGAL/second (multiple) antibody complex, followed by removal of the fluid (test sample) from contact with the solid support. If the at least one first capture antibody is not bound to a solid support, then the (first or multiple) capture antibody/human NGAL/ (second or multiple) detection antibody complex does not have to be removed from the test sample for quantification of the amount of the label.

After formation of the labeled capture antibody/human NGAL/detection antibody complex (e.g., the first capture antibody/human NGAL/second detection antibody complex), the amount of label in the complex is quantified using techniques known in the art. For example, if an enzymatic label is used, the labeled complex is reacted with a substrate for the label that gives a quantifiable reaction such as the development of color. If the label is a radioactive label, the label is quantified using a scintillation counter. If the label is a fluorescent label, the label is quantified by stimulating the label with a light of one color (which is known as the "excitation wavelength") and detecting another color (which is known as the "emission wavelength") that is emitted by the label in response to the stimulation. If the label is a chemiluminescent label, the label is quantified detecting the light emitted either visually or by using luminometers, x-ray film, high speed photographic film, a CCD camera, etc. Once the amount of the label in the complex has been quantified, the concentration of human NGAL dimer and human monomer in the test sample is determined by use of a standard curve that has been generated using serial dilutions of human NGAL dimer and human NGAL monomer of known concentrations. Other than using serial dilutions of human NGAL dimer and human NGAL monomer, the standard curve can be generated gravimetrically, by mass spectroscopy and by other techniques known in the art.

After the amount of human NGAL dimer and human monomer in the test sample is determined, the steps of the immunoassay are repeated. A new aliquot of the test sample is used in repeating the steps of the immunoassay (meaning that the aliquot used in repeating the steps of the immunoassay is different than the aliquot used to determine the amount of human NGAL dimer and human NGAL monomer). The steps of the immunoassay to be performed using the new aliquot of the test sample are identical to those described above, with the exception that one or more of the steps of the immunoassay are performed in the presence of at least one reducing agent. Alternatively, in lieu of performing one or more steps of the immunoassay in the presence of at least one reducing agent, the new aliquot of the test sample to be used repeating the steps of the immunoassay can be treated (namely, pretreated) with at least one reducing agent prior to performing the steps of the immunoassay. The use of the reducing agents in the immunoassay converts any dimer contained in the test sample to monomer. After formation of the labeled capture antibody/human NGAUdetection antibody complex, the amount of label in the complex is quantified using techniques known in the art. For example, if an enzymatic label is used, the labeled complex is reacted with a substrate for the label that gives a quantifiable reaction such as the development of color. If the label is a radioactive label, the label is quantified using a scintillation counter. If the label is a fluorescent label, the label is quantified by stimulating the label with a light of one color (which is known as the "excitation wavelength") and detecting another color (which is known as the "emission wavelength") that is emitted by the label in response to the stimulation. If the label is a chemiluminescent label, the label is quantified detecting the light emitted either visually or by using luminometers, x-ray film, high speed photographic film, a CCD camera, etc. Once the amount of the label in the complex has been quantified, the concentration of human NGAL monomer in the test sample is determined by use of a standard curve that has been generated using serial dilutions of human NGAL monomer of known concentration. Other than using serial dilutions of human NGAL monomer, the standard curve can be generated gravimetrically, by mass spectroscopy and by other techniques known in the art. Once the amount of human NGAL monomer has been determined, the ratio of human NGAL monomer to human NGAL dimer can be determined by comparing the amount of the (first or multiple) capture antibody/human NGAL complex determined when the immunoassay was performed without a reducing agent with the amount of the (first or multiple) capture antibody/human NGAL complex determined when the immunoassay was repeated in with a reducing agent.

By way of example, the steps of the immunoassay can be performed as follows:

(a) contacting at least one capture first antibody that binds to human NGAL with a test sample suspected of containing human NGAL monomer and human NGAL dimer, to form a first antibody/human NGAL complex, wherein the at least one capture first antibody is an antibody selected from the group consisting of: an antibody produced by murine hybridoma cell line 1-2322-455, wherein the cell line has ATCC Accession No. PTA-8024 and an antibody produced by murine hybridoma cell line 1-903-430, wherein the cell line has ATCC Accession No. PTA-8026;

(b) contacting the first antibody/human NGAL complex with a second antibody that binds to human NGAL and that has been conjugated to a detectable label to form a second antibody/human NGAL/first antibody complex, wherein the second antibody is an antibody selected from the group consisting of: an antibody produced by murine hybridoma cell line 1-2322-455, wherein the cell line has ATCC Accession No. PTA-8024 and an antibody produced by murine hybridoma cell line 1-903-430, wherein the cell line has ATCC Accession No. PTA-8026;

(c) determining the amount of the second antibody/human NGAL/first antibody complex formed in step (b), wherein steps (a) and (b) are performed in the absence of any reducing agents;

(d) repeating steps (a), (b) and (c) in the presence of or following treatment of the test sample with at least one reducing agent;

(e) determining the amount of the second antibody/human NGAL complex formed in the repeat of step (c); and

(f) determining the proportion of human NGAL monomer to human NGAL dimer in the test sample based on the amount of the second antibody/human NGAL/first antibody complex determined in step (c) and the amount of the second antibody/human NGAL/first antibody complex determined in step (e).

According to another aspect of the instant disclosure, the NGAL polypeptides also optimally can be employed in an improvement of an immunoassay for the detection of NGAL. This particular method as described herein can be employed in any NGAL immunoassay, with use of any antibodies for capture and/or detection. In one embodiment, the present disclosure thus provides, in an improvement of a method for detecting the presence of mammalian NGAL in a test sample, the method comprising:

(a) contacting a test sample suspected of containing mammalian NGAL with at least one antibody specific for said mammalian NGAL for a time and under conditions that allow the formation of a mammalian NGAL/antibody complex; and
(b) detecting any mammalian NGAL/antibody complex formed as indicating the presence of said mammalian NGAL;

wherein the improvement comprises employing as a calibrator or control a calibrator or control which is an NGAL polypeptide as described herein, particularly a
calibrator or control selected from the group consisting of: (a) glycosylated human NGAL comprising the sequence of SEQ ID NOS:2or 10, and (b) glycosylated human NGAL comprising the sequence of SEQ ID NOS:1 or 13. In one variation of the improved NGAL assay, the calibrator or control is glycosylated human NGAL comprising the sequence of SEQ ID NOS: 1 or 13.

Moreover, the anti-NGAL antibodies described for use herein also can be employed generally as an immunodiagnostic agent, e.g., in a method for detecting the presence of human NGAL antigen in a test sample. The method optionally comprises the steps of:

(1) contacting a test sample suspected of containing human NGAL with the immunodiagnostic reagent as described herein for a time and under conditions that allow formation of a human NGAL/antibody complex; and
(2) detecting any human NGAL/antibody complex formed as indicating the presence of the human NGAL antigen, wherein the immunodiagnostic reagent comprises one or more antibodies selected from the group consisting of:

(a) an antibody that specifically binds to a conformational epitope comprising amino acid residues 112, 118 and 147 of human NGAL protein as set forth in SEQ ID NOS:1, 2, 10 or 13;
(b) an isolated antibody that specifically binds to human NGAL, wherein the antibody has a variable heavy domain region comprising the amino acid sequence of SEQ ID NO:5;
(c) an isolated antibody that specifically bind to human NGAL, wherein the antibody has a variable light domain region comprising the amino acid sequence of SEQ ID NO:6;
(d) an isolated antibody that specifically binds to human NGAL, wherein the antibody has a variable heavy domain region comprising the amino acid sequence of SEQ ID NO:5 and a variable light domain region comprising the amino acid sequence of SEQ ID NO:6;
(e) an antibody produced by murine hybridoma cell line 1-2322-455 having ATCC Accession No. PTA-8024;
(f) an isolated antibody that specifically binds to human NGAL, wherein the antibody has a variable heavy domain region comprising the amino acid sequence of SEQ ID NO:7;
(g) an isolated antibody that specifically bind to human NGAL, wherein the antibody has a variable light domain region comprising the amino acid sequence of SEQ ID NO:8:
(h) an isolated antibody that specifically binds to human NGAL, wherein the antibody has a variable heavy domain region comprising the amino acid sequence of SEQ ID NO:7 and a variable light domain region comprising the amino acid sequence of SEQ ID NO:8; and
(i) an antibody produced by murine hybridoma cell line 1-903-430 having ATCC Accession No. PTA-8026.

More in particular, the instant invention pertains to a method for detecting the presence of human NGAL antigen in a test sample, said method comprising:

(1) contacting a test sample suspected of containing human NGAL with the immunodiagnostic reagent as set forth herein for a time and under conditions that allow formation of a human NGAL/antibody complex; and
(2) detecting any human NGAL/antibody complex formed as indicating the presence of said human NGAL antigen,

wherein said immunodiagnostic reagent comprises one or more antibodies selected from the group consisting of:

(a) an antibody that specifically binds to human NGAL protein at a conformational epitope comprising amino acid residues 112, 118 and 147 of human NGAL protein as set forth in SEQ ID NOS:1, 2, 10 or 13 and at least one additional amino acid that is residue 117 or 119 of wild-type human NGAL of SEQ ID NOS: 1 or 13;
(b) an isolated antibody that specifically binds to human NGAL, wherein said antibody has a variable heavy domain region comprising the amino acid sequence of SEQ ID NO:5 and a variable light domain region comprising the amino acid sequence of SEQ ID NO:6; and

(c) an antibody produced by murine hybridoma cell line 1-2322-455 having ATCC Accession No. PTA-8024;

and one or more antibodies selected from the group consisting of:

(d) an isolated antibody that specifically binds to human NGAL, wherein said antibody has a variable heavy domain region comprising the amino acid sequence of SEQ ID NO:7 and a variable light domain region comprising the amino acid sequence of SEQ ID NO:8; and

(e) an antibody produced by murine hybridoma cell line 1-903-430 having ATCC Accession No. PTA-8026.

The methods described herein (namely, the immunoassays and kits) can be used employed anytime any assessment of NGAL in any disease, disorder, condition, or evaluation of status is carried out. For instance, they can be used to evaluate the renal tubular cell injury status of a subject based on the determination of the level of NGAL present in the test sample. The subject to be evaluated can either currently have renal tubular cell injury or be at risk of developing renal tubular cell injury.

The methods described herein can be carried out on a subject after treatment of a subject for renal tubular cell injury or while the subject is currently experiencing renal tubular cell injury.

The methods described herein can be used to monitor the nephrotoxic side effects of drugs or other therapeutic agents in a subject.

The immunoassays also can be employed after an event experienced by a subject, such as after a surgical procedure (such as after cardiac surgery, coronary bypass surgery, cardiovascular surgery, vascular surgery or kidney transplantation), after the subject has experienced a diminished blood supply to the kidneys, if the subject has or is experiencing a medical condition selected from the group consisting of: impaired heart function, stroke, trauma, sepsis and dehydration, admittance of a subject to an intensive care unit, after administration to the subject of one or more pharmaceuticals, or after administration to the subject of one or more contrast agents.

The methods described herein also can be carried out or performed to assess chronic kidney disease.

It goes without saying that while certain embodiments herein are advantageous when employed to assess renal tubular cell injury status, the immunoassays and kits also optionally can be employed to assess NGAL in other diseases, e.g., cancer, sepsis, and any disease disorder or condition involving assessment of NGAL.

More specifically, in addition to assessment of renal disorders, diseases and injuries (see, e.g., U.S. Pat. App. Pub. Nos. 2008/0090304, 2008/0014644, 2008/0014604, 2007/0254370, and 2007/0037232), the assay and assay components as described herein optionally can also be employed in any other NGAL assay or in any other circumstance in which an assessment of NGAL levels or concentration might prove helpful: e.g., cancer-related assays (e.g., generally, or more specifically including but not limited to pancreatic cancer, breast cancer, ovarian/uterine cancer, leukemia, colon cancer, and brain cancer; see, e.g., U.S. Pat. App. Pub. No. 2007/0196876; see, also, U.S. Pat. Nos. 5,627,034 and 5,846,739); diagnosis of systemic inflammatory response syndrome (SIRS), sepsis, severe sepsis, septic shock and multiple organ dysfunction syndrome (MODS) (see, e.g., U.S. Pat. App. Pub. Nos. 2008/0050832 and 2007/0092911; see, also, U.S. Pat. No. 6,136,526); hematology applications (e.g., estimation of cell type); assessment of preeclampsia, obesity (metabolic syndrome), insulin resistance, hyperglycemia, tissue remodeling (when complexed with MMP-9; see, e.g., U.S. Pat. App. Pub. No. 2007/0105166 and U.S. Pat. No. 7,153,660), autoimmune diseases (e.g., rheumatoid arthritis, inflammatory bowel disease, multiple sclerosis), irritable bowel syndrome (see, e.g., U.S. Pat. App. Pub. Nos. 2008/0166719 and 2008/0085524), neurodegenerative disease, respiratory tract disease, inflammation, infection, periodontal disease (see, e.g., U.S. Pat. No. 5,866,432), and cardiovascular disease including venous thromboembolic disease (see, e.g., U.S. Pat. App. Pub. Nos. 2007/0269836), among others.

## G. NGAL Immunoassay Kits

The present invention also contemplates kits for detecting the presence of mammalian NGAL antigen in a test sample in improved assays for monomer. Such kits can comprise one or more of the immunodiagnostic reagents (e.g., antibodies) described herein. More specifically, if the kit is a kit for performing an immunoassay, the kit optionally can contain (1) at least one capture antibody that specifically binds to mammalian NGAL; (2) at least one conjugate; and (3) one or more instructions for performing the immunoassay. The immunodiagnostic reagents of the present invention can be included in such a test kit as a capture antibody, as a detection antibody or both as a capture antibody and a detection antibody. For example, an antibody produced by murine hybridoma cell line 1-2322-455 can be included in the kit as capture antibody and an antibody produced by murine hybridoma cell line 1-903-422 can be included in the kit as a detection antibody. Alternatively, an antibody produced by murine hybridoma cell line 1-903-422 can be included in the kit as a capture antibody and an antibody produced by hybridoma cell line 1-2322-455 can be included in the kit as a detection antibody. In still yet another alternative, an antibody produced by murine hybridoma cell line 1-2322-455 or an antibody produced by murine hybridoma cell line 1-903-422 can be included in the kit as a capture antibody and a different antibody included in the kit as a detection antibody. In still yet another alternative, an antibody produced by murine hybridoma cell line 1-2322-455 or an antibody produced by murine hybridoma cell line 1-903-422 can be included in the kit as a detection antibody and a different antibody included in the kit as a capture antibody. Optionally, the kit can also contain at least one calibrator or control. Any calibrator or control can be included in the kit. Preferably, however, the calibrator or control is mammalian NGAL, especially glycosylated human NGAL (e.g., wild-type or mutant) described previously herein.

Accordingly, the kits of the invention can comprise at least one calibrator, or at least one control, or a combination of at least one calibrator and at least one control, wherein the calibrator or control comprises a glycosylated mammalian NGAL of the present invention. Preferably, the at least one calibrator or at least one control is a glycosylated mammalian NGAL having the amino acid sequence selected from the group consisting of: SEQ ID NO:1, SEQ ID NO:2, SEQ ID NO:10, SEQ ID NO:13, and combinations of SEQ ID NOS:1, 2, 10 or 13. If the kit is a kit for performing an immunoassay, then the kit optionally further comprises: (1) at least one capture antibody that specifically binds to mammalian NGAL; (2) at least one conjugate; (3) one or more instructions for performing the immunoassay; or (4) or any combination of items (1)-(3).

Thus, the present invention further provides for diagnostic and quality control kits comprising one or more recombinant antibodies or mammalian NGAL of the invention. Optionally the assays, kits and kit components of the invention are optimized for use on commercial platforms (e.g., immunoassays on the Prism®, AxSYM®, ARCHITECT® and EIA (Bead) platforms of Abbott Laboratories, Abbott Park, IL, as well as other commercial and/or in vitro diagnostic assays). Additionally, the assays, kits and kit components can be employed in other formats, for example, on electrochemical or other hand-held or point-of-care assay systems. The present invention is, for example, applicable to the commercial Abbott Point of Care (i-STAT®, Abbott Laboratories, Abbott Park, IL) electrochemical immunoassay system that performs sandwich immunoassays for several cardiac markers, including TnI, CKMB and BNP. Immunosensors and methods of operating them in single-use test devices are described, for example, in US Patent Applications 20030170881, 20040018577, 20050054078 and 20060160164. Additional background on the manufacture of electrochemical and other types of immunosensors is found in US Patent No. 5,063,081.

Optionally the kits include quality control reagents (e.g., sensitivity panels, calibrators, and positive controls). Preparation of quality control reagents is well known in the art, and is described, e.g., on a variety of immunodiagnostic product insert sheets. NGAL sensitivity panel members optionally can be prepared in varying amounts containing, e.g., known quantities of NGAL antigen or antibody ranging from "low" to "high", e.g., by spiking known quantities of the NGAL antigen or antibodies according to the invention into an appropriate assay buffer (e.g., a phosphate buffer). These sensitivity panel members optionally are used to establish assay performance characteristics, and further optionally are useful indicators of the integrity of the immunoassay kit reagents, and the standardization of assays.

In another embodiment, the present invention provides for a quality control kit comprising one or more antigens and/or antibodies of the present invention for use as a sensitivity panel to evaluate assay performance characteristics and/or to quantitate and monitor the integrity of the antigen(s) used in the assay.

In still another embodiment, the mammalian NGAL (e.g., glycosylated mammalian NGAL) according to the invention can be employed as calibrators and/or controls. The antibodies provided in the kit can incorporate a detectable label, such as a fluorophore, radioactive moiety, enzyme, biotin/avidin label, chromophore, chemiluminescent label, or the like, or the kit may include reagents for labeling the antibodies or reagents for detecting the antibodies (e.g., detection antibodies) and/or for labeling the antigens or reagents for detecting the antigen. The antibodies, calibrators and/or controls can be provided in separate containers or pre-dispensed into an appropriate assay format, for example, into microtiter plates.

In yet another embodiment, the kit can comprise, either alone, with instructions, or with other aspects of the kit and kit components an immunodiagnostic agent that comprises one or more antibodies selected from the group consisting of:

(a) an antibody that specifically binds to a conformational epitope comprising amino acid residues 112, 118 and 147 of human NGAL protein as set forth in SEQ ID NOS:1, 2, 10 or 13;
(b) an isolated antibody that specifically binds to human NGAL, wherein the antibody has a variable heavy domain region comprising the amino acid sequence of SEQ ID NO:5;
(c) an isolated antibody that specifically bind to human NGAL, wherein the antibody has a variable light domain region comprising the amino acid sequence of SEQ ID NO:6;
(d) an isolated antibody that specifically binds to human NGAL, wherein the antibody has a variable heavy domain region comprising the amino acid sequence of SEQ ID NO:5 and a variable light domain region comprising the amino acid sequence of SEQ ID NO:6;
(e) an antibody produced by murine hybridoma cell line 1-2322-455 having ATCC Accession No. PTA-8024;
(f) an isolated antibody that specifically binds to human NGAL, wherein the antibody has a variable heavy domain region comprising the amino acid sequence of SEQ ID NO:7;

(g) an isolated antibody that specifically bind to human NGAL, wherein the antibody has a variable light domain region comprising the amino acid sequence of SEQ ID NO:8;

(h) an isolated antibody that specifically binds to human NGAL, wherein the antibody has a variable heavy domain region comprising the amino acid sequence of SEQ ID NO:7 and a variable light domain region comprising the amino acid sequence of SEQ ID NO:8; and

(i) an antibody produced by murine hybridoma cell line 1-903-430 having ATCC Accession No. PTA-8026.

More in particular, the instant invention pertains to a diagnostic kit for the detection of human NGAL comprising instructions and an immunodiagnostic reagent that comprises one or more antibodies selected from the group consisting of:

(a) an antibody that specifically binds to human NGAL protein at a conformational epitope comprising amino acid residues 112, 118 and 147 of human NGAL protein as set forth in SEQ ID NOS:1, 2, 10 or 13 and at least one additional amino acid that is residue 117 or 119 of wild-type human NGAL of SEQ ID NOS: 1 or 13;

(b) an isolated antibody that specifically binds to human NGAL, wherein said antibody has a variable heavy domain region comprising the amino acid sequence of SEQ ID NO:5 and a variable light domain region comprising the amino acid sequence of SEQ ID NO:6; and

(c) an antibody produced by murine hybridoma cell line 1-2322-455 having ATCC Accession No. PTA-8024; and one or more antibodies selected from the group consisting of:

(d) an isolated antibody that specifically binds to human NGAL, wherein said antibody has a variable heavy domain region comprising the amino acid sequence of SEQ ID NO:7 and a variable light domain region comprising the amino acid sequence of SEQ. ID. NO:8; and

(e) an antibody produced by murine hybridoma cell line 1-903-430 having ATCC Accession No. PTA-8026.

The kits can optionally include other reagents required to conduct a diagnostic assay or facilitate quality control evaluations, such as buffers, salts, enzymes, enzyme co-factors, substrates, detection reagents, and the like. Other components, such as buffers and solutions for the isolation and/or treatment of a test sample (e.g., pretreatment reagents), may also be included in the kit. The kit may additionally include one or more other controls. One or more of the components of the kit may be lyophilized and the kit may further comprise reagents suitable for the reconstitution of the lyophilized components.

The various components of the kit optionally are provided in suitable containers. As indicated above, one or more of the containers may be a microtiter plate. The kit further can include containers for holding or storing a sample (e.g., a container or cartridge for a blood or urine sample). Where appropriate, the kit may also optionally contain reaction vessels, mixing vessels and other components that facilitate the preparation of reagents or the test sample. The kit may also include one or more instruments for assisting with obtaining a test sample, such as a syringe, pipette, forceps, measured spoon, or the like.

The kit further can optionally include instructions for use, which may be provided in paper form or in computer-readable form, such as a disc, CD, DVD or the like.

By way of example, and not of limitation, examples of the present invention shall now be given.

## H. Adaptation of Method and Assay Kit

The kit (or components thereof), as well as the method of determining the concentration of NGAL antigen in a test sample by an assay as further described below, can be adapted for use in a variety of automated and semi-automated systems (including those wherein the solid phase comprises a microparticle), as described, e.g., in U.S. Patent Nos. 5,089,424 and 5,006,309, and as commercially marketed, e.g., by Abbott Laboratories (Abbott Park, IL) as ARCHITECT®.

Some of the differences between an automated or semi-automated system as compared to a non-automated system (e.g., ELISA) include the substrate to which the first specific binding partner (e.g., NGAL capture antibody) is attached (which can impact sandwich formation and analyte reactivity), and the length and timing of the capture, detection and/or any optional wash steps. Whereas a non-automated format such as an ELISA may require a relatively longer incubation time with sample and capture reagent (e.g., about 2 hours) an automated or semi-automated format (e.g., ARCHITECT®, Abbott Laboratories) may have a relatively shorter incubation time (e.g., approximately 18 minutes for ARCHITECT®). Similarly, whereas a non-automated format such as an ELISA may incubate a detection antibody such as the conjugate reagent for a relatively longer incubation time (e.g., about 2 hours), an automated or semi-automated format (e.g., ARCHITECT®) may have a relatively shorter incubation time (e.g., approximately 4 minutes for the ARCHITECT®).

Other platforms available from Abbott Laboratories include, but are not limited to, AxSYM®, IMx® (see, e.g.,

U.S. Pat. No. 5,294,404 ), PURISM®, EIA (bead), and Quantum™ II, as well as other platforms. Additionally, the assays, kits and kit components can be employed in other formats, for example, on electrochemical or other hand-held or point-of-care assay systems. The present disclosure is, for example, applicable to the commercial Abbott Point of Care (i-STAT®, Abbott Laboratories) electrochemical immunoassay system that performs sandwich immunoassays. Immunosensors and their methods of manufacture and operation in single-use test devices are described, for example in, U.S. Patent No. 5,063,081, U.S. Pat. App. Pub. No. 2003/0170881, U.S. Pat. App. Pub. No. 2004/0018577, U.S. Pat. App. Pub. No. 2005/0054078, and U.S. Pat. App. Pub. No. 2006/0160164.

In particular, with regard to the adaptation of an NGAL assay to the I-STAT® system, the following configuration is preferred. A microfabricated silicon chip is manufactured with a pair of gold amperometric working electrodes and a silver-silver chloride reference electrode. On one of the working electrodes, polystyrene beads (0.2 mm diameter) with immobilized capture antibody are adhered to a polymer coating of patterned polyvinyl alcohol over the electrode. This chip is assembled into an I-STAT® cartridge with a fluidics format suitable for immunoassay. On a portions of the wall of the sample-holding chamber of the cartridge there is a layer comprising the second detection antibody labeled with alkaline phosphatase (or other label). Within the fluid pouch of the cartridge is an aqueous reagent that includes p-aminophenol phosphate.

In operation, a sample suspected of containing NGAL antigen is added to the holding chamber of the test cartridge and the cartridge is inserted into the I-STAT® reader. After the second antibody (detection antibody) has dissolved into the sample, a pump element within the cartridge forces the sample into a conduit containing the chip. Here it is oscillated to promote formation of the sandwich between NGAL antigen, NGAL capture antibody, and the labeled detection antibody. In the penultimate step of the assay, fluid is forced out of the pouch and into the conduit to wash the sample off the chip and into a waste chamber. In the final step of the assay, the alkaline phosphatase label reacts with p-aminophenol phosphate to cleave the phosphate group and permit the liberated p-aminophenol to be electrochemically oxidized at the working electrode. Based on the measured current, the reader is able to calculate the amount of NGAL antigen in the sample by means of an embedded algorithm and factory-determined calibration curve.

It further goes without saying that the methods and kits as described herein necessarily encompass other reagents and methods for carrying out the immunoassay. For instance, encompassed are various buffers such as are known in the art and/or which can be readily prepared or optimized to be employed, e.g., for washing, as a conjugate diluent, and/or as a calibrator diluent. An exemplary conjugate diluent is ARCHITECT® conjugate diluent employed in certain kits (Abbott Laboratories, Abbott Park, IL) and containing 2-(N-morpholino)ethanesulfonic acid (MES), a salt, a protein blocker, an antimicrobial agent, and a detergent. An exemplary calibrator diluent is ARCHITECT@ human calibrator diluent employed in certain kits (Abbott Laboratories, Abbott Park, IL), which comprises a buffer containing MES, other salt, a protein blocker, and an antimicrobial agent.

### EXAMPLE 1: Exemplary NGAL Assay (Native NGAL) Using Commercial Antibodies

This example illustrates the measurement of native NGAL (as described herein, meaning purified from in vivo, e.g., from a material such as blood) with an exemplary immunoassay that employs two commercially available antibodies.

Native NGAL was purchased from Diagnostics Development (Uppsala, Sweden). The presence of NGAL monomers and dimers was determined using reducing and non-reducing SDS-PAGE. The gels illustrated that the native NGAL as purchased was comprised mostly of dimer (data not shown). A small amount of monomer may have been present but this could not be quantitated from the gels.

For these studies, a 135 $\mu$L sample of the native NGAL was mixed with 15 $\mu$L of 10 mM dithiothreitol (DTT) for one hour at room temperature. For the control condition, 15 $\mu$L of water was substituted for 10 mM DTT. The assay was executed on an automated ARCHITECT® i2000 analyzer (Abbott Laboratories, Abbott Park, IL). The assay was carried out by:

1. Mixing 10 $\mu$L of the test sample with 50 $\mu$L of microparticles coated with anti-NGAL antibody (HYB 211-01 from AntibodyShop A/S, Gentofte, Denmark).

2. Incubating the reaction mixture for approximately 18 minutes at 33 - 38 °C. The NGAL in the sample binds the anti-NGAL antibody on the microparticles.

3. Washing the microparticles with a phosphate buffer.

4. Adding 50 $\mu$L of acridinium-anti-NGAL antibody (HYB 211-02 from AntibodyShop A/S, Gentofte, Denmark) detector molecule to the reaction mixture.

5. Incubating the reaction mixture for approximately 4 minutes at 33 - 38°C. The acridinium-anti-NGAL antibody molecule forms a sandwich with NGAL bound to the microparticle antibody.

6. Washing the microparticles with a phosphate buffer.

7. Adding Pre-trigger (acid solution) and Trigger (basic solution) to cause the captured acridinium-NGAL label to emit light, which is measured by the instrument as RLUs (Relative Light Units).

RLUs are the designation for the optical unit of measurement utilized on the ARCHITECT® systems. The ARCHITECT® optics system is essentially a photomultiplier tube (PMT) that performs photon counting on the light emitted by the chemiluminescent reaction. The amount of light generated by the chemiluminescent reaction is proportional to the amount of acridinium tracer present in the reaction mixture, and thereby allows quantitation of the patient sample analyte that is also proportional to the amount of acridinium remaining in the reaction mixture at the time the chemiluminescent reaction occurs.

The term "Relative Light Units" comes from the relation of the photon counting to a certain amount of acridinium. Each optics module is calibrated with a set of acridinium standards. When the chemiluminescent reaction occurs, light is emitted and the photons are measured over a 3 second time period. The PMT converts the photons counted to digital signal, which is then sent to a circuit board for processing. The optics circuit board converts the digital signal from the PMT to an analog signal that is proportional to the photons counted, which is in turn proportional to the amount of acridinium present. This analog signal is then further processed to produce an RLU value. This relationship was established to produce a standard for calibration of the optics module, where the different acridinium standards have RLU values assigned to them. So, while the RLU unit itself is arbitrary, it is proportional (i.e., relative) to a certain amount of acridinium.

The assay response curve data obtained is shown in Table 1, below.

Table 1

| NGAL (ng/mL) | RLUs |
|---|---|
| 0 | 599 |
| 5 | 15330 |
| 50 | 150815 |
| 250 | 620011 |
| 600 | 1073295 |
| 1000 | 1321753 |

As can be seen from this data, and, as expected, in the Exemplary NGAL assay using native NGAL, as the amount of NGAL increased, the signal detected in the assay increased.

## EXAMPLE 2: Exemplary NGAL Assay Using Commercial Antibodies

This example compares the measurement of native NGAL monomer relative to native NGAL homodimer (dimer) with the exemplary immunoassay of Example 1.

The effect of the reduction of native NGAL by dithiothreitol ("DTT": 15 $\mu$L of 10 mM DTT added to 135 $\mu$L of sample, following by mixing and incubation for about an hour) on the measurement of NGAL also was explored, with results shown in Table 2 below.

Table 2

| Native NGAL Control (non-reduced) (ng/mL) | Native NGAL (plus DTT - reduced) (ng/mL) |
|---|---|
| 13.7 | 23.8 |

As can be seen from Table 2, following reduction of the native NGAL with DTT, the concentration of NGAL detected in the assay increased due to the conversion of NGAL dimer to NGAL monomer. This confirms that the immunoassay using commercial antibodies detects monomer preferentially to dimer. This further suggests that less NGAL is detected upon assay of non-reduced antigen samples with use of the immunoassay of Example 1, and implies that the true amount of NGAL in such samples potentially might be underestimated.

## EXAMPLE 3: Exemplary NGAL Assay Using Recombinant Human NGAL

This example illustrates the preferential measurement of wild-type recombinant human NGAL monomer relative to wild-type recombinant human NGAL dimer with an exemplary immunoassay as described in Example 1 using the HYB 211-01 and HYB 211-02 antibodies.

All wild-type NGAL recombinant antigen (rAg) monomer or dimer, as well as any mutant C87S NGAL rAg clones, subclones, hybrids, and hybridomas (including names and numbering), vectors, vector constructs, and antibodies, not specifically described herein are described in their entirety in U.S. Provisional Application Serial Number 60/981,470 filed October 19, 2007. For ease of reference, certain of these materials, in particular the illustrative depiction from U.S. Provisional Application Serial Number 60/981,470 of **Figure 1** (which shows the human NGAL wild-type antigen sequence (SEQ ID NO:1); also is included herein.

Wild-type recombinant human NGAL expressed in CHO cells was purified by gel filtration chromatography as either monomer or as dimer. The presence of NGAL monomers and dimers was confirmed using reducing and non-reducing SDS-PAGE. following preparation of the samples by gel filtration chromatography. The SDS-PAGE confirmed that the dimer preparation was comprised mostly of dimer (data not shown). Also, calibrators were prepared with the monomer and dimer NGAL in 25 mM inorganic phosphate, 0.5 M NaCl, 1% Triton X-100, 0.05% BSA, 5 $\mu$g/mL Sarafloxacin, 0.1% NaN$_3$, pH 7.5. A 200 $\mu$L sample of each dimer calibrator was mixed with 5 $\mu$L of 100 mM dithiothreitol (DTT) for two hours at room temperature. The assay was executed on an automated ARCHITECT® i2000 analyzer (Abbott Laboratories, Abbott Park, IL) as described in Example 1.

The response curve data are shown in Table 3 below and illustrate that (a) the immunoassay detects NGAL monomers preferentially to dimers and (b) conversion of dimers to monomers with reducing agent (e.g., DTT) makes the dimer calibrator assay response equivalent to the monomer calibrator response.

Table 3

| NGAL Target Concentration (ng/mL) | RLUs | | |
|---|---|---|---|
| | Monomer Calibrators | Dimer Calibrators | Dimer Calibrators plus DTT |
| 0 | 748 | 728 | 872 |
| 5 | 12037 | 4047 | 11360 |
| 50 | 101307 | 31475 | 100508 |
| 250 | 396455 | 141698 | 378465 |
| 600 | 690890 | 282183 | 675772 |
| 1000 | 885013 | 389558 | 855181 |

Table 4, below, shows the same data following conversion to concentration units using the monomer calibrators as the reference standard. Following reduction of dimers to monomers, the concentration of NGAL increased by an average of 248% due to the conversion of dimers to monomers.

Table 4

| NGAL Target Concentration (ng/mL) | Curve Fit Results (ng/mL)* | | | % Increase due to DTT |
|---|---|---|---|---|
| | Monomer Calibrators | Dimer Calibrators | Dimer Calibrators plus DTT | |
| 0 | 0.0 | 0.0 | 0.1 | - |
| 5 | 5.1 | 1.4 | 4.7 | 229 |
| 50 | 49.5 | 14.1 | 49.0 | 247 |
| 250 | 251.1 | 71.8 | 235.8 | 229 |
| 600 | 597.9 | 161.3 | 574.2 | 256 |
| 1000 | 1001.5 | 245.2 | 924.3 | 277 |
| * Curve fit with monomer calibrators | | | | |

**EXAMPLE 4: Immunoassay perfomance comparison using dimeric vs. monomeric NGAL Antigen**

In order to determine the accuracy of various calibrators, monomer, dimer and DTT-treated (2.4 mM DTT) wild-type NGAL calibrators prepared as described in Example 3 wee tested side-by-side on the ARCHITECT® NGAL assay using the HYB 211-01 and HYB 211-02 antibodies. Results obtained are presented in Table 5 (below).

Table 5

| Calibrators | NGAL Concentration (ng/mL) | Monomer Calibrators (RLUs) | Dimer Calibrators (RLUs) | DTT-Treated Dimer Calibrators (RLUs) |
|---|---|---|---|---|
| A | 0 | 747.5 | 728 | 872 |
| B | 5 | 12037 | 4046.5 | 11359.5 |
| C | 50 | 101307 | 31474.5 | 100507.5 |
| D | 250 | 396454.5 | 141698 | 378465 |
| E | 600 | 690890 | 282183 | 675772 |
| F | 1000 | 1885013 | 389557.5 | 855180.5 |

As can be seen from Table 5, a primary advantage for using mutant NGAL is that calibrators made with wild-type NGAL could slowly form dimers over time. Because the assay "sees" monomers better than dimers, this will be perceived in the assay as a loss of monomers (i.e., an instability). The calibration curve will shift due to this phenomenon. Additionally, e.g., when employed for production of commercial product, dimers will form for the wild-type NGAL during the manufacturing process because of the high concentrations necessary to purify the NGAL. This would lead to a process wherein reducing agents would be necessary, thereby complicating the manufacturing process further and very likely leading to stability problems with the "reduced" antigen.

As can be seen from the above, there was near-quantitative recovery of the dimer signal to match the monomer signal upon adding DTT. This would indicate that the loss of signal in the dimer calibrators is actually due to the antibody pair not being able to recognize the dimer as well as the monomer.

**EXAMPLE 5: Exemplary NGAL Assay Using Subject NGAL Antibodies**

This example illustrates the preferential measurement of recombinant human NGAL monomer relative to NGAL dimer with an alternate format using an immunoassay comprised of antibodies produced by the 1-2322-455 and 1-903-430 cell lines, further as described in U.S. Provisional Application Serial Number 60/981,471 filed October 19, 2007.

The recombinant human NGAL monomer and dimer calibrators described in Example 3 were used in this example. The assay was executed on an automated ARCHTTECT® i2000 analyzer (Abbott Laboratories, Abbott Park, IL) as follows:

1. Mixing 10 $\mu$L of the sample (NGAL monomer or dimer calibrator) with 50 $\mu$L of microparticles coated with anti-NGAL antibody produced by cell line 1-2322-455 and 20 $\mu$L of Tris(2-carboxyethyl)phosphine hydrochloride (TCEP). TCEP is a disulfide bond reducing agent.

2. Incubating the reaction mixture for approximately 18 minutes at 33 - 38°C The NGAL in the sample binds the anti-NGAL antibody on the microparticles. TCEP reduces dimers to monomers.

3. Adding 50 $\mu$L of acridinium-anti-NGAL antibody produced by cell line 1-903-430 detector molecule to the reaction mixture.

4. Incubating the reaction mixture for approximately 4 minutes at 33 - 38°C The acridinium-anti-NGAL antibody molecule forms a sandwich with NGAL bound to the microparticle antibody.

5. Washing the microparticles with a phosphate buffer.

6. Adding Pre-trigger (acid solution) and Trigger (basic solution) to cause the captured acridinium-NGAL label to emit light, which is measured by the instrument.

The response curve data are shown in below in Table 6.

Table 6

| NGAL Target Concentration (ng/mL) | RLUs | | | |
|---|---|---|---|---|
| | Monomer Calibrators | Monomer Calibrators plus TCEP | Dimer Calibrators | Dimer Calibrators plus TCEP |
| 0 | 788 | 917 | 924 | 843 |

(continued)

| NGAL Target Concentration (ng/mL) | RLUs | | | |
| --- | --- | --- | --- | --- |
| | Monomer Calibrators | Monomer Calibrators plus TCEP | Dimer Calibrators | Dimer Calibrators plus TCEP |
| 5 | 29336 | 29274 | 9117 | 14997 |
| 50 | 239507 | 241194 | 76836 | 131985 |
| 250 | 900324 | 928140 | 336240 | 535201 |
| 600 | 1505685 | 1528509 | 679832 | 988936 |
| 1000 | 1932662 | 1947234 | 992684 | 1371239 |

These results illustrate that the immunoassay detects NGAL monomers preferentially to dimers. It also shows that conversion of dimers to monomers can be accomplished with a reducing agent other than DTT, in this case, TCEP. Further, the reducing agent may be added during the assay incubation sequence as opposed to during a sample pretreatment step as described in Examples 1 and 2.

Table 7 shows the same data following conversion to concentration units using the monomer calibrators as the reference.

Table 7

| NGAL Target Concentration (ng/mL) | Curve Fit Results (ng/mL)* | | | |
| --- | --- | --- | --- | --- |
| | Monomer Calibrators | Monomer Calibrators plus TCEP | Dimer Calibrators | Dimer Calibrators plus TCEP |
| 0 | 0.0 | 0.0 | 0.0 | 0.0 |
| 5 | 4.7 | 4.6 | 1.2 | 2.2 |
| 50 | 48.2 | 48.6 | 13.5 | 24.5 |
| 250 | 254.7 | 266.3 | 71.7 | 126.5 |
| 600 | 590.6 | 607.8 | 172.6 | 292.5 |
| 1000 | 1006.9 | 1025.6 | 294.2 | 497.4 |
| * Curve fit with monomer calibrators | | | | |

**EXAMPLE 6: Increased Specificity of ARCHITECT® Assay Compared to Commercial ELISA Kit**

This example illustrates the preferential measurement of recombinant human NGAL monomer relative to NGAL dimer with an alternate format using an immunoassay comprised of antibodies produced by the 1-2322-455 and 1-903-430 cell lines, further as described in U.S. Provisional Application Serial Number 60/981,471 filed October 19, 2007 compared to the AntibodyShop NGAL Rapid ELISA Kit (AntibodyShop A/S, Gentofte, Denmark).

The recombinant human NGAL dimer calibrators described in Example 3 were used in this example. The dimer calibrators were used for these experiments at concentrations of 1500 ng/mL ("NGAL Dimer High") and 150 ng/mL ("NGAL Dimer Low").

The ARCHITECT® assay was executed on an automated ARCHITECT® i2000 analyzer (Abbott Laboratories, Abbott Park, IL) as follows:

1. Mixing 10 μL of the sample (NGAL dimer calibrator) with 90 μL 0.5% saline.
2. Incubating the reaction mixture for approximately 7 minutes at 33 - 38°C.
3. Removing 25 μL of this mixture and mixing with 50 μL of microparticles coated with anti-NGAL antibody produced by cell line 1-2322-455 and 90 μL ARCHTTECT® line diluent (phosphate buffered saline with Brij detergent).
4. Incubating the reaction mixture for approximately 18 minutes at 33 - 38°C The NGAL in the sample binds the anti-NGAL antibody on the microparticles.
5. Adding 50 μL of acridinium-anti-NGAL antibody produced by cell line 1-903-430 detector molecule to the reaction mixture.

6. Incubating the reaction mixture for approximately 4 minutes at 33 - 38°C The acridinium-anti-NGAL antibody molecule forms a sandwich with NGAL bound to the microparticle antibody.

7. Washing the microparticles with a phosphate buffer.

8. Adding Pre-trigger (acid solution) and Trigger (basic solution) to cause the captured acridinium-NGAL label to emit light, which is measured by the instrument.

The AntibodyShop NGAL Rapid ELISA Kit was executed as described in the manufacturer's instructions (Kit 037, Revision 2006-09-EN).

An exemplary response obtained with use of the NGAL dimer is shown below in Table 8.

Table 8

| Sample | ELISA (ng/mL) | ARCHITECT (ng/mL) |
|---|---|---|
| NGAL Dimer High | 477.6 | 308.6 |
| NGAL Dimer Low | 30.5 | 33.9 |

These results coupled with those, e.g., in Example 4 (showing improved specificity extending down to 5 ng/mL) confirm that the assay as described herein shows improved specificity throughout the calibration range of the assay (e.g., from about 1 ng/mL up to about 2000 ng/ml). The results furthermore confirm that the ARCHITECT® NGAL assay is more specific than the AntibodyShop NGAL Rapid ELISA Kit at higher dimer concentrations because the AntibodyShop NGAL Rapid ELISA Kit detects more dimer than the ARCHITECT® assay. It follows that at the higher concentrations of NGAL that are associated with acute kidney injury the ARCHTTECT® NGAL assay is more specific for monomer than the AntibodyShop NGAL Rapid ELISA Kit. In other words, ARCHITECT® NGAL assay is more specific for monomer at a level of NGAL greater than about 100 ng/mL.

**EXAMPLE 7: ATCC Deposit Information**

The wild-type NGAL rAg CHO 662 cell line was deposited with the American Type Culture Collection (ATCC) at 10801 University Boulevard, Manassas, VA 20110-2209 on November 21, 2006 and received ATCC Accession No. PTA-8020.

The mutant NGAL rAg CHO C87S cell line (CHO cell clone #734, also known as "mutant C87S NGAL rAg CHO 734") was deposited with the American Type Culture Collection (ATCC) at 10801 University Boulevard, Manassas, VA 20110-2209 on January 23, 2007 and received ATCC Accession No. PTA-8168.

Murine hybridoma cell lines 1-903-430 and 1-2322-455 were each deposited with the American Type Culture Collection (hereinafter referred to as "A.T.C.C"), 10801 University Blvd., Manassas, VA. 20110-2209, on November 21, 2006. Cell line 1-903-430 was assigned ATCC Accession No. PTA-8026. Cell line 1-2322-455 was assigned ATCC Accession No. PTA-8024.

One skilled in the art would readily appreciate that the present invention is well adapted to carry out the objects and obtain the ends and advantages mentioned, as well as those inherent therein. The molecular complexes and the methods, procedures, treatments, molecules, specific compounds described herein are presently representative of preferred embodiments, are exemplary, and are not intended as limitations on the scope of the invention.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as encompassed by the appended claims. Moreover, it should be understood that where certain terms are defined under "Definitions" and are otherwise defined, described, or discussed elsewhere in the "Detailed Description," all such definitions, descriptions, and discussions are intended to be attributed to such terms. There also is no intention in the use of such terms and expressions of excluding any equivalents of the features shown and described or portions thereof. Furthermore, while subheadings, e.g., "Definitions," are used in the "Detailed Description," such use is solely for ease of reference and is not intended to limit any disclosure made in one section to that section only; rather, any disclosure

# EP 2 225 268 B1

made under one subheading is intended to constitute a disclosure under each and every other subheading.

Application No: PCTUS0880331 Version No: 1.0

**Input Set:**

**Output Set:**

| | |
|---|---|
| **Started:** | 2008-10-17 18:02:16.852 |
| **Finished:** | 2008-10-17 18:02:19.208 |
| **Elapsed:** | 0 hr(s) 0 min(s) 2 sec(s) 356 ms |
| **Total Warnings:** | 11 |
| **Total Errors:** | 0 |
| **No. of SeqIDs Defined:** | 13 |
| **Actual SeqID Count:** | 13 |

| Error code | Error Description |
|---|---|
| W 213 | Artificial or Unknown found in <213> in SEQ ID (2) |
| W 213 | Artificial or Unknown found in <213> in SEQ ID (4) |
| W 213 | Artificial or Unknown found in <213> in SEQ ID (5) |
| W 213 | Artificial or Unknown found in <213> in SEQ ID (6) |
| W 213 | Artificial or Unknown found in <213> in SEQ ID (7) |
| W 213 | Artificial or Unknown found in <213> in SEQ ID (8) |
| W 213 | Artificial or Unknown found in <213> in SEQ ID (9) |
| W 213 | Artificial or Unknown found in <213> in SEQ ID (10) |
| W 213 | Artificial or Unknown found in <213> in SEQ ID (11) |
| W 213 | Artificial or Unknown found in <213> in SEQ ID (12) |
| W 213 | Artificial or Unknown found in <213> in SEQ ID (13) |

## Claims

1. A method for determining the amount of human NGAL monomer in a test sample, the method comprising the steps of:

   (a) contacting a test sample suspected of containing human NGAL monomer and human NGAL dimer with at least one first antibody so as to form a first antibody/human NGAL complex, wherein said at least one capture first antibody binds to human NGAL and is an antibody selected from the group consisting of an antibody produced by murine hybridoma cell line 1-2322-455 having ATCC Accession No. PTA-8024 and an antibody produced by murine hybridoma cell line 1-903-430 having ATCC Accession No. PTA-8026;
   (b) contacting said antibody/human NGAL complex with a second antibody that binds to human NGAL and that has been conjugated to a detectable label to form a second antibody/human NGAL/first antibody complex, wherein said second antibody differs from said first antibody and is an antibody selected from the group consisting of: an antibody produced by murine hybridoma cell line 1-2322-455 having ATCC Accession No. PTA-8024 and an antibody produced by murine hybridoma cell line 1-903-430 having ATCC Accession No. PTA-8026; and
   (c) determining with at least about 75% specificity the amount of human NGAL monomer contained in the test sample based on the amount of the second antibody/human NGAL/first antibody complex formed in step (b).

2. The method of claim 1, wherein said method is performed in the absence of any reducing agents.

3. The method of any of the preceding claims, wherein steps (a), (b) and (c) are performed in the presence of or following treatment of said test sample with at least one reducing agent.

4. The method of any of the preceding claims, wherein said method is about four times as specific for NGAL monomer a compared to NGAL dimer at a level of urine NGAL greater than about 100 ng/mL.

5. A method for determining the proportion of human NGAL monomer to human NGAL dimer contained in a test sample, said method comprising the steps of:

(a) contacting a test sample suspected of containing human NGAL monomer and human NGAL dimer with at least one first antibody so as to form a first antibody/human NGAL complex, wherein said at least one first antibody binds to human NGAL and is an antibody selected from the group consisting of an antibody produced by murine hybridoma cell line 1-2322-455 having ATCC Accession No. PTA-8024 and an antibody produced by murine hybridoma cell line 1-903-430 having ATCC Accession No. PTA-8026;
(b) contacting said first antibody/human NGAL complex with a second antibody that binds to human NGAL and that has been conjugated to a detectable label so as to form a second antibody/human NGAL/first antibody complex, wherein said second antibody differs from said first antibody and is an antibody selected from the group consisting of an antibody produced by murine hybridoma cell line 1-2322-455 having ATCC Accession No. PTA-8024 and an antibody produced by murine hybridoma cell line 1-903-430 having ATCC Accession No. PTA-8026;
(c) determining the amount of the second antibody/human NGAL/first antibody complex formed in step (b), wherein steps (a) and (b) are performed in the absence of any reducing agents;
(d) determining the amount of said second antibody/human NGAL complex formed in step (b), wherein steps (a) and (b) are performed in the presence of or following treatment of said test sample with at least one reducing agent; and
(e) determining the proportion of human NGAL monomer to human NGAL dimer in said test sample based on the amount of the second antibody/human NGAL/first antibody complex determined in step (c) and the amount of the second antibody/human NGAL/first antibody complex determined in step (d).

6. The method of claim 1 or 5, wherein said at least one first antibody is immobilized on a solid phase either prior to or following contacting with said test sample.

7. The method of claim 6, wherein said at least one first antibody is immobilized on a solid phase prior to formation of said second antibody/human NGAL/first antibody complex.

8. The method of claim 7, wherein said at least one first antibody is immobilized on a solid phase either prior to or after formation of said first antibody/human NGAL. complex.

9. The method of any of the preceding claims 3 or 5-8, wherein said at least one reducing agent is selected from the group consisting of dithiothreitol, 2-mercaptoethanol, 2-mercaptoethylamine, and Tris(2-carboxyethyl)phosphine.

10. The method of any of the preceding claims 3 or 5-9, wherein said at least one reducing agent is present in the amount of from about 0.1 mM to about 500 mM.

11. The method of any of the preceding claims 5-10, wherein said test sample is a urine or blood sample.

12. A method for evaluating the renal tubular cell injury status of a subject, in which method the level of human NGAL monomer and/or the proportion of human NGAL monomer to human NGAL dimer in a test sample is determined according to one or more of claims 1-11.

13. The method of claim 12, wherein said renal tubular cell injury comprises an injury selected from the group consisting of an ischemic renal injury, a nephrotoxic injury, and an other injury that affects the tubular cells of the kidney.

14. A method for detecting the presence of human NGAL antigen in a test sample, said method comprising:

(1) contacting a test sample suspected of containing human NGAL with the immunodiagnostic reagent as set forth herein for a time and under conditions that allow formation of a human NGAL/antibody complex; and
(2) detecting any human NGAL/antibody complex formed as indicating the presence of said human NGAL antigen,

wherein said immunodiagnostic reagent comprises one or more antibodies selected from the group consisting of:

(a) an antibody that specifically binds to human NGAL protein at a conformational epitope comprising amino acid residues 112, 118 and 147 of human NGAL protein as set forth in SEQ ID NOS: 1, 2, 10 or 13 and at least one additional amino acid that is residue 117 or 119 of wild-type human NOAL of SEQ ID NOS: 1 or 13;
(b) an isolated antibody that specifically binds to human NGAL, wherein said antibody has a variable heavy domain region comprising the amino acid sequence of SEQ ID NO:5 and a variable light domain region comprising the amino acid sequence of SEQ ID NO:6; and
(c) an antibody produced by murine hybridoma cell line 1-2322-455 having ATCC Accession No. PTA-8024;

and one or more antibodies selected from the group consisting of:

(d) an isolated antibody that specifically binds to human NGAL, wherein said antibody has a variable heavy domain region comprising the amino acid sequence of SEQ ID NO:7 and a variable light domain region comprising the amino acid sequence of SEQ ID NO:8; and
(e) an antibody produced by murine hybridoma cell line 1-903-430 having ATCC Accession No. PTA-8026.

15. The method of any of claims 1-14, wherein the method is adapted for use in an automated system or semi-automated system.

16. A diagnostic kit for the detection of human NGAL comprising instructions and an immunodiagnostic reagent that comprises one or more antibodies selected from the group consisting of:

(a) an antibody that specifically binds to human NGAL protein at a conformational epitope comprising amino acid residues 112,118 and 147 of human NGAL protein as set forth in SEQ ID NOS:1, 2, 10 or 13 and at least one additional amino acid that is residue 117 or 119 of wild-type human NGAL of SEQ ID NOS: 1 or 13;
(b) an isolated antibody that specifically binds to human NGAL, wherein said antibody has a variable heavy domain region comprising the amino acid sequence of SEQ ID NO:5 and a variable light domain region comprising the amino acid sequence of SEQ ID NO:6; and
(c) an antibody produced by murine hybridoma cell line 1-2322-455 having ATCC Accession No. PTA-8024; and one or more antibodies selected from the group consisting of:
(d) an isolated antibody that specifically binds to human NGAL, wherein said antibody has a variable heavy domain region comprising the amino acid sequence of SEQ ID NO:7 and a variable light domain region comprising the amino acid sequence of SEQ. ID. NO:8; and
(e) an antibody produced by murine hybridoma cell line 1-903-430 having ATCC Accession No. PTA-8026.

**Patentansprüche**

1. Ein Verfahren zur Bestimmung der Menge an menschlichem NGAL-Monomer in einer Testprobe, wobei das Verfahren die folgenden Schritte umfasst:

(a) In-Kontakt-bringen einer Testprobe, von der vermutet wird, dass sie menschliches NGAL-Monomer und menschliches NGAL-Dimer enthält, mit mindestens einem ersten Antikörper, um einen ersten Antikörper/ menschliches NGAL-Komplex zu bilden, worin der mindestens eine erste Einfang-Antikörper an menschliches NGAL bindet und ein Antikörper ist, gewählt aus der Gruppe bestehend aus einem Antikörper produziert von der Maushybridomzelllinie 1-2322-455 mit der ATCC Zugriffsnummer PTA-8024, und einem Antikörper produziert durch die Maushybridomzelllinie 1-903-430 mit der ATCC Zugriffsnummer PTA-8026;
(b) In-Kontakt-bringen des Antikörper/menschliches NGAL-Komplexes mit einem zweiten Antikörper, der an menschliches NGAL bindet, und der an einen detektierbaren Marker konjugiert wurde, um einen zweiten Antikörper/menschliches NGAL/erster Antikörper-Komplex zu bilden, worin der zweite Antikörper sich von dem ersten Antikörper unterscheidet und ein Antikörper ist, der gewählt ist aus der Gruppe bestehend aus: einem Antikörper produziert durch die Maushybridomzelllinie 1-2322-455 mit der ATCC Zugriffsnummer PTA-8024, und einem Antikörper produziert durch die Maushybridomzelllinie 1-903-430 mit der ATCC Zugriffsnummer PTA-8026; und
(c) Bestimmen mit mindestens ungefähr 75 %iger Spezifität der Menge an menschlichem NGAL-Monomer, die in der Testprobe enthalten ist, basierend auf der Menge des zweiten Antikörper/menschliches NGAL/erster Antikörper-Komplexes, der in Schritt (b) gebildet wurde.

**2.** Das Verfahren gemäß Anspruch 1, worin das Verfahren in der Abwesenheit von irgendwelchen Reduktionsmitteln durchgeführt wird.

**3.** Das Verfahren gemäß irgendeinem der zuvor genannten Ansprüche, worin Schritte (a), (b) und (c) in der Anwesenheit von oder nach Behandlung der Testprobe mit mindestens einem Reduktionsmittel durchgeführt werden.

**4.** Das Verfahren gemäß irgendeinem der vorhergehenden Ansprüche, worin das Verfahren ungefähr vier mal so spezifisch für NGAL-Monomer ist verglichen zu NGAL-Dimer, bei einem Level von Urin-NGAL von größer als ungefähr 100 ng/ml.

**5.** Ein Verfahren zur Bestimmung des Verhältnisses von menschlichem NGAL-Monomer zu menschlichem NGAL-Dimer, enthalten in einer Testprobe, wobei das Verfahren die folgenden Schritte umfasst:

(a) In-Kontakt-bringen einer Testprobe, von der vermutet wird, dass sie menschliches NGAL-Monomer und menschliches NGAL-Dimer enthält, mit mindestens einem ersten Antikörper, um einen ersten Antikörper/ menschliches NGAL-Komplex zu bilden, worin dieser mindestens eine Antikörper an menschliches NGAL bindet und ein Antikörper ist, gewählt aus der Gruppe bestehend aus einem Antikörper produziert durch die Maushybridomzelllinie 1-2322-455 mit der ATCC Zugriffsnummer PTA-8024, und einem Antikörper, produziert durch die Maushybridomzelllinie 1-903-430 mit der ATCC Zugriffsnummer PTA-8026;

(b) In-Kontakt-bringen des ersten Antikörper/menschliches NGAL-Komplexes mit einem zweiten Antikörper, der an menschliches NGAL bindet und der an einen detektierbaren Marker konjugiert wurde, um einen zweiten Antikörper/menschliches NGAL/erster Antikörper-Komplex zu bilden, worin der zweite Antikörper sich von dem ersten Antikörper unterscheidet und ein Antikörper ist, der gewählt ist aus der Gruppe bestehend aus: einem Antikörper produziert durch Maushybridomzelllinie 1-2322-455 mit der ATCC Zugriffsnummer PTA-8024 und einem Antikörper produziert durch die Maushybridomzelllinie 1-903-430 mit der ATCC Zugriffsnummer PTA-8026;

(c) Bestimmen der Menge des zweiten Antikörper/menschliches NGAL/erster Antikörper-Komplexes, der in Schritt (b) gebildet wurde, worin Schritte (a) und (b) in der Abwesenheit irgendwelcher Reduktionsmittel durchgeführt werden;

(d) Bestimmen der Menge des zweiten Antikörper/menschliches NGAL-Komplexes, der in Schritt (b) gebildet wurde, worin Schritte (a) und (b) in der Anwesenheit von oder nach Behandlung der Testprobe mit mindestens einem Reduktionsmittel durchgeführt werden; und

(e) Bestimmen des Verhältnisses von menschlichem NGAL-Monomer zu menschlichem NGAL-Dimer in der Testprobe, basierend auf der Menge des zweiten Antikörper/menschliches NGAL/erster Antikörper-Komplexes, die in Schritt (c) bestimmt wurde und der Menge des zweiten Antikörper/menschliches NGAL/erster Antikörper-Komplexes, die in Schritt (d) bestimmt wurde.

**6.** Das Verfahren gemäß Anspruch 1 oder 5, worin der mindestens eine erste Antikörper entweder vor oder nach dem In-Kontakt-bringen mit der Testprobe auf einer Festphase immobilisiert wird.

**7.** Das Verfahren gemäß Anspruch 6, worin der mindestens eine erste Antikörper vor der Bildung des zweiten Antikörper/menschliches NGAL/erster Antikörper-Komplexes auf einer Festphase immobilisiert wird.

**8.** Das Verfahren gemäß Anspruch 7, worin der mindestens eine erste Antikörper entweder vor oder nach der Bildung des ersten Antikörper/menschliches NGAL-Komplexes auf einer Festphase immobilisiert wird.

**9.** Das Verfahren gemäß irgendeinem der vorhergehenden Ansprüche 3 oder 5 bis 8, worin das mindestens eine Reduktionsmittel gewählt ist aus der Gruppe bestehend aus Dithiothreitol, 2-Mercaptoethanol, 2-Mercaptoethylamin, und Tris(2-carboxyethyl)phosphin.

**10.** Das Verfahren gemäß irgendeinem der vorhergehenden Ansprüche 3 oder 5 bis 9, worin das mindestens eine Reduktionsmittel in der Menge von ungefähr 0,1 mM bis ungefähr 500 mM vorhanden ist.

**11.** Das Verfahren gemäß irgendeinem der vorhergehenden Ansprüche 5 bis 10, worin die Testprobe eine Urin- oder Blutprobe ist.

**12.** Ein Verfahren zur Bewertung des renalen tubulären Zellverletzungsstatus einer Person, wobei in dem Verfahren der Spiegel an menschlichem NGAL-Monomer und / oder das Verhältnis von menschlichem NGAL-Monomer zu menschlichem NGAL-Dimer in einer Testprobe gemäß einem oder mehreren der Ansprüche 1 bis 11 bestimmt wird.

13. Das Verfahren gemäß Anspruch 12, worin die renale tubuläre Zellverletzung eine Verletzung umfasst, gewählt aus der Gruppe bestehend aus einer ischämischen renalen Verletzung, einer nephrotoxischen Verletzung und einer anderen Verletzung, die die tubulären Zellen der Niere schädigt.

14. Ein Verfahren zur Detektion des Vorhandenseins von menschlichem NGAL-Antigen in einer Testprobe, wobei das Verfahren folgendes umfasst:

(1) In-Kontakt-bringen einer Testprobe, von der vermutet wird, dass sie menschliches NGAL enthält, mit dem immunodiagnostischen Reagenz wie hierin dargelegt, für eine Zeit und unter Bedingungen, die die Bildung eines menschliches NGAL/Antikörper-Komplexes erlauben; und
(2) Detektieren von jeglichem menschliches NGAL /Antikörper-Komplex, der sich gebildet hat, als Indiz für das Vorhandensein von menschlichem NGAL-Antigen,

worin das immunodiagnostische Reagenz einen oder mehrere Antikörper umfasst, die gewählt sind aus der Gruppe bestehend aus:

(a) einem Antikörper, der spezifisch an menschliches NGAL-Protein bindet an einem Konformationsepitop umfassend Aminosäurereste 112, 118 und 147 von menschlichem NGAL-Protein, wie in Sequenzidentifikationsnummern 1, 2, 10 oder 13 dargelegt, und mindestens eine zusätzliche Aminosäure, die Rest 117 oder 119 von menschlichem Wildtyp-NGAL der Sequenzidentifikationsnummern 1 oder 13 ist;
(b) einem isolierten Antikörper, der spezifisch an menschliches NGAL bindet, worin der Antikörper eine variable Domain der schweren Kette hat umfassend die Aminosäuresequenz von Sequenzidentifikationsnummer 5, und eine variable Domain der leichten Kette umfassend die Aminosäuresequenz von Sequenzidentifikationsnummer 6; und
(c) einem Antikörper produziert durch die Maushybridomzelllinie 1-2322-455 mit der ATCC Zugriffsnummer PTA-8024; und einem oder mehreren Antikörpern gewählt aus der Gruppe bestehend aus:
(d) einem isolierten Antikörper, der spezifisch an menschliches NGAL bindet, worin der Antikörper eine variable Domain der schweren Kette hat umfassend die Aminosäuresequenz von Sequenzidentifikationsnummer 7, und eine variable Domain der leichten Kette umfassend die Aminosäuresequenz von Sequenzidentifikationsnummer 8; und
(e) einem Antikörper produziert durch die Maushybridomzelllinie 1-903-430 mit der ATCC Zugriffsnummer PTA-8026.

15. Das Verfahren gemäß irgendeinem der Ansprüche 1 bis 14, worin das Verfahren für die Verwendung in einem automatisierten System oder einem halbautomatisierten System angepasst ist.

16. Ein diagnostischer Kit für die Detektion von menschlichem NGAL umfassend Anleitungen und ein immunodiagnostisches Reagenz, das einen oder mehrere Antikörper umfasst, die gewählt sind aus der Gruppe bestehend aus:

(a) einem Antikörper, der spezifisch an menschliches NGAL-Protein bindet an einem Konformationsepitop umfassend Aminosäurereste 112, 118 und 147 von menschlichem NGAL-Protein, wie in Sequenzidentifikationsnummern 1, 2, 10 oder 13 dargelegt, und mindestens eine zusätzliche Aminosäure, die Rest 117 oder 119 von menschlichem Wildtyp-NGAL der Sequenzidentifikationsnummer 1 oder 13 ist;
(b) einem isolierten Antikörper, der spezifisch an menschliches NGAL bindet, worin der Antikörper eine variable Region der schweren Kette hat umfassend die Aminosäuresequenz von Sequenzidentifikationsnummer 5, und eine variable Region der leichten Kette umfassend die Aminosäuresequenz von Sequenzidentifikationsnummer 6; und
(c) einem Antikörper, produziert durch die Maushybridomzelllinie 1-2322-455 mit der ATCC Zugriffsnummer PTA-8024;
und einem oder mehreren Antikörpern gewählt aus der Gruppe bestehend aus:
(d) einem isolierten Antikörper, der spezifisch an menschliches NGAL bindet, worin der Antikörper eine variable Region der schweren Kette hat umfassend die Aminosäuresequenz von Sequenzidentifikationsnummer 7, und eine variable Region der leichten Kette umfassend die Aminosäuresequenz von Sequenzidentifikationsnummer 8; und
(e) einem Antikörper produziert durch die Maushybridomzelllinie 1-903-430 mit der ATCC-Zugangsnummer PTA-8026.

**Revendications**

1. Procédé pour déterminer la quantité de monomère de NGAL humaine dans un échantillon test, le procédé comprenant les étapes de :

   (a) mise en contact d'un échantillon test suspecté de contenir un monomère de NGAL humaine et un dimère de NGAL humaine avec au moins un premier anticorps de manière à former un complexe premier anticorps/ NGAL humaine, où ledit au moins un premier anticorps de capture se lie à NGAL humaine et est un anticorps choisi dans le groupe consistant en un anticorps produit par la lignée cellulaire d'hybridome murine 1-2322-455 ayant le numéro d'ordre ATCC PTA-8024 et un anticorps produit par la lignée cellulaire d'hybridome murine 1-903-430 ayant le numéro d'ordre ATCC PTA-8026 ;
   (b) mise en contact dudit complexe anticorps/NGAL humaine avec un second anticorps qui se lie à NGAL humaine et qui a été conjugué à un marqueur détectable pour former un complexe second anticorps/NGAL humaine/premier anticorps, où ledit second anticorps diffère dudit premier anticorps et est un anticorps choisi dans le groupe consistant en : un anticorps produit par la lignée cellulaire d'hybridome murine 1-2322-455 ayant le numéro d'ordre ATCC PTA-8024 et un anticorps produit par la lignée cellulaire d'hybridome murine 1-903-430 ayant le numéro d'ordre ATCC PTA-8026 ; et
   (c) détermination avec une spécificité d'au moins environ 75 % de la quantité de monomère de NGAL humaine contenue dans l'échantillon test sur la base de la quantité du complexe second anticorps/NGAL humaine/ premier anticorps formé dans l'étape (b).

2. Procédé selon la revendication 1, où ledit procédé est mis en oeuvre en l'absence de tout agent réducteur.

3. Procédé selon l'une quelconque des revendications précédentes, où les étapes (a), (b) et (c) sont mises en oeuvre en présence de ou après traitement dudit échantillon test avec au moins un agent réducteur.

4. Procédé selon l'une quelconque des revendications précédentes, où ledit procédé est environ 4 fois plus spécifique pour un monomère de NGAL que pour un dimère de NGAL à un niveau de NGAL dans l'urine supérieur à environ 100 ng/mL.

5. Procédé pour déterminer la proportion de monomère de NGAL humaine par rapport au dimère de NGAL humaine contenu dans un échantillon test, ledit procédé comprenant les étapes de :

   (a) mise en contact d'un échantillon test suspecté de contenir un monomère de NGAL humaine et un dimère de NGAL humaine avec au moins un premier anticorps de manière à former un complexe premier anticorps/ NGAL humaine, où ledit au moins un premier anticorps se lie à NGAL humaine et est un anticorps choisi dans le groupe consistant en un anticorps produit par la lignée cellulaire d'hybridome murine 1-2322-455 ayant le numéro d'ordre ATCC PTA-8024 et un anticorps produit par la lignée cellulaire d'hybridome murine 1-903-430 ayant le numéro d'ordre ATCC PTA-8026 ;
   (b) mise en contact dudit complexe premier anticorps/NGAL humaine avec un second anticorps qui se lie à NGAL humaine et qui a été conjugué à un marqueur détectable de manière à former un complexe second anticorps/NGAL humaine/premier anticorps, où ledit second anticorps diffère dudit premier anticorps et est un anticorps choisi dans le groupe consistant en un anticorps produit par la lignée cellulaire d'hybridome murine 1-2322-455 ayant le numéro d'ordre ATCC PTA-8024 et un anticorps produit par la lignée cellulaire d'hybridome murine 1-903-430 ayant le numéro d'ordre ATCC PTA-8026 ;
   (c) détermination de la quantité du complexe second anticorps/NGAL humaine/premier anticorps formé dans l'étape (b), où les étapes (a) et (b) sont mises en oeuvre en l'absence de tout agent réducteur ;
   (d) détermination de la quantité dudit complexe second anticorps/NGAL humaine formé dans l'étape (b), où les étapes (a) et (b) sont mises en oeuvre en présence de ou après traitement dudit échantillon test avec au moins un agent réducteur ; et
   (e) détermination de la proportion de monomère de NGAL humaine par rapport au dimère de NGAL humaine dans ledit échantillon test sur la base de la quantité du complexe second anticorps/NGAL humaine/premier anticorps déterminée dans l'étape (c) et de la quantité du complexe second anticorps/NGAL humaine/premier anticorps déterminée dans l'étape (d).

6. Procédé selon la revendication 1 ou 5, où ledit au moins un premier anticorps est immobilisé sur une phase solide avant ou après la mise en contact avec ledit échantillon test.

**7.** Procédé selon la revendication 6, où ledit au moins un premier anticorps est immobilisé sur une phase solide avant la formation dudit complexe second anticorps/NGAL humaine/premier anticorps.

**8.** Procédé selon la revendication 7, où ledit au moins un premier anticorps est immobilisé sur une phase solide avant ou après la formation dudit complexe premier anticorps/NGAL humaine.

**9.** Procédé selon l'une quelconque des revendications 3 ou 5-8 précédentes, où ledit au moins un agent réducteur est choisi dans le groupe consistant en dithiothréitol, 2-mercaptoéthanol, 2-mercaptoéthylamine et tris(2-carboxyéthyl)phosphine.

**10.** Procédé selon l'une quelconque des revendications 3 ou 5-9 précédentes, où ledit au moins un agent réducteur est présent en la quantité d'environ 0,1 mM à environ 500 mM.

**11.** Procédé selon l'une quelconque des revendications 5-10 précédentes, où ledit échantillon test un échantillon d'urine ou de sang.

**12.** Procédé pour évaluer l'état de lésion des cellules tubulaires rénales d'un sujet, procédé dans lequel le niveau de monomère de NGAL humaine et/ou la proportion de monomères de NGAL humaine par rapport au dimère de NGAL humaine dans un échantillon test est déterminé selon une ou plusieurs des revendications 1-11.

**13.** Procédé selon la revendication 12, où ladite lésion des cellules tubulaires rénales comprend une lésion choisie dans le groupe consistant en une lésion rénale ischémique, une lésion néphrotoxique et une autre lésion qui affecte les cellules tubulaires du rein.

**14.** Procédé pour détecter la présence d'antigène de NGAL humaine dans un échantillon test, ledit procédé comprenant :

(1) la mise en contact d'un échantillon test suspecté de contenir NGAL humaine avec le réactif d'immunodiagnostic présenté ici pendant une durée et dans des conditions qui permettent la formation d'un complexe NGAL humaine/anticorps ; et
(2) la détection de tout complexe NGAL humaine/anticorps formé comme indiquant la présence dudit antigène de NGAL humaine,
où ledit réactif d'immunodiagnostic comprend un ou plusieurs anticorps choisis dans le groupe consistant en :

(a) un anticorps qui se lie spécifiquement à la protéine NGAL humaine à un épitope conformationnel comprenant les résidus d'aminoacides 112, 118 et 147 de la protéine NGAL humaine comme présenté dans SEQ ID NO:1, 2, 10 ou 13 et au moins un aminoacide supplémentaire qui est le résidu 117 ou 119 de NGAL humaine de type sauvage de SEQ ID NO:1 ou 13 ;
(b) un anticorps isolé qui se lie spécifique à NGAL humaine, où ledit anticorps a une région de domaine lourd variable comprenant la séquence d'aminoacides de SEQ ID NO:5 et une région de domaine léger variable comprenant la séquence d'aminoacides de SEQ ID NO:6 ; et
(c) un anticorps produit par la lignée cellulaire d'hybridome murine 1-2322-455 ayant le numéro d'ordre ATCC PTA 8024 ;
et un ou plusieurs anticorps choisis dans le groupe consistant en :
(d) un anticorps isolé qui se lie spécifiquement à NGAL humaine, où ledit anticorps a une région de domaine lourd variable comprenant la séquence d'aminoacides de SEQ ID NO:7 et une région de domaine léger variable comprenant la séquence d'aminoacides de SEQ ID NO:8 ; et
(e) un anticorps produit par la lignée cellulaire d'hybridome murine 1-903-430 ayant le numéro d'ordre ATCC PTA-8026.

**15.** Procédé selon l'une quelconque des revendications 1-14, où le procédé est adapté pour être utilisé dans un système automatisé ou un système semi-automatisé.

**16.** Kit de diagnostic pour la détection de NGAL humaine comprenant des instructions et un réactif d'immunodiagnostic qui comprend un ou plusieurs anticorps choisis dans le groupe consistant en :

(a) un anticorps qui se lie spécifiquement à la protéine NGAL humaine à un épitope conformationnel comprenant les résidus d'aminoacides 112, 118 et 147 de la protéine NGAL humaine comme présenté dans SEQ ID NO: 1, 2, 10 ou 13 et au moins un aminoacide supplémentaire qui est le résidu 117 ou 119 de NGAL humaine de type sauvage de SEQ ID NO:1 ou 13 ;

(b) un anticorps isolé qui se lie spécifique à NGAL humaine, où ledit anticorps a une région de domaine lourd variable comprenant la séquence d'aminoacides de SEQ ID NO:5 et une région de domaine léger variable comprenant la séquence d'aminoacides de SEQ ID NO:6 ; et

(c) un anticorps produit par la lignée cellulaire d'hybridome murine 1-2322-455 ayant le numéro d'ordre ATCC PTA 8024 ;

et un ou plusieurs anticorps choisis dans le groupe consistant en :

(d) un anticorps isolé qui se lie spécifiquement à GNAL humaine, où ledit anticorps a une région de domaine lourd variable comprenant la séquence d'aminoacides de SEQ ID NO:7 et une région de domaine léger variable comprenant la séquence d'aminoacides de SEQ ID NO:8 ; et

(e) un anticorps produit par la lignée cellulaire d'hybridome murine 1-903-430 ayant le numéro d'ordre ATCC PTA-8026.

Signal Peptide

*MPLGLLWLGL*    *ALLGALHAQA*    QDSTSDLIPA    PPLSKVPLQQ

NFQDNQFQGK    WYVVGLAGNA    ILREDKDPQK    MYATIYELKE

DKSYNVTSVL    FRKKKCDYWI    RTFVPGCQPG    EFTLGNIKSY

PGLTSYLVRV    VSTNYNQHAM    VFFKKVSQNR    EYFKITLYGR

TKELTSELKE    NFIRFSKSLG    LPENHIVFPV    PIDQCIDG<u>HH</u>

<u>HHHH</u>

6X His Tag

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 981470 P
- US 60981471 B
- US 60981473 B
- US 104408 A
- US 12104410 A
- US 12104413 A
- US 98147007 P
- US 98147107 P
- WO 8705330 A
- US 4931373 A
- US 4470461 A, Kaufinan
- US 5122464 A
- EP 0338841 A
- WO 9005783 A
- US 5023328 A
- US 4870008 A
- WO 8702670 A
- EP 238023 A
- US 5679543 A
- WO 9600787 A
- US 5077214 A
- WO 9100906 A
- WO 9110741 A
- WO 9203918 A
- WO 9305796 A
- US 5411749 A
- EP 0614984 A
- US 8602269 W
- EP 184187 A
- EP 171496 A
- US 5225539 A
- WO 9306213 A
- US 20070196876 A
- EP 0756708 A
- US 6136526 A
- WO 2002031507 A
- US 7056702 B
- US 20040115728 A
- US 5135875 A
- EP 0471293 A
- US 87801706 P
- US 49062406 A
- US 20080090304 A
- US 20080014644 A
- US 20080014604 A
- US 20070254370 A
- US 20070037232 A
- US 5627034 A
- US 5846739 A
- US 20080050832 A
- US 20070092911 A
- US 20070105166 A
- US 7153660 B
- US 20080166719 A
- US 20080085524 A
- US 5866432 A
- US 20070269836 A
- US 20030170881 A
- US 20040018577 A
- US 20050054078 A
- US 20060160164 A
- US 5063081 A
- US 5089424 A
- US 5006309 A
- US 5294404 A
- US 0880331 W

### Non-patent literature cited in the description

- **Aplin et al.** *CRC Crit. Rev. Biochem.,* 1981, 259-306
- **Lundblad et al.** Chemical Reagents for Protein Modification. CRC Press Inc,
- **Yan et al.** *Biochemistry,* 1982, vol. 23, 3759-3765
- **Doebber et al.** *J. Biol. Chem,* 1982, vol. 257, 2193-2199
- *Immunology,* 1977, vol. 32, 49
- Essential Immunology. Blackwell Scientific Publications, 1991, 74
- **Nelson ; Long.** *Analytical Biochemistry,* 1989, vol. 180, 147-151
- **Okkels.** *Ann. New YorkAcad. Sci.,* 1996, vol. 782, 202-207
- **Cate et al.** Isolation of the Bovine and Human Genes for Mullerian Inhibiting Substance And Expression of the Human Gene In Animal Cells. *Cell,* 1986, vol. 45, 685-698

- **Kaufinan et al.** Construction Of A Modular Dihydrofolate Reductase cDNA Gene: Analysis Of Signals Utilized For Efficient Expression. *Mol. Cell. Biol.,* 1982, vol. 2, 1304-1319
- **P. R. Russell.** *Gene,* 1985, vol. 40, 125-130
- **Kozak.** *J Mol Biol,* 1987, vol. 196, 947-50
- **Murphy et al.** *Protein Expression and Purification,* 1993, vol. 4, 349-357
- *Methods in Enzymology,* 1997, vol. 284, 262-272
- **Coloma, M.** *J. Imm. Methods,* 1992, vol. 152, 89-104
- **O. Hagenbuchle et al.** *Nature,* 1981, vol. 289, 643-646
- **L. A. Valls et al.** *Cell,* 1987, vol. 48, 887-897
- **M. Egel-Mitani et al.** *Yeast,* 1990, vol. 6, 127-137
- **Chang et al.** *Molecular General Genetics,* 1979, vol. 168, 111-115
- **Young et al.** *Journal of Bacteriology,* 1961, vol. 81, 823-829
- **Dubnau et al.** *Journal of molecular Biology,* 1971, vol. 56, 209-221
- **Shigekawa et al.** *Biotechniques,* 1988, vol. 6, 742-751
- **Koehler et al.** *Journal of Bacteriology,* 1987, vol. 169, 5771-5278
- **Malardier et al.** *Gene,* 1989, vol. 78, 147-156
- Guide to Yeast Genetics and Molecular Biology. **Becker ; Guarente.** Methods in Enzymology. Academic Press, Inc, vol. 194, 182-187
- **Ito et al.** *Journal of Bacteriology,* 1983, vol. 153, 163
- **Hinnen et al.** *Proceedings of the National Academy of Sciences USA,* 1978, vol. 75, 1920
- **Reeves et al.** *FEMS Microbiology Letters,* 1992, vol. 99, 193-198
- **Manivasakam et al.** *Nucleic Acids Research,* 1993, vol. 21, 4414-4415
- **Ganeva et al.** *FEMS Microbiology Letters,* 1994, vol. 121, 159-164
- Current Protocols in Molecular Biology. John Wiley & Sons, 1996
- Animal Cell Biotechnology, Methods and Protocols. Human Press Inc, 1999
- **Harrison ; Rae.** General Techniques of Cell Culture. Cambridge University Press, 1997
- Protein Purification. VCH Publishers, 1989
- **McCune et al.** *Science,* 1988, vol. 241, 1632-1639
- **Kohler ; Milstein.** *Nature,* 1975, vol. 256, 495497
- **Kozbar et al.** *Immunology Today,* 1983, vol. 4, 72
- **Cole et al.** Monoclonal Antibodies and Cancer Therapy. Alan R. Liss, Inc, 1985, 77-96
- **Wands et al.** *Gastroenterology,* 1981, vol. 80, 225-232
- **R. H. Kenneth.** Monoclonal Antibodies: A New Dimension In Biological Analyses. Plenum Publishing Corp, 1980
- **Morrison, S. L.** *Science,* 1985, vol. 229, 1202-1207
- **Oi et al.** *BioTechniques,* 1986, vol. 4, 214
- **Jones et al.** *Nature,* 1986, vol. 321, 552-525
- **Verhoeyan et al.** *Science,* 1988, vol. 239
- **Beidler et al.** *J. Immunol,* 1988, vol. 141, 4053-4060
- **Morimoto et al.** *J. Biochem. Biophys. Methods,* 1992, vol. 24, 107-117
- **Brennan et al.** *Science,* 1985, vol. 229, 81
- **Carter et al.** *Bio/Technology,* 1992, vol. 10, 163-167
- **Bird et al.** *Science,* 1998, vol. 242, 423-426
- **Bird et al.** *Science,* 1988, vol. 242, 423-426
- **Huston et al.** *Proc. Natl. Acad. Sci. USA,* 1988, vol. 85, 5879-5883
- **McCafferty et al.** *Nature,* 1990, vol. 348, 552-554
- **Holliger, P. et al.** *Proc. Natl. Acad. Sci. USA,* 1993, vol. 90, 6444-6448
- **Poljak, R. J. et al.** *Structure,* 1994, vol. 2, 1121-1123
- **Kjeldsen et al.** *J. Biolog. Chem.,* 1993, vol. 268, 10425-32
- **Kjeldsen et al.** *J. Immunolog. Methods,* 1996, vol. 198 (2), 155-64
- **Yatscoff et al.** Abbott TDx Monoclonal Antibody Assay Evaluated for Measuring Cyclosporine in Whole Blood. *Clin. Chem.,* 1990, vol. 36, 1969-1973
- **Wallemacq et al.** Evaluation of the New AxSYM Cyclosporine Assay:Comparison with TDx Monoclonal Whole Blood and EMIT Cyclosporine Assays. *Clin. Chem.,* 1999, vol. 45, 432-435
- **Polak ; Van Noorden.** Introduction to Immunocytochemistry. Springer Verlag, 1997
- **Haugland.** Handbook of Fluorescent Probes and Research Chemicals. Molecular Probes, Inc, 1996

SEQUENCE LISTING

<110> Abbott Laboratories
Birkenmeyer, Larry
Desai, Suresh
Grenier, Frank
Hawksworth, David
Olejniczak, Edward
Ruan, Qiaoqiao
Siegel, Robert
Tetin, Sergey
Tieman, Bryan
Tu, Bailin
Tyner, Joan
Workman, Ryan
Ziemann, Robert

<120> IMMUNOASSAYS AND KITS FOR THE DETECTION OF NGAL

<130> 8507W001

<140> PCTUS0880331
<141> 2008-10-17

<160> 13

<170> PatentIn version 3.4

<210> 1
<211> 198
<212> PRT
<213> Homo sapiens

<220>
<221> MISC_FEATURE
<222> (1)..(20)
<223> The first 20 amino acids are the signal peptide which would be labeled -1 to -20, with Gln (at position 21) being considered amino acid 1 of the NGAL peptide

<400> 1

```
Met Pro Leu Gly Leu Leu Trp Leu Gly Leu Ala Leu Leu Gly Ala Leu
1               5               10              15


His Ala Gln Ala Gln Asp Ser Thr Ser Asp Leu Ile Pro Ala Pro Pro
            20              25              30


Leu Ser Lys Val Pro Leu Gln Gln Asn Phe Gln Asp Asn Gln Phe Gln
        35              40              45


Gly Lys Trp Tyr Val Val Gly Leu Ala Gly Asn Ala Ile Leu Arg Glu
        50              55              60
```

```
        Asp Lys Asp Pro Gln Lys Met Tyr Ala Thr Ile Tyr Glu Leu Lys Glu
        65              70              75              80


        Asp Lys Ser Tyr Asn Val Thr Ser Val Leu Phe Arg Lys Lys Lys Cys
                    85              90              95


        Asp Tyr Trp Ile Arg Thr Phe Val Pro Gly Cys Gln Pro Gly Glu Phe
                    100             105             110


        Thr Leu Gly Asn Ile Lys Ser Tyr Pro Gly Leu Thr Ser Tyr Leu Val
                    115             120             125


        Arg Val Val Ser Thr Asn Tyr Asn Gln His Ala Met Val Phe Phe Lys
                    130             135             140


        Lys Val Ser Gln Asn Arg Glu Tyr Phe Lys Ile Thr Leu Tyr Gly Arg
        145             150             155             160


        Thr Lys Glu Leu Thr Ser Glu Leu Lys Glu Asn Phe Ile Arg Phe Ser
                    165             170             175


        Lys Ser Leu Gly Leu Pro Glu Asn His Ile Val Phe Pro Val Pro Ile
                    180             185             190


        Asp Gln Cys Ile Asp Gly
                    195
```

<210> 2
<211> 198
<212> PRT
<213> Artificial sequence

<220>
<223> Chemically synthesized

<220>
<221> MISC_FEATURE
<222> (1)..(20)
<223> The first 20 amino acids are the signal peptide which would be labeled -1 to -20, with Gln (at position 21) being considered amino acid 1 of the NGAL peptide

<400> 2

```
        Met Pro Leu Gly Leu Leu Trp Leu Gly Leu Ala Leu Leu Gly Ala Leu
```

```
                1              5                      10                    15


                His Ala Gln Ala Gln Asp Ser Thr Ser Asp Leu Ile Pro Ala Pro Pro
                            20                  25                  30


                Leu Ser Lys Val Pro Leu Gln Gln Asn Phe Gln Asp Asn Gln Phe Gln
                            35                  40                  45


                Gly Lys Trp Tyr Val Val Gly Leu Ala Gly Asn Ala Ile Leu Arg Glu
                            50                  55                  60


                Asp Lys Asp Pro Gln Lys Met Tyr Ala Thr Ile Tyr Glu Leu Lys Glu
                65                  70                  75                  80


                Asp Lys Ser Tyr Asn Val Thr Ser Val Leu Phe Arg Lys Lys Lys Cys
                            85                  90                  95


                Asp Tyr Trp Ile Arg Thr Phe Val Pro Gly Ser Gln Pro Gly Glu Phe
                            100                 105                 110


                Thr Leu Gly Asn Ile Lys Ser Tyr Pro Gly Leu Thr Ser Tyr Leu Val
                            115                 120                 125


                Arg Val Val Ser Thr Asn Tyr Asn Gln His Ala Met Val Phe Phe Lys
                            130                 135                 140


                Lys Val Ser Gln Asn Arg Glu Tyr Phe Lys Ile Thr Leu Tyr Gly Arg
                145                 150                 155                 160


                Thr Lys Glu Leu Thr Ser Glu Leu Lys Glu Asn Phe Ile Arg Phe Ser
                            165                 170                 175


                Lys Ser Leu Gly Leu Pro Glu Asn His Ile Val Phe Pro Val Pro Ile
                            180                 185                 190


                Asp Gln Cys Ile Asp Gly
                            195
```

<210> 3
<211> 612
<212> DNA
<213> Homo sapiens

<400> 3

```
atgcccctag gtctcctgtg gctgggccta gccctgttgg gggctctgca tgcccaggcc      60

caggactcca cctcagacct gatcccagcc ccacctctga gcaaggtccc tctgcagcag     120

aacttccagg acaaccaatt ccaggggaag tggtatgtgg taggcctggc agggaatgca     180

attctcagag aagacaaaga cccgcaaaag atgtatgcca ccatctatga gctgaaagaa     240

gacaagagct acaatgtcac ctccgtcctg tttaggaaaa agaagtgtga ctactggatc     300

aggacttttg ttccaggttg ccagcccggc gagttcacgc tgggcaacat taagagttac     360

cctggattaa cgagttacct cgtccgagtg gtgagcacca actacaacca gcatgctatg     420

gtgttcttca agaaagtttc tcaaaacagg gagtacttca agatcaccct ctacgggaga     480

accaaggagc tgacttcgga actaaaggag aacttcatcc gcttctccaa atctctgggc     540

ctccctgaaa accacatcgt cttccctgtc ccaatcgacc agtgtatcga cggccatcat     600

caccatcacc at                                                        612
```

<210> 4
<211> 612
<212> DNA
<213> Artificial sequence

<220>
<223> Chemically synthesized

<400> 4

```
atgcccctag gtctcctgtg gctgggccta gccctgttgg gggctctgca tgcccaggcc      60

caggactcca cctcagacct gatcccagcc ccacctctga gcaaggtccc tctgcagcag     120

aacttccagg acaaccaatt ccaggggaag tggtatgtgg taggcctggc agggaatgca     180

attctcagag aagacaaaga cccgcaaaag atgtatgcca ccatctatga gctgaaagaa     240

gacaagagct acaatgtcac ctccgtcctg tttaggaaaa agaagtgtga ctactggatc     300

aggacttttg ttccaggttc gcagcccggc gagttcacgc tgggcaacat taagagttac     360

cctggattaa cgagttacct cgtccgagtg gtgagcacca actacaacca gcatgctatg     420

gtgttcttca agaaagtttc tcaaaacagg gagtacttca agatcaccct ctacgggaga     480

accaaggagc tgacttcgga actaaaggag aacttcatcc gcttctccaa atctctgggc     540

ctccctgaaa accacatcgt cttccctgtc ccaatcgacc agtgtatcga cggccatcat     600

caccatcacc at                                                        612
```

<210> 5
<211> 119
<212> PRT
<213> Artificial sequence

42

<220>
<223> Chemically synthesized

<400> 5

Glu Val Gln Leu Val Glu Ser Gly Gly Gly Leu Val Gln Pro Gly Gly
1               5               10              15

Ser Leu Lys Leu Ser Cys Ala Ala Ser Gly Phe Thr Phe Asn Asn Tyr
                20              25              30

Tyr Met Ser Trp Val Arg Gln Thr Pro Glu Arg Arg Leu Glu Trp Val
            35              40              45

Ala Tyr Ile Ser Ser Ser Gly Gly Ser Thr Tyr Tyr Ser Asp Ser Val
        50              55              60

Arg Gly Arg Phe Thr Ile Ser Arg Asp Thr Ala Arg Asn Thr Leu Tyr
65              70              75              80

Leu Gln Met Thr Ser Leu Lys Ser Glu Asp Thr Ala Met Tyr Tyr Cys
                85              90              95

Ala Arg His Phe Gly Asp Tyr Ser Tyr Phe Asp Tyr Trp Gly Gln Gly
            100             105             110

Thr Thr Leu Thr Val Ser Ser
            115

<210> 6
<211> 108
<212> PRAT
<213> Artificial sequence

<220>
<223> Chemically synthesized

<400> 6

Asp Ile Gln Met Thr Gln Ser Pro Ala Ser Leu Ser Ala Ser Val Gly
1               5               10              15

Glu Thr Val Thr Ile Thr Cys Arg Ala Ser Glu Asn Phe Tyr Ser Tyr
                20              25              30

```
Leu Ala Trp Tyr Gln Gln Lys Gln Gly Lys Ser Pro Gln Leu Leu Val
        35              40              45

Tyr Asn Ala Lys Thr Leu Ala Glu Gly Val Pro Ser Arg Phe Ser Gly
        50              55              60

Ser Gly Ser Gly Thr Gln Phe Ser Leu Lys Ile Asn Ser Leu Gln Pro
65              70              75              80

Glu Asp Phe Gly Thr Tyr Tyr Cys Gln His His Tyr Asp Ile Pro Leu
                85              90              95

Thr Phe Gly Ala Gly Thr Lys Leu Glu Leu Lys Arg
            100             105
```

<210> 7
<211> 118
<212> PRT
<213> Artificial sequence

<220>
<223> Chemically synthesized

<400> 7

```
Lys Ile Gln Leu Val Gln Ser Gly Pro Glu Leu Lys Lys Pro Gly Glu
1                   5               10              15

Thr Val Lys Ile Ser Cys Lys Ala Ser Gly Tyr Thr Phe Thr Asn Tyr
            20              25              30

Gly Met Asn Trp Val Lys Gln Ala Pro Gly Lys Gly Leu Lys Trp Met
        35              40              45

Gly Trp Ile Asn Ile Asn Thr Gly Glu Pro Thr Tyr Ala Glu Glu Phe
        50              55              60

Lys Gly Arg Phe Ala Phe Ser Leu Glu Thr Ser Ala Thr Thr Ala Phe
65              70              75              80

Leu Gln Ile Asn Asn Leu Lys Asn Glu Asp Thr Ala Thr Tyr Leu Cys
                85              90              95

Ala Arg Asp Ser Tyr Ser Gly Gly Phe Asp Tyr Trp Gly Gln Gly Thr
            100             105             110

Ile Val Thr Val Ser Ser
                115
```

<210> 8
<211> 114
<212> PRT
<213> Artificial sequence

<220>
<223> Chemically synthesized

<400> 8

```
            Asp Ile Val Met Thr Gln Ser Pro Ser Ser Leu Ser Val Ser Ala Gly
            1               5                   10                  15


            Glu Lys Val Thr Leu Ser Cys Lys Ser Ser Gln Ser Leu Leu Ile Ser
                            20                  25                  30


            Gly Asp Gln Lys Asn Tyr Leu Ala Trp Tyr Gln Gln Lys Pro Gly Gln
                        35                  40                  45


            Pro Pro Lys Leu Leu Ile Tyr Gly Ala Ser Thr Arg Asp Ser Gly Val
                        50                  55                  60


            Pro Asp Arg Phe Thr Gly Ser Gly Ser Gly Ala Asp Phe Thr Leu Thr
            65                  70                  75                  80


            Ile Ser Ser Val Gln Ala Glu Asp Leu Ala Val Tyr Tyr Cys Gln Asn
                            85                  90                  95


            Asp His Ser Phe Pro Pro Thr Phe Gly Ala Gly Thr Lys Leu Glu Leu
                            100                 105                 110


            Lys Arg
```

<210> 9
<211> 558
<212> DNA
<213> Artificial sequence

<220>
<223> Chemically synthesized

<400> 9

```
            atgcaggact ctacttccga cctgattccg gctccgccgc tgtctaaagt gccgctgcag        60
```

```
cagaactttc aagacaacca gttccagggt aaatggtacg ttgtgggcct ggctggtaac    120

gcgatcctgc gtgaagacaa agatccgcag aaaatgtatg ctaccatcta cgaactgaaa    180

gaagacaaat cttataacgt gaccagcgtt ctgtttcgta aaaagaaatg tgactactgg    240

attcgcacct tcgtgccggg ctctcagccg ggcgagttca ctctgggtaa catcaaatct    300

tacccgggtc tgactagcta cctggtgcgt gtggtttcta ctaactataa ccagcatgct    360

atggtgttct tcaagaaagt ttctcagaac cgtgaatact tcaagattac tctgtacggt    420

cgtaccaaag agctgacctc tgagctgaaa gaaaacttca tccgtttctc taaatctctg    480

ggcctgccgg agaaccatat cgtgtttccg gttccgatcg atcagtgcat cgacggtcat    540

catcaccatc accattga                                                  558
```

<210> 10
<211> 178
<212> PRT
<213> Artificial sequence

<220>
<223> Chemically synthesized

<220>
<221> MISC_FEATURE
<222> (1)..(1)
<223> Gln can be preceded by a Met residue (e.g., when synthetic and/or produced in prokaryotes)

<220>
<221> MISC_FEATURE
<222> (178)..(178)
<223> Can, optionally, be succeeded at the C-terminus by one or more His residues, and especially. 6 His residues (HHHHHH).

<400> 10

```
Gln Asp Ser Thr Ser Asp Leu Ile Pro Ala Pro Pro Leu Ser Lys Val
1               5               10              15


Pro Leu Gln Gln Asn Phe Gln Asp Asn Gln Phe Gln Gly Lys Trp Tyr
            20              25              30


Val Val Gly Leu Ala Gly Asn Ala Ile Leu Arg Glu Asp Lys Asp Pro
            35              40              45


Gln Lys Met Tyr Ala Thr Ile Tyr Glu Leu Lys Glu Asp Lys Ser Tyr
        50              55              60
```

```
Asn Val Thr Ser Val Leu Phe Arg Lys Lys Lys Cys Asp Tyr Trp Ile
65              70          75          80

Arg Thr Phe Val Pro Gly Ser Gln Pro Gly Glu Phe Thr Leu Gly Asn
              85          90          95

Ile Lys Ser Tyr Pro Gly Leu Thr Ser Tyr Leu Val Arg Val Val Ser
            100         105         110

Thr Asn Tyr Asn Gln His Ala Met Val Phe Phe Lys Lys Val Ser Gln
        115         120         125

Asn Arg Glu Tyr Phe Lys Ile Thr Leu Tyr Gly Arg Thr Lys Glu Leu
        130         135         140

Thr Ser Glu Leu Lys Glu Asn Phe Ile Arg Phe Ser Lys Ser Leu Gly
145         150         155         160

Leu Pro Glu Asn His Ile Val Phe Pro Val Pro Ile Asp Gln Cys Ile
            165         170         175

Asp Gly
```

<210> 11
<211> 15
<212> PRT
<213> Artificial Sequence

<220>
<223> scFV linking sequence

<400> 11

```
Gly Pro Ala Lys Glu Leu Thr Pro Leu Lys Glu Ala Lys Val Ser
1           5           10          15
```

<210> 12
<211> 552
<212> DNA
<213> Artificial sequence

<220>
<223> Chemically synthesized

<220>
<221> misc_feature
<222> (1)..(1)
<223> Optionally, can be preceded at the N-terminus by an initiation codon encoding Met, namely, an ATG.

<400> 12

```
caggactcca cctcagacct gatcccagcc ccacctctga gcaaggtccc tctgcagcag      60

aacttccagg acaaccaatt ccaggggaag tggtatgtgg taggcctggc agggaatgca     120

attctcagag aagacaaaga cccgcaaaag atgtatgcca ccatctatga gctgaaagaa     180

gacaagagct acaatgtcac ctccgtcctg tttaggaaaa agaagtgtga ctactggatc     240

aggacttttg ttccaggttc gcagcccggc gagttcacgc tgggcaacat taagagttac     300

cctggattaa cgagttacct cgtccgagtg gtgagcacca actacaacca gcatgctatg     360

gtgttcttca agaaagtttc tcaaaacagg gagtacttca agatcaccct ctacgggaga     420

accaaggagc tgacttcgga actaaaggag aacttcatcc gcttctccaa atctctgggc     480

ctccctgaaa accacatcgt cttccctgtc ccaatcgacc agtgtatcga cggccatcat     540

caccatcacc at                                                        552
```

<210> 13
<211> 178
<212> PRT
<213> Artificial sequence

<220>
<223> Chemically synthesized

<220>
<221> MISC_FEATURE
<222> (1)..(1)
<223> Gln can be preceded by a Met residue (e.g., when synthetic and/or produced in prokaryotes.

<220>
<221> MISC_FEATURE
<222> (178)..(178)
<223> Can, optionally, be succeeded at the C-terminus by one or more His residues, and especially, 6 His residues (HHHHHH).

<400> 13

```
Gln Asp Ser Thr Ser Asp Leu Ile Pro Ala Pro Pro Leu Ser Lys Val
1               5               10              15

Pro Leu Gln Gln Asn Phe Gln Asp Asn Gln Phe Gln Gly Lys Trp Tyr
        20              25              30
```

```
Val Val Gly Leu Ala Gly Asn Ala Ile Leu Arg Glu Asp Lys Asp Pro
        35                  40                  45

Gln Lys Met Tyr Ala Thr Ile Tyr Glu Leu Lys Glu Asp Lys Ser Tyr
        50                  55                  60

Asn Val Thr Ser Val Leu Phe Arg Lys Lys Lys Cys Asp Tyr Trp Ile
65                  70                  75                  80

Arg Thr Phe Val Pro Gly Cys Gln Pro Gly Glu Phe Thr Leu Gly Asn
                85                  90                  95

Ile Lys Ser Tyr Pro Gly Leu Thr Ser Tyr Leu Val Arg Val Val Ser
            100                 105                 110

Thr Asn Tyr Asn Gln His Ala Met Val Phe Phe Lys Lys Val Ser Gln
        115                 120                 125

Asn Arg Glu Tyr Phe Lys Ile Thr Leu Tyr Gly Arg Thr Lys Glu Leu
        130                 135                 140

Thr Ser Glu Leu Lys Glu Asn Phe Ile Arg Phe Ser Lys Ser Leu Gly
145                 150                 155                 160

Leu Pro Glu Asn His Ile Val Phe Pro Val Pro Ile Asp Gln Cys Ile
                165                 170                 175

Asp Gly
```